# FASCICULE DE BREVET EUROPEEN

(11) **EP 2 931 708 B1**
(45) Date de publication et mention de la délivrance du brevet: **10.05.2017**
(21) Numéro de dépôt: 13815083.4
(22) Date de dépôt: 12.12.2013
(51) Int. Cl.: C07D 213/85, C07D 215/14, C07D 217/16, C07D 317/46, C07D 231/12, C07D 333/16, C07D 239/26, C07F 5/02, C07D 213/55, C07D 213/61, C07D 307/46, C07D 307/79, C07C 67/30, C07C 69/743, C07C 69/75, C07C 69/757

(54) **COMPOSES CYCLOPROPYLBORONIQUES, LEUR PROCEDE DE PREPARATION ET LEUR UTILISATION**
CYCLOPROPYLBORVERBINDUNGEN, VERFAHREN ZUR HERSTELLUNG DAVON UND VERWENDUNG DAVON
CYCLOPROPYLBORONIC COMPOUNDS, METHOD FOR PREPARING SAME AND USE THEREOF

(30) Priorité: 12.12.2012 FR 1261973
(43) Date de publication de la demande: 21.10.2015
(73) Titulaire: Diverchim, 95700 Roissy (FR)
(72) Inventeur: OOSTING, Peter, F-94200 Ivry Sur Seine (FR); THOMAS, Emmanuel, F-75019 Paris (FR); PAMUK, Rukiye, F-60810 Rully (FR); FOLLEAS, Benoit, F-60300 Senlis (FR); BRAYER, Jean-Louis, F-60440 Nanteuil Le Haudouin (FR); DE CARNE CARNAVALET, Benoït, F-75005 Paris (FR); MEYER, Christophe, F-95700 Roissy en France (FR); COSSY, Janine, 75005 Paris Cedex 05 (FR)
(74) Mandataire: Grosset-Fournier, Chantal Catherine
(86) Numéro de dépôt international: PCT/FR2013/053057
(87) Numéro de publication internationale: WO 2014/091167

(56) Documents cités:
- WO-A1-2007/025144
- JÖRG PIETRUSZKA ET AL: "Enantiomerically Pure Cyclopropylamines from Cyclopropylboronic Esters", EUROPEAN JOURNAL OF ORGANIC CHEMISTRY, vol. 2009, no. 34, 1 décembre 2009 (2009-12-01), pages 5998-6008, XP055077858, ISSN: 1434-193X, DOI: 10.1002/ejoc.200900882
- ERWIN HOHN ET AL: "Synthesis of Enantiomerically Pure Cyclopropyl Trifluoroborates", SYNLETT, vol. 2006, no. 10, 12 juin 2006 (2006-06-12), pages 1531-1534, XP055077830, ISSN: 0936-5214, DOI: 10.1055/s-2006-944185
- VAULTIER M ET AL: "Product Subclass 28: Vinylboranes", 23 décembre 2004 (2004-12-23), SCIENCE OF SYNTHESIS; [METHODS OF MOLECULAR TRANSFORMATIONS. (HOUBEN-WEYL)], STUTTGART : GEORG THIEME VERLAG, DE, PAGE(S) 721 - 853, XP002560453, ISBN: 978-3-13-112191-2 page 825

## Description

La présente invention a pour objet des composés cyclopropylboroniques, leur procédé de préparation et leur utilisation.

Le motif cyclopropane est présent dans des nombreux produits naturels (terpènes, stéroïdes, polycétides, phéromones, métabolites d'acides gras, aminoacides non usuels) possédant des activités biologiques très variées (antibiotique, antivirale, antifongique, antitumorale, neuromédiatrice, insecticide, régulation de la croissance des plantes, maturation des fruits).

L'acide chrysanthémique et les pyréthrines, insecticides isolés de la fleur *Chrysanthemum cinerariaefolium,* peuvent être cités comme exemples de cyclopropanes naturels bioactifs:

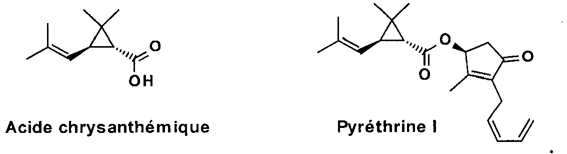

Les propriétés électroniques et stériques du cyclopropane, notamment sa rigidité conformationnelle qui permet d'orienter dans l'espace des groupements fonctionnels de manière parfaitement définie, en font un motif structural particulièrement important et intéressant en chimie médicinale.

Le motif cyclopropane, notamment substitué par un hétéroatome (azote ou oxygène) se retrouve dans de nombreux médicaments actuellement sur le marché, et candidats médicaments en développement, par exemple le Tranylcypromine, le Trovafloxacin, le Tasimelteon et des anti-viraux contre l'hépatite C, en particulier le MR 200 (J. Med. Chem. 2011, 54(10), 3669), le Simeprevir, le Danoprevir, l'Asunaprevir, le MK 5172, le Sovaprevir et le Vanipevir.

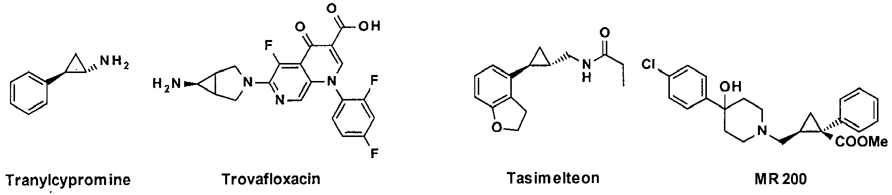

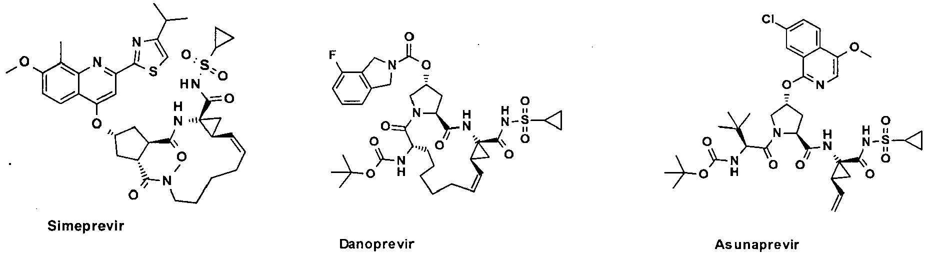

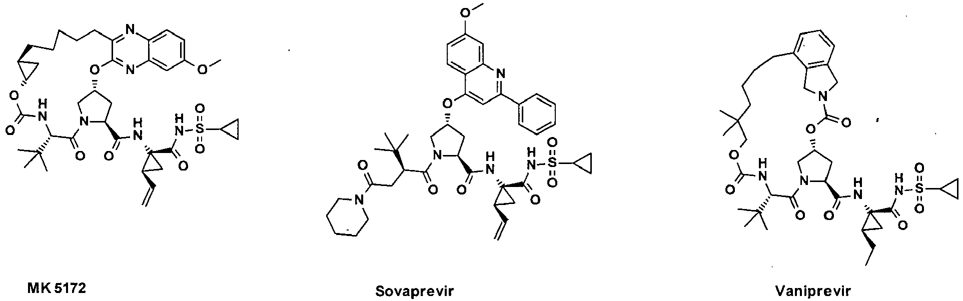

Les composés cyclopropaniques constituent également des intermédiaires importants en synthèse organique. Selon la nature des substituants et leurs propriétés électroniques, le cycle à trois chaînons peut être ouvert par des réactions thermiques, photochimiques, promues par des électrophiles, des nucléophiles, des radicaux ou catalysées par des complexes organométalliques, dont la force motrice est le relâchement de la tension de cycle.

Au vu de l'importance de cet élément structural, de nombreuses synthèses de cyclopropanes ont été publiées. La majorité des réactions formant un cycle cyclopropyle font appel à la réaction de Simmons-Smith, la réaction de Corey-Chaikovski ou alors l'addition d'un carbène formé à partir d'un composé diazoïque.

Cependant, ces réactions ne permettent pas l'introduction directe et convergente d'un motif cyclopropyle sur une molécule d'intérêt.

WO2007/025144 et Pietruszka et al. (Synlett 2006, 10, 1531-1534) divulguent des cyclopropyl boroniques. Leur structure est différente de celle des composés de la présente invention.

Pietruszka et al. (Eur. J. Org. Chem. 2009, 34, 5998-6008) divulgue un procédé pour la préparation de cyclopropyl boroniques avec un dérivé diazoïque CH₂N₂. Vaultier et al. (Product Subclass 28: Vinylboranes, Science of Synthesis, Stuttgart ; Georg Thieme Verlag, 721-853) décrit un procédé pour la préparation de cyclopropyl boroniques avec un dérivé diazoïque CH₃O-C(=O)-CHN₂. Ces procédés sont différents de celui de la présente invention.

Dans le cadre des couplages organométalliques, les organotrifluoroborates présentent en tant que réactifs de nombreux avantages par rapport aux acides boroniques et aux esters desdits acides : lesdits organotrifluoroborates sont des complexes « ates » tétravalents présentant une exceptionnelle stabilité à l'air, l'humidité et les composés nucléophiles. La grande majorité peut être stockée indéfiniment à température ambiante sans précaution particulière. Enfin, malgré cette stabilité, les dérivés organotrifluoroborates présentent une très grande réactivité dans une large gamme de réactions et particulièrement les couplages organométalliques catalysés par un métal de transition.

Ainsi, l'un des objectifs de la présente invention consiste à fournir des composés cyclopropyle trifluoroborates fonctionnalisés.

Un autre objectif de l'invention consiste à préparer lesdits composés par un procédé simple et rapide.

Un autre objectif de l'invention est l'utilisation desdits composés cyclopropyle trifluoroborates fonctionnalisés dans une réaction de couplage organométallique catalysé par un métal de transition afin d'introduire un motif cyclopropyle fonctionnalisé à une molécule d'intérêt.

La description a par conséquent pour objet un procédé de préparation d'un composé répondant à la formule (I) suivante : 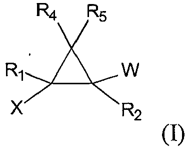 dans laquelle :
▪ X représente un atome de bore substitué choisi dans le groupe comprenant B(OH)₂, B(OR)₂, BF₃M, B(OR')₃M dans lesquels :
   ∘ R est un groupe alkyle comportant de 1 à 14 atomes de carbone ou un groupe aryle, éventuellement substitués, ou est tel que (OR)₂ forme un cycle entre les deux atomes d'oxygène, (OR)₂ étant notamment choisi dans le groupe comprenant les radicaux bivalents dérivant de diols, tels que O-CH₂-CH₂-O, O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O et ses esters, et les radicaux bivalents dérivant de diacides, tels que OCO-CH₂-N(CH₃)-CH₂-COO,
   ∘ R' est un groupe alkyle comportant de 1 à 14 atomes de carbone ou est tel que :
      - (OR')₃ forme un cycle entre deux des atomes d'oxygène, (OR')₃ étant alors sous la forme OR'(OR)₂, où R' est un groupe alkyle comportant de 1 à 14 atomes de carbone et (OR)₂ est tel que défini ci-dessus, ou
      - (OR')₃ forme un bicycle entre les trois atomes d'oxygène, (OR')₃ étant notamment choisi dans le groupe comprenant les radicaux trivalents dérivant de triols, tels que H₃C-C-(CH₂-O)₃,
   ∘ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} R^{e}, R^{f} sont choisis parmi H ou une chaîne carbonée saturée comportant notamment 1 à 6 atomes de carbone choisis indépendamment les uns des autres,
   et notamment X représente B(OH)₂, B(OR)₂ ou BF₃K,
▪ R₁, R₄ et R₅ identiques ou différents sont choisis dans le groupe constitué:
   1. de H
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   R₁ et R₄, ou R₁ et R₅ pouvant former un cycle à 5, 6, ou 7 chainons comportant éventuellement un hétéroatome choisi parmi l'oxygène, de l'azote et le soufre, ledit cycle pouvant être substitué ;
▪ R₂ est choisi dans le groupe constitué par les groupes pouvant être représentés par R₁, R₄ ou R₅, ainsi que -COR^{a}, -COOR^{a}, -CONH₂, -CONHR^{a}, -CONR^{a}R^{b}, -CN et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CONH-SO₂-R^{a}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a},-OH, -OR^{a}, -OZ', -NH₂, -NHR^{a}, -NR^{a}R^{b}, -NHZ et -NZZ₂,
dans lesquels Z et Z₂ représentent un groupe protecteur d'une fonction amine, et Z' représente un groupe protecteur d'une fonction alcool, et
dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
❖ lorsque W représente un groupe fonctionnel choisi parmi -CHO, -COR^{a}, -COOH,-COOR^{a}, -CONH₂, -CONHR^{a}, -CONR^{a}R^{b}, -CH₂OH, -CH₂OR^{a}, -CHR^{b}OH, -CHR^{b}OR^{a},-CR^{b}R^{b'}OH, -CR^{b}R^{b'}OR^{a}, -CH₂NH₂, -CH₂NHZ, -CHR^{a}NHZ, ledit procédé comprenant :
   - une étape de réaction entre :
      ▪ un dérivé diazoïque de formule suivante : 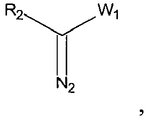 dans laquelle R₂ est tel que défini ci-dessus, W₁ étant choisi dans le groupe constitué de -COR^{a}, -COOR^{a}, -CONH₂, -CONHR^{a}, et -CONR^{a}R^{b},
         et
      ▪ un composé vinyltrifluoroborate de formule suivante : 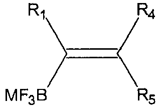
         dans laquelle R₁, R₄, R₅ et M sont tels que définis ci-dessus,
         en présence d'un catalyseur contenant un métal de transition, pour obtenir un composé de formule suivante : 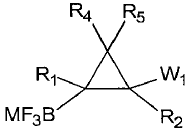
         dans laquelle R₁, R₂, R₄, R₅, W₁ et M sont tels que définis ci-dessus,
   si W est différent de W₁ et/ou X est différent de MF₃B, ledit procédé comprenant en outre les étapes suivantes :
   • une étape de conversion de W₁ en W permettant d'obtenir 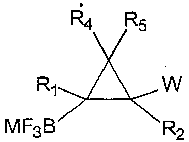 à partir de 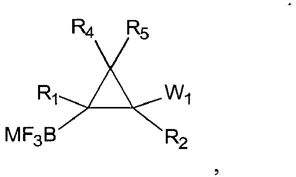 en particulier, lorsque W₁ = COOR^{a} et W = CHO, par réduction pour former l'alcool correspondant, puis oxydation dudit alcool, lorsque W₁ = -COOR^{a} et W= CH₂OH ou - CH₂OR^{b}, par formation d'un aldéhyde tel que décrit précédemment, puis par réduction dudit aldéhyde et alkylation éventuelle, lorsque W₁ = -COR^{a} et W = -CHR^{a}OH, -CHR^{a}OR^{b}, par réduction puis alkylation éventuelle de l'alcool obtenu, lorsque W₁= -COR^{a} et W= CR^{a}R^{b}OH ou -CR^{a}R^{b}OR^{b'} par addition d'un Grignard puis alkylation éventuelle de l'alcool obtenu, lorsque W₁ = -CONH₂, -CONHR^{a} ou -CONR^{a}R^{b} et W = -CH₂NH₂, -CH₂NHR^{a}, -CH₂NR^{a}R^{b}, -CH₂NHZ ou -CH₂-NH-COR^{a}, par réduction puis protection éventuelle par Z de l'amine obtenue ou réaction éventuelle avec le chlorure d'acide R^{a}COCl, lorsque W₁ = -CONH₂ et W = -CONHSO₂R^{a}, par action du chlorure de sulfonyle ClSO₂R^{a} sur l'amide,
      et
   • une étape de conversion de -BF₃M en -X permettant d'obtenir 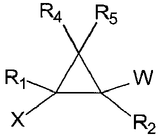 à partir de 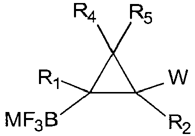 en particulier, lorsque X = B(OH)₂, par hydrolyse basique ou acide, ou par passage par un dihalogénoborane, plus particulièrement un dichloroborane, lorsque X = B(OR)₂, par passage par X = B(OH)₂ comme décrit précédemment puis par action d'un alcool, en particulier un alcool de formule ROH, un diol ou un triol, ou par passage par un dihalogénoborane, plus particulièrement un dichloroborane, puis par action d'un alcool, en particulier un alcool de formule ROH, un diol ou un triol,
      ou
   • une étape de conversion de -BF₃M en -X permettant d'obtenir 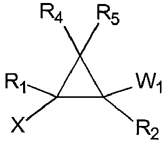 à partir de 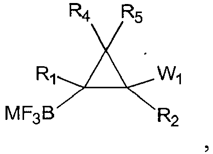 et
   • une étape de conversion de W₁ en W permettant d'obtenir 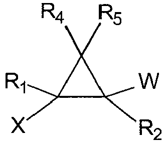 à partir de 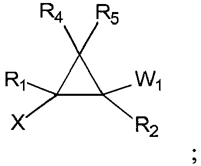
❖ lorsque W représente un groupe fonctionnel choisi parmi -OH, -OR^{a}, -OZ', -NH₂, -NHR^{a}, -NR^{a}R^{b}, -NHZ et -NZZ₂, en particulier parmi -OH, -OR^{a} et -OZ', ledit procédé comprenant :
   • le traitement d'un composé de formule suivante : 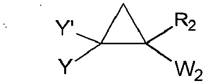 dans laquelle R₂ est tel que défini ci-dessus, et dans laquelle W₂ représente un groupe fonctionnel choisi parmi -OR^{a}, -OZ', -NR^{a}R^{b} et -NZZ₂, en particulier parmi -OR^{a} et -OZ', Y étant un halogénure, en particulier -Br, Y' étant un halogénure, en particulier -Br, ou H, par :
      • une base forte, en particulier un alkyl lithium, plus particulièrement le n-butyllithium ou le *sec*-butyllithium, puis
      • un composé de formule X"-B(OR)₂, R étant tel que défini ci-dessus, X" représentant H, un groupe O-alkyle comportant de 1 à 14 atomes de carbone ou un groupe O-aryle éventuellement substitués,
      pour obtenir un composé de formule suivante : 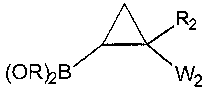 dans laquelle R, R₂ et W₂ sont tels que définis ci-dessus,
      ou
   • une réaction de type Simmons-Smith à partir d'un composé de formule suivante : 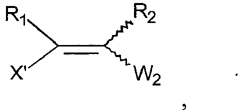 dans laquelle R₁ et R₂ sont tels que définis précédemment, W₂ représentant un groupe fonctionnel choisi parmi -OR^{a}, -OZ', -NR^{a}R^{b} et -NZZ₂, en particulier parmi -OR^{a} et - OZ', X' représentant B(OR)₂ ou BF₃M, en particulier B(OR)₂, dans lesquels R et M sont tels que définis précédemment,
      pour obtenir un composé de formule suivante : 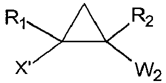 dans laquelle R₁, R₂, X' et W₂ sont tels que définis ci-dessus,
   si W représente -OH ou -NH₂, et/ou X est différent de B(OR)₂, ledit procédé comprenant en outre les étapes suivantes, R₁ représentant en particulier H:
   - une étape de conversion de W₂ en W, en particulier la déprotection du groupement Z' lorsque W₂ représente -OZ' et des groupements Z et Z₂ lorsque W₂ représente -NZZ₂, permettant d'obtenir 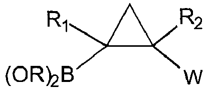 à partir de 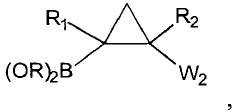 et
   - une étape de conversion de B(OR)₂ en -X permettant d'obtenir 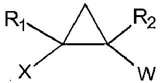 à partir de 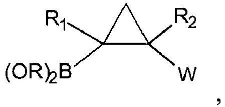 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
      ou
   - une étape de conversion de B(OR)₂ en -X permettant d'obtenir 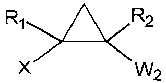 à partir de 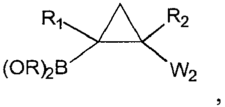 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
      et
   - une étape de conversion de W₂ en W, en particulier la déprotection du groupement Z' lorsque W₂ représente -OZ' et des groupements Z et Z₂ lorsque W₂ représente -NZZ₂, permettant d'obtenir 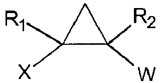 à partir de 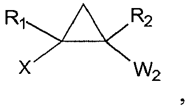 ou, lorsque X' représente BF₃M, W représentant -OH ou -NH₂,
   - une étape de conversion de W₂ en W, en particulier la déprotection du groupement Z' lorsque W₂ représente -OZ' ou des groupements Z et Z₂ lorsque W₂ représente -NZZ₂, permettant d'obtenir 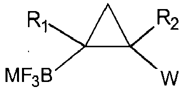 à partir de

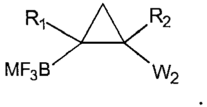

Dans ce qui précède et ce qui suit, en particulier à propos des aryles, des hétérocycles, des hétéroaryles, des alcényles, des alcynyles, des alkyles, des cycles éventuellement formés par R₁ et R₄, ou R₁ et R₅, et des groupes R^{a}, R^{b} et R^{b'}, on entend par substitué le fait d'être substitué par :
∘ par 1 ou plusieurs atomes d'halogène comprenant le fluor, le chlore, le brome ou l'iode,
∘ par des radicaux hydroxy, amino ou thio éventuellement protégés par des groupes protecteurs « *ad hoc »,*
∘ par des radicaux -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a},-N(C=NH)NH₂, -N-(C=NCOR^{a})NHCOR^{b}, -N(C=NCOOR^{a})NHCOOR^{b},
   dans lesquels R^{a} et R^{b} identiques ou différents représentent des groupements alkyles, alcényles, alcynyles linéaires ou ramifiés, cycloalkyles, cycloalcényles, aromatiques ou hétérocycliques aromatiques ou non aromatiques comportant de 1 à 15 atomes de carbone, éventuellement substitués,
   par des radicaux alkyles comportant de 1 à 15 atomes de carbone éventuellement substitués,
o par des radicaux alcényles comportant de 1 à 15 atomes de carbone, linéaires ou ramifiés, éventuellement substitués,
o par des radicaux alcynyles comportant de 1 à 15 atomes de carbone, linéaires ou ramifiés éventuellement substitués,
o par des radicaux aryles comportant de 6 à 12 atomes de carbone, linéaires ou ramifiés, éventuellement substitués,
o par des hétérocycles aromatiques ou non-aromatiques comportant de 2 à 12 atomes de carbone éventuellement substitués.

Dans ce qui précède et ce qui suit, les groupes R^{a}, R^{b} et R^{b'}, identiques ou différents, représentent des groupements alkyles, alcényles, alcynyles linéaires ou ramifiés, cycloalkyles, cycloalcényles, aromatiques ou hétérocycliques aromatiques ou non aromatiques comportant de 1 à 15 atomes de carbone, éventuellement substitués.

Le terme « groupe protecteur d'une fonction alcool » représente un groupe destiné à protéger un alcool contre les réactions indésirables au cours des étapes de synthèse. Les groupements protecteurs des alcools communément utilisés sont décrits dans Greene, "Protective Groups In Organic Synthesis" (John Wiley & Sons, New York (1981). Des exemples de tels groupes sont les groupes acétyle, benzoyle, benzyle pivaloyle, trityle, méthoxytrityle, diméthoxytrityle, tetrahydropyranyle, et éthers tels que les éthers méthylique, benzylique, allylique, éthoxyéthylique, β-méthoxyéthoxyméthylique, méthoxyméthylique, p-méthoxybenzylique, méthylthiométhylique, et silyliques.

Le terme « groupe protecteur d'une fonction thiol » représente un groupe destiné à protéger un thiol contre les réactions indésirables au cours des étapes de synthèse.

Les groupements protecteurs des thiols communément utilisés sont décrits dans Greene, "Protective Groups In Organic Synthesis" (John Wiley & Sons, New York (1981). Des exemples de tels groupes sont les groupes benzoyle et éthers tels que les éthers méthylique, éthoxyéthylique, benzylique, p-méthoxybenzylique et silyliques.

Le terme « groupe protecteur d'une fonction amine» représente un groupe destiné à protéger une amine contre les réactions indésirables au cours des étapes de synthèse. Les groupements protecteurs des amines communément utilisés sont décrits dans Greene (*ibid.*)*.* Des exemples de tels groupes sont les groupes carbamate, amide et les dérivés N-alkyle, acétal, N-benzyle, imine, enamine et N-hétéroatome. En particulier, les groupements protecteurs des amines comprennent les groupes formyle, acétyle, benzoyle, pivaloyle, phénysulfonyle, *p*-toluènesulfonyle (tosyle), benzyle, t-butyloxycarbonyle (Boc), benzyloxycarbonyle (Cbz) et fluorenylméthyloxycarbonyle (Fmoc).

En ce qui concerne la conversion du groupe W₁ en W, la conversion d'un ester - COOR^{a} en l'acide correspondant peut être effectué par des méthodes bien connues de l'homme du métier, par exemple par saponification, ou par hydrolyse acide.

L'acide ainsi obtenu peut être converti en l'aldéhyde correspondant par passage par un amide de Weinreb de type CONR-OR' puis réduction subséquente par un hydrure, par exemple DIBALH, dans un solvant, par exemple le THF, à basse température.

La conversion d'un ester en l'aldéhyde correspondant peut par exemple être réalisée par réduction de l'ester par un hydrure, notamment le DIBAlH, dans un solvant, par exemple le THF, à basse température, ou par réduction en alcool par action d'un hydrure, notamment NaBH₄ ou LiAlH₄, puis oxydation subséquente en aldéhyde par action d'un réactif oxydant, par exemple un réactif chromate de type PDC ou PCC, un periodinane de type réactif de Dess-Martin ou IBX, un sulfoxyde en présence d'une base selon la réaction de Swern.

La conversion d'une cétone -COR^{a} en l'alcool -CHR^{a}OH correspondant peut par exemple être réalisée par une réaction de réduction dans des conditions classiques connues par l'homme de l'art notamment l'action d'un borohydrure, préférentiellement NaBH₄, dans un solvant alcoolique, en particulier le méthanol.

Ledit alcool -CHR^{a}OH peut être converti en -CHR^{a}OR^{b} par l'action d'une base forte telle que NaH ou la LDA puis alkylation par un halogénure d'alkyle R^{b}-X.

La conversion d'une cétone -COR^{a} en alcool -CR^{a}R^{b}OH correspondant peut par exemple être réalisée par l'action d'un Grignard de type R^{b}-MgX dans un solvant type THF ou éther diéthylique. Ledit alcool -CR^{a}R^{b}OH peut être converti en -CR^{a}R^{b}OR^{b'} par une réaction d'alkylation comme décrit précédemment.

La conversion d'un amide -CONH₂, -CONHR^{a} ou -CONR^{a}R^{b} en respectivement l'amine -CH₂NH₂, -CH₂NHR^{a} et -CH₂NR^{a}R^{b} correspondante peut par exemple être réalisée par une réaction de réduction de la fonction amide par l'action d'un hydrure, préférentiellement LiAlH₄, dans un solvant, en particulier le THF. Si nécessaire, la protection de l'amine par un groupe protecteur, par exemple le groupement CBz est effectué selon un mode opératoire connu de l'homme du métier ; lorsque le groupe protecteur est par exemple un amide, l'action d'un chlorure d'acide R^{a}COCl (par exemple EtCOCl) en présence d'une base minérale ou organique dans un solvant, préférentiellement le diméthylformamide, permet de former l'amide correspondant.

La conversion de -CONH₂ en -CONHSO₂R^{a} est par exemple réalisée par action du chlorure de sulfonyle ClSO₂R^{a} sur l'amide.

En ce qui concerne la conversion du groupe W₂ en W, la déprotection de Z', Z et/ou Z₂ est effectué selon des modes opératoires bien connus de l'homme du métier. Par exemple la conversion -NZZ₂ en -NHZ peut être effectuée par déprotection sélective du groupement protecteur Z₂, par exemple, pour Z= -COAlkyl et Z₂= Boc par hydrolyse acide (par exemple acide trifluoroacétique) dans un solvant organique type dichlorométhane ou dioxane, ou par exemple, pour Z= -Bn et Z₂= Fmoc par traitement par une base secondaire (préférentiellement la pipéridine) dans le DMF, ou par exemple, pour Z= COAlkyl et Z₂= CBz par hydrogénolyse en présence d'un catalyseur (préférentiellement Pd/C) dans un solvant alcoolique.

En ce qui concerne la conversion du groupe -BF₃M en X, la conversion d'un groupe-BF3M en -B(OH)₂ peut par exemple être réalisée par hydrolyse basique en présence d'une base minérale ou organique, par exemple Na₂CO₃ ou LiOH, dans un mélange eau / solvant organique, par exemple le dichlorométhane, l'acétonitrile ou le THF, ou par hydrolyse acide en présence d'un acide de Lewis, par exemple FeCl₃, ou préférentiellement par passage à un dihalogénoborane par traitement par un silane, par exemple SiCl₄ ou TMSCl, dans un mélange eau / solvant organique, par exemple le dichlorométhane, l'acétonitrile, le THF, le dioxane ou l'acétone à température ambiante puis hydrolyse *in situ.*

La conversion d'un groupe -BF₃M en B(OR)₂ peut par exemple être réalisée par passage intermédiaire par B(OH)₂ comme décrit précédemment puis protection subséquente en ester boronique, par exemple le B(Opinacol)₂, par action du groupement protecteur alcool (par exemple le diol pinacol) dans un solvant alcoolique en présence facultative d'un catalyseur acide, par exemple l'APTS, et d'un agent desséchant, par exemple MgSO₄ ou un tamis moléculaire dans un solvant, en particulier un alcool, le toluène, ou l'éther diéthylique à température ambiante ou à reflux du solvant ou préférentiellement par passage à un dihalogénoborane par traitement par un silane, en particulier SiCl₄ ou TMSCl, dans un mélange eau / solvant organique, par exemple le dichlorométhane, l'acétonitrile, le THF, le dioxane ou l'acétone, à température ambiante puis traitement par le groupement protecteur sous forme de diol, par exemple le diol pinacol, dans un solvant alcoolique.

La conversion d'un groupe -BF₃M en B(OR')₃M peut par exemple être réalisée par passage intermédiaire par B(OH)₂ comme décrit précédemment puis traitement par un alcool R'OH en présence d'une base forte minérale, préférentiellement la potasse, dans un solvant, préférentiellement le toluène à chaud.

En ce qui concerne la conversion du groupe - B(OR)₂ en X, ladite étape de conversion est en particulier en présence de MHF₂ lorsque X représente BF₃M, ou est en particulier une hydrolyse, plus particulièrement en présence d'une base minérale, organique ou en présence d'un acide de Lewis, lorsque X représente B(OH)₂.

La conversion d'un groupe -B(OR)₂ en B(OR')₃M peut par exemple être réalisée par traitement par un alcool R'OH en présence d'une base forte minérale, préférentiellement la potasse, dans un solvant, préférentiellement le toluène à chaud.

Selon un mode de réalisation avantageux, la présente description concerne un procédé de préparation d'un composé répondant à la formule (I), dans laquelle W représente un groupe fonctionnel choisi parmi -CHO, -COR^{a}, -COOH, -COOR^{a}, -CONH₂, -CONHR^{a}, -CONR^{a}R^{b}, - CH₂OH, -CH₂OR^{a}, -CHR^{b}OH, -CHR^{b}OR^{a}, -CR^{b}R^{b'}OH, -CR^{b}R^{b'}OR^{a}, -CH₂NH₂, -CH₂NHZ, -CHR^{a}NHZ, ledit procédé comprenant :
- une étape de réaction entre :
   ▪ un dérivé diazoïque de formule suivante : 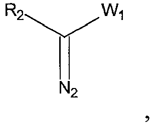 dans laquelle R₂ est tel que défini précédemment, W₁ étant choisi dans le groupe constitué de -COR^{a}, -COOR^{a}, -CONH₂, -CONHR^{a}, et -CONR^{a}R^{b},
      et
   ▪ un composé vinyltrifluoroborate de formule suivante : 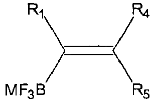
   dans laquelle R₁, R₄, R₅ et M sont tels que définis précédemment,
   en présence d'un catalyseur contenant un métal de transition, pour obtenir un composé de formule suivante : 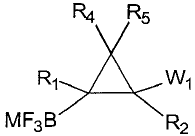 dans laquelle R₁, R₂, R₄, R₅, W₁ et M sont tels que définis précédemment,
si W est différent de W₁ et/ou X est différent de MF₃B, ledit procédé comprenant en outre les étapes suivantes :
- une étape de conversion de W₁ en W permettant d'obtenir 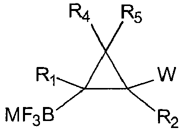 à partir de 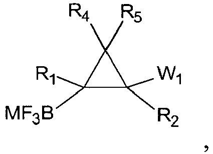 et
- une étape de conversion de -BF₃M en -X permettant d'obtenir 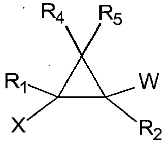 à partir de 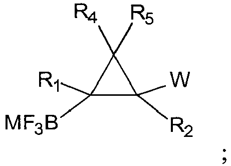 ou
- une étape de conversion de -BF₃M en -X permettant d'obtenir 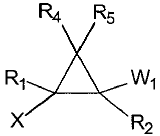 à partir de 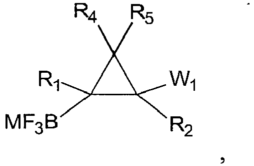 et
- une étape de conversion de W₁ en W permettant d'obtenir 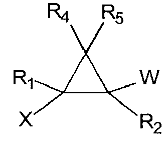 à partir de

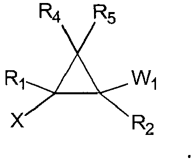

Selon un mode de réalisation avantageux, la présente description concerne un procédé de préparation d'un composé répondant à la formule (I), dans laquelle W représente un groupe fonctionnel W₁ choisi parmi -COR^{a}, -COOR^{a}, -CONH₂, -CONHR^{a}, et -CONR^{a}R^{b}, ledit procédé comprenant :
- une étape de réaction entre :
   ▪ un dérivé diazoïque de formule suivante : 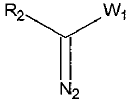 dans laquelle R₂ est tel que défini précédemment, W₁ étant tel que défini ci-dessus,
      et
   ▪ un composé vinyltrifluoroborate de formule suivante : 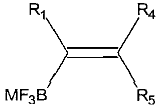 dans laquelle R₁, R₄, R₅ et M sont tels que définis précédemment,
   en présence d'un catalyseur contenant un métal de transition, pour obtenir un composé de formule suivante : 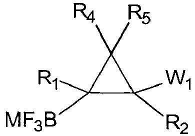 dans laquelle R₁, R₂, R₄, R₅, W₁ et M sont tels que définis ci-dessus.

Selon un mode de réalisation avantageux, la présente description concerne un procédé de préparation d'un composé répondant à la formule (I), dans laquelle W représente un groupe fonctionnel choisi parmi -CHO, -CH₂OH, -CH₂OR^{a}, -CHR^{b}OH, -CHR^{b}OR^{a}, -CR^{b}R^{b'}OH, - CR^{b}R^{b'}OR^{a}, -CH₂NH₂, -CH₂NHZ, -CHR^{a}NHZ, ledit procédé comprenant :
- une étape de réaction entre :
   ▪ un dérivé diazoïque de formule suivante : 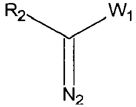 dans laquelle R₂ est tel que défini précédemment, W₁ étant choisi dans le groupe constitué de -COR^{a}, -COOR^{a}, -CONH₂, -CONHR^{a}, et -CONR^{a}R^{b},
      et
   ▪ un composé vinyltrifluoroborate de formule suivante : 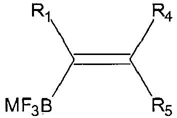 dans laquelle R₁, R₄, R₅ et M sont tels que définis précédemment,
   en présence d'un catalyseur contenant un métal de transition, pour obtenir un composé de formule suivante : 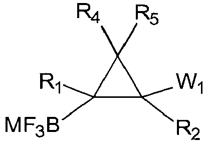 dans laquelle R₁, R₂, R₄, R₅, W₁ et M sont tels que définis précédemment, ledit procédé comprenant en outre l'étape suivante :
   • une étape de conversion de W₁ en W permettant d'obtenir 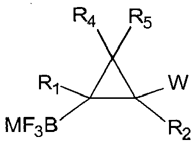 à partir de

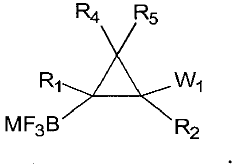

Selon un mode de réalisation avantageux, la présente description concerne un procédé de préparation d'un composé répondant à la formule (I), dans laquelle W représente un groupe fonctionnel W₁ choisi parmi -COR^{a}, -COOR^{a}, -CONH₂, -CONHR^{a}, et -CONR^{a}R^{b}, ledit procédé comprenant :
- une étape de réaction entre :
   ▪ un dérivé diazoïque de formule suivante : 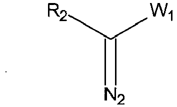 dans laquelle R₂ est tel que défini précédemment, W₁ étant tel que défini ci-dessus,
      et
   ▪ un composé vinyltrifluoroborate de formule suivante : 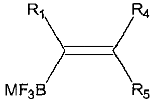 dans laquelle R₁, R₄, R₅ et M sont tels que définis précédemment,
   en présence d'un catalyseur contenant un métal de transition, pour obtenir un composé de formule suivante : 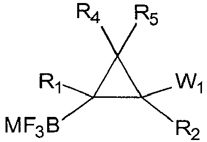 dans laquelle R₁, R₂, R₄, R₅, W₁ et M sont tels que définis ci-dessus, ledit procédé comprenant en outre l'étape suivante :
   • une étape de conversion de BF₃M en X, X représentant B(OH)₂, B(OR)₂, ou B(OR')₃M, permettant d'obtenir 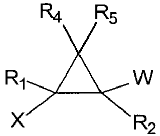 à partir de

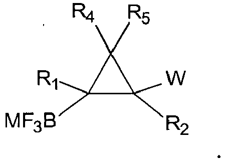

Selon un mode de réalisation avantageux, la présente description concerne un procédé de préparation d'un composé répondant à la formule (I), dans laquelle W représente un groupe fonctionnel choisi parmi -CHO, -CH₂OH, -CH₂OR^{a}, -CHR^{b}OH, -CHR^{b}OR^{a}, -CR^{b}R^{b'}OH, - CR^{b}R^{b'}OR^{a}, -CH₂NH₂, -CH₂NHZ, -CHR^{a}NHZ, ledit procédé comprenant :
- une étape de réaction entre :
   ▪ un dérivé diazoïque de formule suivante : 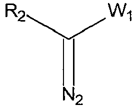 dans laquelle R₂ est tel que défini précédemment, W₁ étant choisi dans le groupe constitué de -COR^{a}, -COOR^{a}, -CONH₂, -CONHR^{a}, et -CONR^{a}R^{b},
      et
   ▪ un composé vinyltrifluoroborate de formule suivante : 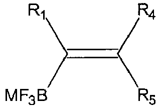 dans laquelle R₁, R₄, R₅ et M sont tels que définis précédemment,
   en présence d'un catalyseur contenant un métal de transition, pour obtenir un composé de formule suivante : 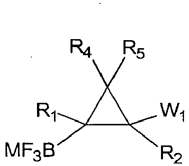 dans laquelle R₁, R₂, R₄, R₅, W₁ et M sont tels que définis précédemment,
ledit procédé comprenant en outre les étapes suivantes :
- une étape de conversion de W₁ en W permettant d'obtenir 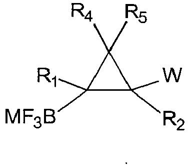 à partir de 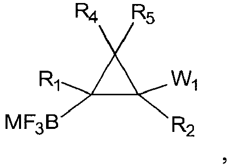 et
- une étape de conversion de BF₃M en X, X représentant B(OH)₂, B(OR)₂, ou B(OR')₃M, permettant d'obtenir 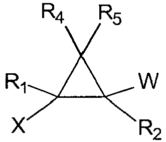 à partir de 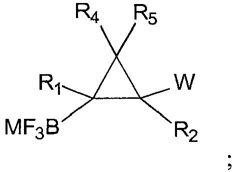 ou
- une étape de conversion de BF₃M en X, X représentant B(OH)₂, B(OR)₂, ou B(OR')₃M, permettant d'obtenir 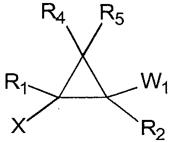 à partir de 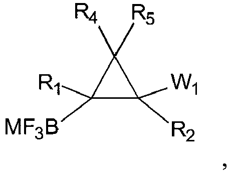 et
- une étape de conversion de W₁ en W permettant d'obtenir 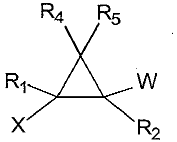 à partir de

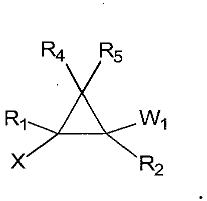

Selon un mode de réalisation avantageux, la présente invention concerne un procédé de préparation dans lequel ledit catalyseur contenant un métal de transition, ledit catalyseur étant en particulier un complexe de palladium (II), plus particulièrement Pd(OAc)₂ ou Pd(acac)₂, un complexe du cuivre (II), plus particulièrement CuSO₄, Cu(acac)₂, Cu(tBuSalen)₂, Cu(OTf)₂, un complexe de cuivre (I), plus particulièrement CuI ou Cu(OTf), ou un complexe du rhodium (II), plus particulièrement Rh₂(OAc)₄, Rh₂(Octanoate)₄ ou Rh₂(5S-MEPY)₄ (catalyseur de Doyle).

Selon un mode de réalisation avantageux, la présente invention concerne un procédé de préparation, dans lequel W₁ représente -COOR^{a}, R^{a} étant tel que défini précédemment.

Selon un mode de réalisation avantageux, la présente invention concerne un procédé de préparation, dans lequel R₂ est choisi dans le groupe constitué par les groupes -COR^{a},-COOR^{a}, -CONH₂, -CONHR^{a}, -CONR^{a}R^{b}, -CN et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation, dans lequel R₂ représente H.

Selon un mode de réalisation avantageux, la présente invention concerne un procédé de préparation, dans lequel R₁, R₄ et R₅ représentent H, un aryle, un hétérocycle, un hétéroaryle ou un alkyle tels que définis précédemment.

Selon un mode de réalisation avantageux, la présente invention concerne un procédé de préparation, dans lequel R₁, R₂, R₄ et R₅ représentent H.

Selon un mode de réalisation avantageux, la présente invention concerne un procédé de préparation, dans lequel W représente un groupe fonctionnel choisi parmi -CONH-SO₂-cyclopropyle, -CH₂-NH-CO-CH₂-CH₃, et le groupe de formule suivante :

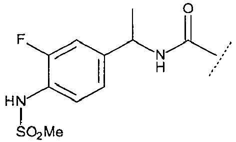

Selon un mode de réalisation avantageux, la présente invention concerne un procédé de préparation d'un composé répondant à la formule (I-A) suivante 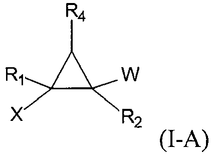 dans laquelle :
▪ X représente un atome de bore substitué choisi dans le groupe comprenant B(OH)₂, B(OR)₂, BF₃M, B(OR')₃M dans lesquels :
   ∘ R est un groupe alkyle comportant de 1 à 14 atomes de carbone ou un groupe aryle, éventuellement substitués, ou est tel que (OR)₂ forme un cycle entre les deux atomes d'oxygène, (OR)₂ étant notamment choisi dans le groupe comprenant les radicaux bivalents dérivant de diols, tels que O-CH₂-CH₂-O, O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O et ses esters, et les radicaux bivalents dérivant de diacides, tels que OCO-CH₂-N(CH₃)-CH₂-COO,
   ∘ R' est un groupe alkyle comportant de 1 à 14 atomes de carbone ou est tel que :
      - (OR')₃ forme un cycle entre deux des atomes d'oxygène, (OR')₃ étant alors sous la forme OR'(OR)₂, où R' est un groupe alkyle comportant de 1 à 14 atomes de carbone et (OR)₂ est tel que défini ci-dessus, ou
      - (OR')₃ forme un bicycle entre les trois atomes d'oxygène, (OR')₃ étant notamment choisi dans le groupe comprenant les radicaux trivalents dérivant de triols, tels que H₃C-C-(CH₂-O)₃,
   ∘ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}; R^{d} R^{e}, R^{f} sont choisis parmi H ou une chaîne carbonée saturée comportant notamment 1 à 6 atomes de carbone choisis indépendamment les uns des autres,
   et notamment X représente B(OH)₂, B(OR)₂ ou BF₃K,
▪ R₁ et R₄ identiques ou différents sont choisis dans le groupe constitué:
   1. de H
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   l'un au moins des groupes R₁ et R₄ représentant H,
▪ R₂ est choisi dans le groupe constitué par les groupes pouvant être représentés par R₁ ou R₄, ainsi que -COR^{a}, -COOR^{a}, -CONH₂, -CONHR^{a}, -CONR^{a}R^{b}, -CN et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CONH-SO₂-R^{a}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a}, dans lesquels Z représente un groupe protecteur d'une fonction amine, et
   dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués ;
   ledit procédé comprenant :
   - une étape de réaction entre :
      ▪ un dérivé diazoïque de formule suivante : 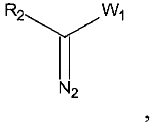 dans laquelle R₂ est tel que défini ci-dessus, W₁ étant choisi dans le groupe constitué de -COR^{a}, -COOR^{a}, -CONH₂, -CONHR^{a}, et -CONR^{a}R^{b},
         et
      ▪ un composé vinyltrifluoroborate de formule suivante : 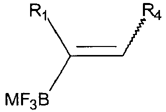
      dans laquelle R₁, R₄ et M sont tels que définis ci-dessus,
      en présence d'un catalyseur contenant un métal de transition, pour obtenir un composé de formule suivante : 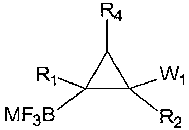 dans laquelle R₁, R₂, R₄, W₁ et M sont tels que définis ci-dessus,
   si W est différent de W₁ et/ou X est différent de MF₃B, ledit procédé comprenant en outre les étapes suivantes :
   - une étape de conversion de W₁ en W permettant d'obtenir 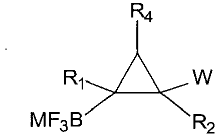 à partir de 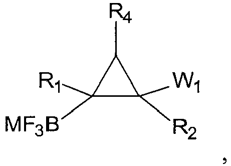 en particulier, lorsque W₁ = COOR^{a} et W = CHO, par réduction pour former l'alcool correspondant, puis oxydation dudit alcool, lorsque W₁ = -COOR^{a} et W= CH₂OH ou - CH₂OR^{b}, par formation d'un aldéhyde tel que décrit précédemment, puis par réduction dudit aldéhyde et alkylation éventuelle, lorsque W₁ = -COR^{a} et W = -CHR^{a}OH,-CHR^{a}OR^{b}, par réduction puis alkylation éventuelle de l'alcool obtenu, lorsque W₁=-COR^{a} et W= CR^{a}R^{b}OH ou -CR^{a}R^{b}OR^{b'} par addition d'un Grignard puis alkylation éventuelle de l'alcool obtenu, lorsque W₁ = -CONH₂, -CONHR^{a} ou -CONR^{a}R^{b} et W =-CH₂NH₂, -CH₂NHR^{a}, -CH₂NR^{a}R^{b}, -CH₂NHZ ou -CH₂-NH-COR^{a}, par réduction puis protection éventuelle par Z de l'amine obtenue ou réaction éventuelle avec le chlorure d'acide R^{a}COCl, lorsque W₁ = -CONH₂ et W = -CONHSO₂R^{a}, par action du chlorure de sulfonyle ClSO₂R^{a} sur l'amide,
      et
   - une étape de conversion de -BF₃M en -X permettant d'obtenir 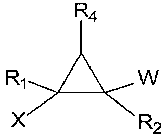 à partir de 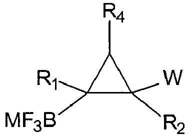 en particulier, lorsque X = B(OH)₂, par hydrolyse basique ou acide, ou par passage par un dihalogénoborane, plus particulièrement un dichloroborane, lorsque X = B(OR)₂, par passage par X = B(OH)₂ comme décrit précédemment puis par action d'un alcool, en particulier un alcool de formule ROH, un diol ou un triol, ou par passage par un dihalogénoborane, plus particulièrement un dichloroborane, puis par action d'un alcool, en particulier un alcool de formule ROH, un diol ou un triol, ou
   - une étape de conversion de -BF₃M en -X permettant d'obtenir 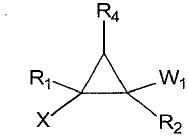 à partir de 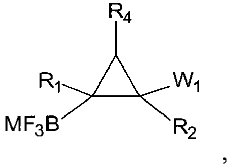 et
   - une étape de conversion de W₁ en W permettant d'obtenir 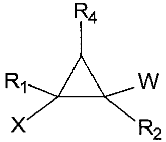 à partir de

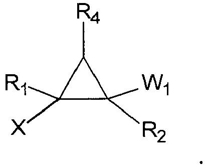

Selon un mode de réalisation avantageux, la présente invention concerne un procédé de préparation d'un composé répondant à la formule (I-B) suivante 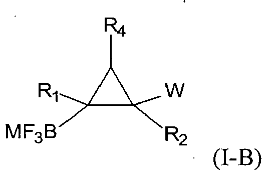 dans laquelle :
▪ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} R^{e}, R^{f} sont choisis parmi H ou une chaîne carbonée saturée comportant notamment 1 à 6 atomes de carbone choisis indépendamment les uns des autres,
   BF₃M représentant notamment BF₃K,
▪ R₁ et R₄ identiques ou différents sont choisis dans le groupe constitué:
   1. de H
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   l'un au moins des groupes R₁ et R₄ représentant H,
▪ R₂ est choisi dans le groupe constitué par les groupes pouvant être représentés par R₁ ou R₄, ainsi que -COR^{a}, -COOR^{a}, -CONH₂, -CONHR^{a}, -CONR^{a}R^{b}, -CN et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CONH-SO₂-R^{a}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a}, dans lesquels Z représente un groupe protecteur d'une fonction amine, et
   dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués ;
ledit procédé comprenant :
- une étape de réaction entre :
   ▪ un dérivé diazoïque de formule suivante : 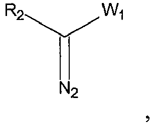 dans laquelle R₂ est tel que défini ci-dessus, W₁ étant choisi dans le groupe constitué de -COR^{a}, -COOR^{a}, -CONH₂, -CONHR^{a}, et -CONR^{a}R^{b},
      et
   ▪ un composé vinyltrifluoroborate de formule suivante : 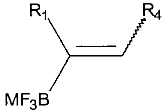 dans laquelle R₁, R₄ et M sont tels que définis ci-dessus, en présence d'un catalyseur contenant un métal de transition, pour obtenir un composé de formule suivante : 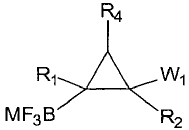 dans laquelle R₁, R₂, R₄, W₁ et M sont tels que définis ci-dessus,
si W est différent de W₁, ledit procédé comprenant en outre les étapes suivantes :
- une étape de conversion de W₁ en W permettant d'obtenir 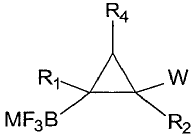 à partir de 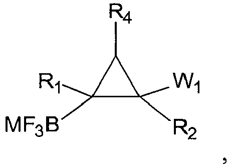 en particulier, lorsque W₁ = COOR^{a} et W = CHO, par réduction pour former l'alcool correspondant, puis oxydation dudit alcool, lorsque W₁ = -COOR^{a} et W= CH₂OH ou - CH₂OR^{b}, par formation d'un aldéhyde tel que décrit précédemment, puis par réduction dudit aldéhyde et alkylation éventuelle, lorsque W₁ = -COR^{a} et W = -CHR^{a}OH,-CHR^{a}OR^{b}, par réduction puis alkylation éventuelle de l'alcool obtenu, lorsque W₁=-COR^{a} et W= CR^{a}R^{b}OH ou -CR^{a}R^{b}OR^{b'} par addition d'un Grignard puis alkylation éventuelle de l'alcool obtenu, lorsque W₁ = -CONH₂, -CONHR^{a} ou -CONR^{a}R^{b} et W =-CH₂NH₂, -CH₂NHR^{a}, -CH₂NR^{a}R^{b}, -CH₂NHZ ou -CH₂-NH-COR^{a}, par réduction puis protection éventuelle par Z de l'amine obtenue ou réaction éventuelle avec le chlorure d'acide R^{a}COCl, lorsque W₁ = -CONH₂ et W = -CONHSO₂R^{a}, par action du chlorure de sulfonyle ClSO₂R^{a} sur l'amide.

Selon un mode de réalisation avantageux, la présente invention concerne un procédé de préparation d'un composé de formule (I-A), dans laquelle :
▪ lorsque R₁, R₂ et R₄ représentent H et que B représente B(OH)₂, B(OR)₂, ou B(OR')₃M, alors W est choisi parmi -COR^{a}, -CONH₂, -CONHR^{a}, -CONR^{a}R^{b},
▪ lorsque R₁, R₂ et R₄ représentent H et que B représente BF₃M, alors W est choisi parmi-COOH, -COOR^{a}, -CHO, -COR^{a} -CONH₂, -CONHR^{a}, -CONR^{a}R^{b},
▪ lorsque W représente CH₂OH, -CH₂OR^{a}, -CHR^{b}OH, -CHR^{b}OR^{a}, -CR^{b}R^{b'}OH, ou - CR^{b}R^{b'}OR^{a}, et que B représente B(OH)₂, B(OR)₂, ou B(OR')₃M, alors :
   ∘ R₁ ne représente pas H, ou
   ∘ R₂ et R₄ ne représentent pas H,
▪ lorsque W représente CH₂OH, -CH₂OR^{a}, -CHR^{b}OH, -CHR^{b}OR^{a}, -CR^{b}R^{b'}OH, ou - CR^{b}R^{b'}OR^{a}, et que B représente BF₃M, alors R₁ ou R₂ ne représente pas H.
▪ lorsque W représente -COOH ou -COOR^{a}, et que B représente B(OH)₂, B(OR)₂, ou B(OR')₃M, alors :
   ∘ R₁ ne représente pas H, ou
   ∘ R₂ et R₄ ne représentent pas H.

Selon un mode de réalisation avantageux, la présente invention concerne un procédé de préparation d'un composé de formule (I-B), dans laquelle :
▪ lorsque R₁, R₂ et R₄ représentent H, alors W est choisi parmi -COOH, -COOR^{a}, -CHO,-COR^{a} -CONH₂, -CONHR^{a}, -CONR^{a}R^{b},
▪ lorsque W représente CH₂OH, -CH₂OR^{a}, -CHR^{b}OH, -CHR^{b}OR^{a}, -CR^{b}R^{b'}OH, ou-CR^{b}R^{b'}OR^{a}, alors R₁ ou R₂ ne représente pas H.

Selon un mode de réalisation avantageux, la présente invention concerne un procédé de préparation d'un composé de formule (I-A) ou (I-B) dans lequel ledit catalyseur contenant un métal de transition est un complexe de palladium (II), plus particulièrement Pd(OAc)₂ ou Pd(acac)₂, un complexe du cuivre (II), plus particulièrement CuSO₄, Cu(acac)₂, Cu(tBuSalen)₂, Cu(OTf)₂, un complexe de cuivre (I), plus particulièrement CuI ou Cu(OTf), ou un complexe du rhodium (II), plus particulièrement Rh₂(OAc)₄, Rh₂(Octanoate)₄ ou Rh₂(5S-MEPY)₄ (catalyseur de Doyle).

Selon un mode de réalisation avantageux, la présente invention concerne un procédé de préparation d'un composé de formule (I-A) ou (I-B) dans lequel ledit catalyseur contenant un métal de transition est un complexe de palladium (II), plus particulièrement Pd(OAc)₂ ou Pd(acac)₂, ou un complexe du cuivre (II), plus particulièrement CuSO₄, Cu(acac)₂, Cu(tBuSalen)₂, Cu(OTf)₂.

Selon un mode de réalisation avantageux, la présente invention concerne un procédé de préparation d'un composé de formule (I-A) ou (I-B), dans laquelle W₁ est choisi dans le groupe constitué de -COOR^{a}, -CONH₂, -CONHR^{a}, et -CONR^{a}R^{b}, R^{a} et R^{b} étant tel que défini précédemment.

Selon un mode de réalisation avantageux, la présente invention concerne un procédé de préparation d'un composé de formule (I-A) ou (I-B), dans laquelle W₁ représente -COOR^{a}, R^{a} étant tel que défini précédemment, R^{a} représentant en particulier un alkyle, un cycloalkyle, un benzyle, éventuellement substitués.

Selon un mode de réalisation avantageux, la présente invention concerne un procédé de préparation d'un composé de formule (I-A) ou (I-B) dans laquelle W représente un groupe fonctionnel choisi parmi -CHO, -COR^{a}, -COOH, -COOR^{a}, -CONH₂, -CONHR^{a}, -CONR^{a}R^{b}, -CONH-SO₂-R^{a},
W₁ étant en particulier choisi dans le groupe constitué de -COOR^{a}, -CONH₂, -CONHR^{a}, et - CONR^{a}R^{b}, R^{a} et R^{b} étant tel que défini précédemment, W₁ représentant plus particulièrement-COOR^{a}, R^{a} étant tel que défini précédemment, R^{a} représentant en particulier un alkyle, un cycloalkyle, un benzyle, éventuellement substitués.

Selon un mode de réalisation avantageux, la présente invention concerne un procédé de préparation d'un composé de formule (I-A) ou (I-B) dans lequel W représente un groupe fonctionnel choisi parmi -CHO, -COR^{a}, -COOH et -COOR^{a}, W₁ représentant en particulier - COOR^{a}, R^{a} étant tel que défini précédemment, R^{a} représentant en particulier un alkyle, un cycloalkyle, un benzyle, éventuellement substitués.

Selon un mode de réalisation avantageux, la présente invention concerne un procédé de préparation d'un composé de formule (I-A) ou (I-B) dans lequel W et W₁ représentent - COOR^{a}, R^{a} étant tel que défini précédemment, R^{a} représentant en particulier un alkyle, un cycloalkyle, un benzyle, éventuellement substitués.

Selon un mode de réalisation avantageux, la présente invention concerne un procédé de préparation d'un composé de formule (I-A) ou (I-B) dans lequel W représente un groupe fonctionnel choisi parmi -CONH-SO₂-cyclopropyle, -CH₂-NH-CO-CH₂-CH₃ et le groupe de formule suivante :

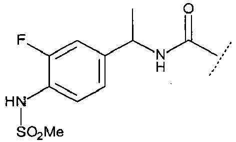

Selon un mode de réalisation avantageux, la présente invention concerne un procédé de préparation d'un composé de formule (I-A) ou (I-B) dans lequel R₂ représente H.

Selon un mode de réalisation avantageux, la présente invention concerne un procédé de préparation d'un composé de formule (I-A) ou (I-B) dans lequel R₂ est choisi dans le groupe constitué par les groupes -COR^{a}, -COOR^{a}, -CONH₂, -CONHR^{a}, -CONR^{a}R^{b}, -CN et - NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation d'un composé de formule (I-A) ou (I-B) dans lequel R₂ est choisi dans le groupe constitué par les groupes -COR^{a}, -COOR^{a}, -CONH₂, -CONHR^{a}, -CONR^{a}R^{b} et -NO₂, dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation d'un composé de formule (I-A) ou (I-B) dans lequel R₂ est choisi dans le groupe constitué par les groupes -COOR^{a}, -CONH₂, -CONHR^{a} et -CONR^{a}R^{b},
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation d'un composé de formule (I-A) ou (I-B) dans lequel R₁, R₂ et R₄ représentent H.

Selon un mode de réalisation avantageux, la présente invention concerne un procédé de préparation d'un composé de formule (I-A) ou (I-B) dans lequel R₁ représente H.

Selon un mode de réalisation avantageux, la présente invention concerne un procédé de préparation d'un composé de formule (I-A) ou (I-B) dans lequel R₁ représente H et R₄ représente un aryle, un hétérocycle, un hétéroaryle ou un alkyle tels que définis précédemment.

Selon un mode de réalisation avantageux, la présente invention concerne un procédé de préparation d'un composé de formule (I-A) ou (I-B) dans lequel R₄ représente H.

Selon un mode de réalisation avantageux, la présente invention concerne un procédé de préparation d'un composé de formule (I-A) ou (I-B) dans lequel R₄ représente H et R₁ représente un aryle, un hétérocycle, un hétéroaryle ou un alkyle tels que définis précédemment.

Selon un mode de réalisation avantageux, la présente invention concerne un procédé de préparation d'un composé de formule (I-A) ou (I-B) dans lequel R₁ et R₂ représentent H et R₄ représente un aryle, un hétérocycle, un hétéroaryle ou un alkyle tels que définis précédemment.

Selon un mode de réalisation avantageux, la présente invention concerne un procédé de préparation d'un composé de formule (I-A) ou (I-B) dans lequel R₂ et R₄ représentent H et R₁ représente un aryle, un hétérocycle, un hétéroaryle ou un alkyle tels que définis précédemment.

Selon un mode de réalisation avantageux, la présente invention concerne un procédé de préparation d'un composé de formule (I-A) ou (I-B) dans lequel R₁ représente H, R₄ représente un aryle, un hétérocycle, un hétéroaryle ou un alkyle tels que définis précédemment, et R₂ est choisi dans le groupe constitué par les groupes -COR^{a}, -COOR^{a}, - CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation d'un composé de formule (I-A) ou (I-B) dans lequel R₄ représente H, R₁ représente un aryle, un hétérocycle, un hétéroaryle ou un alkyle tels que définis précédemment, et R₂ est choisi dans le groupe constitué par les groupes -COR^{a}, -COOR^{a}, - CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation d'un composé de formule (I-A) ou (I-B) dans lequel R₁ et R₄ représentent H, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation d'un composé de formule (I-A) ou (I-B) dans laquelle R₁ représente H, ledit catalyseur étant un complexe de palladium (II), plus particulièrement Pd(OAc)₂ ou Pd(acac)₂.

Selon un mode de réalisation avantageux, la présente invention concerne un procédé de préparation d'un composé de formule (I-A) ou (I-B) dans laquelle R₄ représente H, ledit catalyseur étant un complexe du cuivre (II), en particulier CuSO₄, Cu(acac)₂, Cu(tBuSalen)₂, Cu(OTf)₂, ou un complexe de cuivre (I), en particulier CuI ou Cu(OTf), ledit catalyseur étant plus particulièrement CuSO₄, Cu(acac)₂, Cu(tBuSalen)₂ ou Cu(OTf)₂.

Selon un mode de réalisation avantageux, la présente invention concerne un procédé de préparation d'un composé répondant à la formule (I-C) suivante 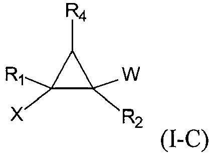 dans laquelle :
▪ X représente un atome de bore substitué choisi dans le groupe comprenant B(OH)₂, B(OR)₂, BF₃M, B(OR')₃M dans lesquels :
   o R est un groupe alkyle comportant de 1 à 14 atomes de carbone ou un groupe aryle, éventuellement substitués, ou est tel que (OR)₂ forme un cycle entre les deux atomes d'oxygène, (OR)₂ étant notamment choisi dans le groupe comprenant les radicaux bivalents dérivant de diols, tels que O-CH₂-CH₂-O, O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O et ses esters, et les radicaux bivalents dérivant de diacides, tels que OCO-CH₂-N(CH₃)-CH₂-COO,
   ∘ R' est un groupe alkyle comportant de 1 à 14 atomes de carbone ou est tel que :
      - (OR')₃ forme un cycle entre deux des atomes d'oxygène, (OR')₃ étant alors sous la forme OR'(OR)₂, où R' est un groupe alkyle comportant de 1 à 14 atomes de carbone et (OR)₂ est tel que défini ci-dessus, ou
      - (OR')₃ forme un bicycle entre les trois atomes d'oxygène, (R')₃ étant notamment choisi dans le groupe comprenant les radicaux trivalents dérivant de triols, tels que H₃C-C-(CH₂-O)₃,
   ∘ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} R^{e}, R^{f} sont choisis parmi H ou une chaîne carbonée saturée comportant notamment 1 à 6 atomes de carbone choisis indépendamment les uns des autres,
      et notamment X représente B(OH)₂, B(OR)₂ ou BF₃K,
▪ R₁ et R₄ identiques ou différents sont choisis dans le groupe constitué:
   1. de H
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   l'un au moins des groupes R₁ et R₄ représentant H,
▪ R₂ est choisi dans le groupe constitué par H et les groupes -COR^{a}, -COOR^{a}, -CONH₂, - CONHR^{a}, -CONR^{a}R^{b} et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a}, - CONH₂, -CONHR^{a}, -CONR^{a}R^{b},
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués ;
   ledit procédé comprenant :
   - une étape de réaction entre :
      ▪ un dérivé diazoïque de formule suivante : 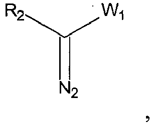 dans laquelle R₂ est tel que défini ci-dessus, W₁ étant choisi dans le groupe constitué de -COOR^{a}, -CONH₂, -CONHR³, et -CONR^{a}R^{b},
         et
      ▪ un composé vinyltrifluoroborate de formule suivante : 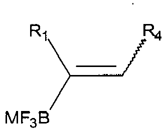 dans laquelle R₁, R₄ et M sont tels que définis ci-dessus,
         en présence d'un catalyseur contenant un métal de transition, ledit catalyseur étant un complexe de palladium (II), plus particulièrement Pd(OAc)₂ ou Pd(acac)₂, ou
         un complexe du cuivre (II), plus particulièrement CuSO₄, Cu(acac)₂, Cu(tBuSalen)₂, Cu(OTf)₂,
         ledit catalyseur étant en particulier un complexe de palladium (II), plus particulièrement Pd(OAc)₂ ou Pd(acac)₂, lorsque R₁ représente H,
         ledit catalyseur étant en particulier un complexe du cuivre (II), en particulier CuSO₄, Cu(acac)₂, Cu(tBuSalen)₂, Cu(OTf)₂, lorsque R₄ représente H,
      pour obtenir un composé de formule suivante : 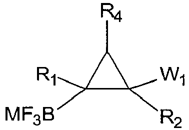 dans laquelle R₁, R₂, R₄, W₁ et M sont tels que définis ci-dessus,
   si W est différent de W₁ et/ou X est différent de MF₃B, ledit procédé comprenant en outre les étapes suivantes :
   - une étape de conversion de W₁ en W permettant d'obtenir 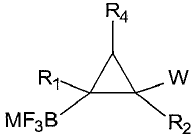 à partir de 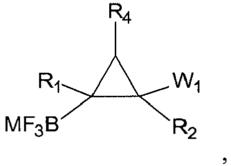 en particulier, lorsque W₁ = COOR^{a} et W = CHO, par réduction pour former l'alcool correspondant, puis oxydation dudit alcool, lorsque W₁ = -CONH₂ et W = - CONHSO₂R^{a}, par action du chlorure de sulfonyle ClSO₂R^{a} sur l'amide, et
   - une étape de conversion de -BF₃M en -X permettant d'obtenir 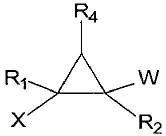 à partir de 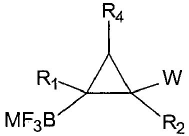 en particulier, lorsque X = B(OH)₂, par hydrolyse basique ou acide, ou par passage par un dihalogénoborane, plus particulièrement un dichloroborane, lorsque X = B(OR)₂, par passage par X = B(OH)₂ comme décrit précédemment puis par action d'un alcool, en particulier un alcool de formule ROH, un diol ou un triol, ou par passage par un dihalogénoborane, plus particulièrement un dichloroborane, puis par action d'un alcool, en particulier un alcool de formule ROH, un diol ou un triol,
      ou
   - une étape de conversion de -BF₃M en -X permettant d'obtenir 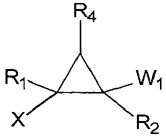 à partir de 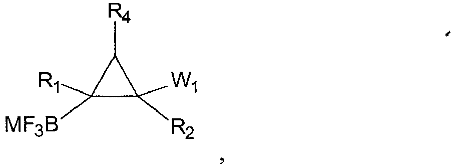 et
   - une étape de conversion de W₁ en W permettant d'obtenir 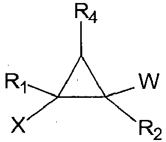 à partir de

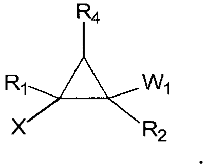

Selon un mode de réalisation avantageux, la présente invention concerne un procédé de préparation d'un composé répondant à la formule (I-D) suivante 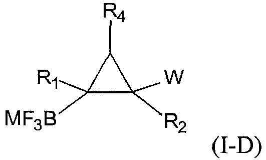 dans laquelle :
▪ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} R^{e}, R^{f} sont choisis parmi H ou une chaîne carbonée saturée comportant notamment 1 à 6 atomes de carbone choisis indépendamment les uns des autres,
   BF₃M représentant notamment BF₃K,
▪ R₁ et R₄ identiques ou différents sont choisis dans le groupe constitué:
   1. de H
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   l'un au moins des groupes R₁ et R₄ représentant H,
▪ R₂ est choisi dans le groupe constitué par H et les groupes -COR^{a}, -COOR^{a}, -CONH₂, - CONHR^{a}, -CONR^{a}R^{b} et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a}, - CONH₂, -CONHR^{a}, -CONR^{a}R^{b},
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués ;
   ledit procédé comprenant :
   - une étape de réaction entre :
      ▪ un dérivé diazoïque de formule suivante : 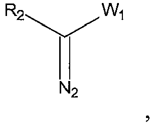 dans laquelle R₂ est tel que défini ci-dessus, W₁ étant choisi dans le groupe constitué de -COOR^{a}, -CONH₂, -CONHR^{a}, et -CONR^{a}R^{b},
         et
      ▪ un composé vinyltrifluoroborate de formule suivante : 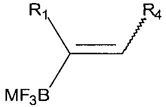 dans laquelle R₁, R₄ et M sont tels que définis ci-dessus,
      en présence d'un catalyseur contenant un métal de transition, ledit catalyseur étant un complexe de palladium (II), plus particulièrement Pd(OAc)₂ ou Pd(acac)₂, ou
      un complexe du cuivre (II), plus particulièrement CuSO₄, Cu(acac)₂, Cu(tBuSalen)₂, Cu(OTf)₂,
      ledit catalyseur étant en particulier un complexe de palladium (II), plus particulièrement Pd(OAc)₂ ou Pd(acac)₂, lorsque R₁ représente H,
      ledit catalyseur étant en particulier un complexe du cuivre (II), en particulier CuSO₄, Cu(acac)₂, Cu(tBuSalen)₂, Cu(OTf)₂, lorsque R₄ représente H,
      pour obtenir un composé de formule suivante : 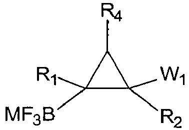 dans laquelle R₁, R₂, R₄, W₁ et M sont tels que définis ci-dessus,
      si W est différent de W₁, ledit procédé comprenant en outre les étapes suivantes :
   - une étape de conversion de W₁ en W permettant d'obtenir 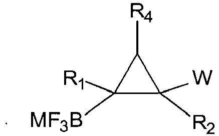 à partir de 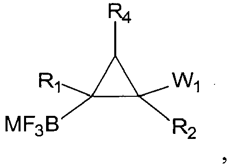 en particulier, lorsque W₁ = COOR^{a} et W = CHO, par réduction pour former l'alcool correspondant, puis oxydation dudit alcool, lorsque W₁ = -CONH₂ et W = - CONHSO₂R^{a}, par action du chlorure de sulfonyle ClSO₂R^{a} sur l'amide.

Selon un mode de réalisation avantageux, la présente invention concerne un procédé de préparation d'un composé de formule (I-C), dans laquelle :
▪ lorsque R₁, R₂ et R₄ représentent H et que B représente B(OH)₂, B(OR)₂, ou B(OR')₃M, alors W est choisi parmi -COR^{a}, -CONH₂, -CONHR^{a}, -CONR^{a}R^{b},
▪ lorsque W représente -COOH ou -COOR^{a}, et que B représente B(OH)₂, B(OR)₂, ou B(OR')₃M, alors :
   ∘ R₁ ne représente pas H, ou
   ∘ R₂ et R₄ ne représentent pas H.

Selon un mode de réalisation avantageux, la présente invention concerne un procédé de préparation d'un composé de formule (I-C) ou (I-D), dans laquelle W₁ représente -COOR^{a}, R^{a} étant tel que défini précédemment, R^{a} représentant en particulier un alkyle, un cycloalkyle, un benzyle, éventuellement substitués.

Selon un mode de réalisation avantageux, la présente invention concerne un procédé de préparation d'un composé de formule (I-C) ou (I-D) dans lequel W représente un groupe fonctionnel choisi parmi -CHO, -COR^{a}, -COOH et -COOR^{a}, W₁ représentant en particulier - COOR^{a}, R^{a} étant tel que défini précédemment, R^{a} représentant en particulier un alkyle, un cycloalkyle, un benzyle, éventuellement substitués.

Selon un mode de réalisation avantageux, la présente invention concerne un procédé de préparation d'un composé de formule (I-C) ou (I-D) dans lequel W et W₁ représentent - COOR^{a}, R^{a} étant tel que défini précédemment, R^{a} représentant en particulier un alkyle, un cycloalkyle, un benzyle, éventuellement substitués.

Selon un mode de réalisation avantageux, la présente invention concerne un procédé de préparation d'un composé de formule (I-C) ou (I-D) dans lequel R₂ représente H.

Selon un mode de réalisation avantageux, la présente invention concerne un procédé de préparation d'un composé de formule (I-C) ou (I-D) dans lequel R₂ est choisi dans le groupe constitué par les groupes -COOR^{a}, -CONH₂, -CONHR^{a} et -CONR^{a}R^{b},
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation d'un composé de formule (I-C) ou (I-D) dans lequel R₁, R₂ et R₄ représentent H.

Selon un mode de réalisation avantageux, la présente invention concerne un procédé de préparation d'un composé de formule (I-C) ou (I-D) dans lequel R₁ représente H.

Selon un mode de réalisation avantageux, la présente invention concerne un procédé de préparation d'un composé de formule (I-C) ou (I-D) dans lequel R₁ représente H et R₄ représente un aryle, un hétérocycle, un hétéroaryle ou un alkyle tels que définis précédemment.

Selon un mode de réalisation avantageux, la présente invention concerne un procédé de préparation d'un composé de formule (I-C) ou (I-D) dans lequel R₄ représente H.

Selon un mode de réalisation avantageux, la présente invention concerne un procédé de préparation d'un composé de formule (I-C) ou (I-D) dans lequel R₄ représente H et R₁ représente un aryle, un hétérocycle, un hétéroaryle ou un alkyle tels que définis précédemment.

Selon un mode de réalisation avantageux, la présente invention concerne un procédé de préparation d'un composé de formule (I-C) ou (I-D) dans lequel R₁ et R₂ représentent H et R₄ représente un aryle, un hétérocycle, un hétéroaryle ou un alkyle tels que définis précédemment:

Selon un mode de réalisation avantageux, la présente invention concerne un procédé de préparation d'un composé de formule (I-C) ou (I-D) dans lequel R₂ et R₄ représentent H et R₁ représente un aryle, un hétérocycle, un hétéroaryle ou un alkyle tels que définis précédemment.

Selon un mode de réalisation avantageux, la présente invention concerne un procédé de préparation d'un composé de formule (I-C) ou (I-D) dans lequel R₁représente H, R₄ représente un aryle, un hétérocycle, un hétéroaryle ou un alkyle tels que définis précédemment, et R₂ est choisi dans le groupe constitué par les groupes -COR^{a}, -COOR^{a}, - CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation d'un composé de formule (I-C) ou (I-D) dans lequel R₄ représente H, R₁ représente un aryle, un hétérocycle, un hétéroaryle ou un alkyle tels que définis précédemment, et R₂ est choisi dans le groupe constitué par les groupes -COR^{a}, -COOR^{a}, - CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation d'un composé de formule (I-C) ou (I-D) dans lequel R₁ et R₄ représentent H, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un procédé de préparation, dans laquelle W représente -COOR^{a}, -CONH₂, -CONHR^{a}, -CONR^{a}R^{b}, -CH₂OH, -CH₂OR^{a}, -CHR^{b}OH, -CHR^{b}OR^{a}, -CR^{b}R^{b'}OH, -CR^{b}R^{b'}OR^{a}, -CH₂NH₂, -CH₂NHZ, - CHR^{a}NHZ, ledit procédé comprenant :
- une étape de réaction entre :
   un dérivé diazoïque de formule suivante : 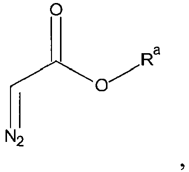 dans laquelle R^{a} est tel que défini précédemment,
      et
   un composé vinyltrifluoroborate de formule suivante : 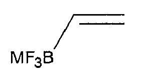 dans laquelle M est tel que défini précédemment, M représentant en particulier K, en présence d'un catalyseur contenant un métal de transition, pour obtenir un composé de formule suivante : 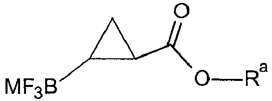 dans laquelle R^{a} et M sont tels que définis ci-dessus,
si W est différent de -COOR^{a} et/ou X est différent de MF₃B, ledit procédé comprenant en outre les étapes suivantes :
- une étape de conversion de -COOR^{a} en W permettant d'obtenir 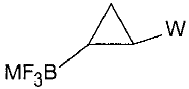 à partir de 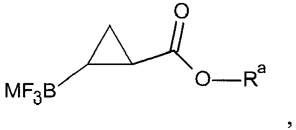 et
- une étape de conversion de -BF₃M en -X permettant d'obtenir 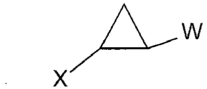 à partir de 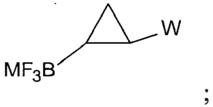 ou
- une étape de, conversion de -BF₃M en -X permettant d'obtenir 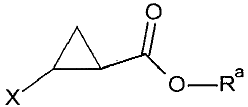 à partir de 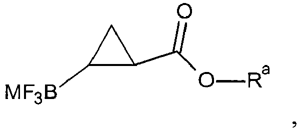 et
- une étape de conversion de -COOR^{a} en W permettant d'obtenir 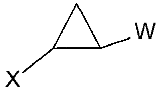 à partir de

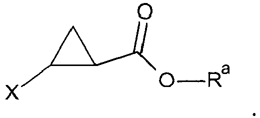

Selon un mode de réalisation avantageux, la présente description concerne un procédé de préparation d'un composé de formule (I), sous la forme d'un composé *trans* racémique de formule (I-1) : 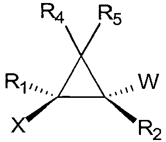 et son énantiomère, (I-1) dans laquelle X, R₁, R₂, R₄, R₅ et W sont tels que définis précédemment, ledit procédé comprenant :
- une étape de réaction entre :
   ▪ un dérivé diazoïque de formule suivante : 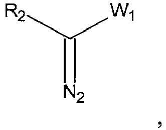 dans laquelle R₂ est tel que défini ci-dessus, W₁ étant choisi dans le groupe constitué de -COR^{a}, -COOR^{a}, -CONH₂, -CONHR^{a}, et -CONR^{a}R^{b},
      et
   ▪ un composé vinyltrifluoroborate de formule suivante : 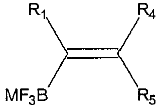 dans laquelle R₁, R₄, R₅ et M sont tels que définis ci-dessus,
      en présence d'un catalyseur contenant un métal de transition, pour obtenir un composé de formule suivante : 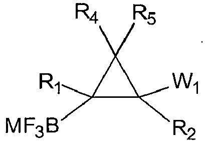 dans laquelle R₁, R₂, R₄, R₅, W₁ et M sont tels que définis ci-dessus, sous la forme d'un mélange comprenant les couples d'énantiomères de formules suivantes : 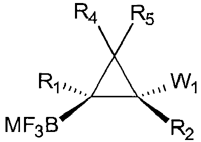 et son énantiomère, et 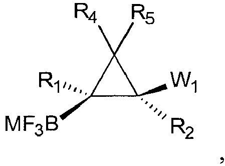 et son énantiomère,
- une étape de séparation desdits couples d'énantiomères, en particulier par recristallisation, pour obtenir le couple d'énantiomères *trans* suivant : 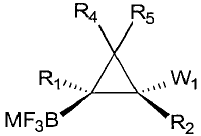 et son énantiomère,
si W est différent de W₁ et/ou X est différent de MF₃B, ledit procédé comprenant en outre les étapes suivantes :
- une étape de conversion de W₁ en W permettant d'obtenir 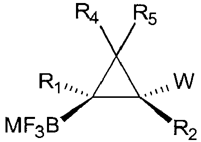 et son énantiomère à partir de 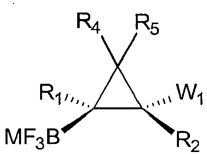 et son énantiomère, et
- une étape de conversion de -BF₃M en -X permettant d'obtenir 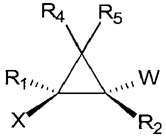 et son énantiomère à partir de 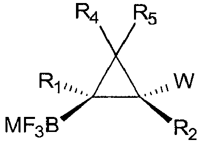 et son énantiomère
   ou
- une étape de conversion de -BF₃M en -X permettant d'obtenir 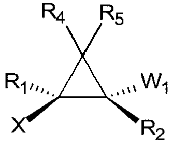 et son énantiomère à partir de 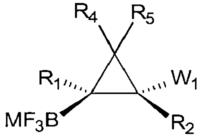 et son énantiomère, et
- une étape de conversion de W₁ en W permettant d'obtenir 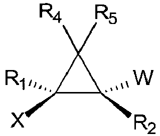 et son énantiomère à partir de 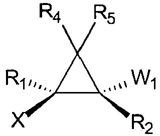 et son énantiomère.

Le terme « *trans* » s'entend relativement au groupe X et au groupe W, et signifie que lesdits groupes X et W ne sont pas dans le même demi-espace, par rapport au plan défini par le cyclopropyle.

Par exemple le composé [(1S,2S)-2-éthoxycarbonylcyclopropyl]-trifluoroborate de potassium de formule suivante : 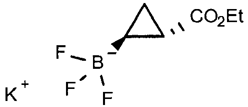 est *trans*.

Le terme *« trans »* s'entend pour un couple d'énantiomères. Les deux composés suivants : 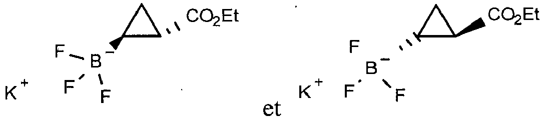 sont *trans*.

Ainsi, lorsqu'est mentionné un composé « *trans* » de formule suivante : 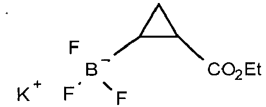 il est fait référence au couple d'énantiomères suivant :

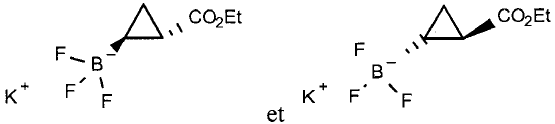

Lorsque R₂ = W, par exemple R₂ = W = -COOR^{a}, la notion de « *trans* » ne peut être définie, et on considère le couple d'énantiomères suivant :

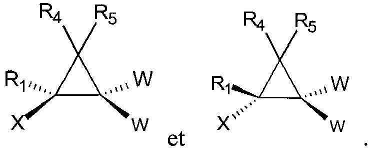

Selon un mode de réalisation avantageux, la présente invention concerne un procédé de préparation d'un composé de formule (I), dans lequel R₁, R₂, R₄ et R₅ représentent H, sous la forme d'un composé *trans* racémique de formule (I-1a) : 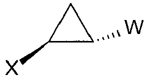 et son énantiomère, (I-1a) dans laquelle X et W sont tels que définis précédemment, ledit procédé comprenant :
- une étape de réaction entre :
   un dérivé diazoïque de formule suivante : 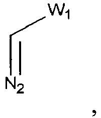
      W₁ étant choisi dans le groupe constitué de -COR^{a}, -COOR^{a}, -CONH₂, -CONHR^{a}, et -CONR^{a}R^{b}, W₁ représentant en particulier -COOR^{a},
      et
   un composé vinyltrifluoroborate de formule suivante : 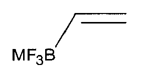 dans laquelle M est tel que défini ci-dessus,
   en présence d'un catalyseur contenant un métal de transition, pour obtenir un composé de formule suivante : 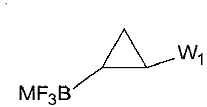 dans laquelle W₁ et M sont tels que définis ci-dessus, sous la forme d'un mélange comprenant les couples d'énantiomères de formules suivantes : 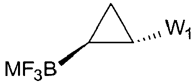 et son énantiomère, et 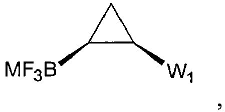 et son énantiomère,
- une étape de séparation desdits couples d'énantiomères, en particulier par recristallisation, pour obtenir le couple d'énantiomères *trans* suivant : 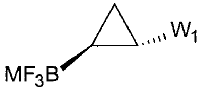 et son énantiomère,
si W est différent de W₁ et/ou X est différent de MF₃B, ledit procédé comprenant en outre les étapes suivantes :
- une étape de conversion de W₁ en W permettant d'obtenir 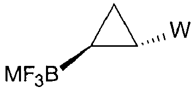 et son énantiomère à partir de 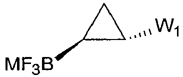 et son énantiomère,
   et
- une étape de conversion de -BF₃M en -X permettant d'obtenir 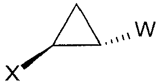 et son énantiomère à partir de 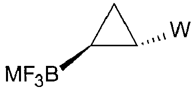 et son énantiomère
   ou
- une étape de conversion de -BF₃M en -X permettant d'obtenir 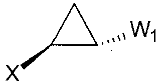 et son énantiomère à partir de 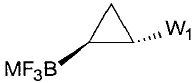 et son énantiomère,
   et
- une étape de conversion de W₁ en W permettant d'obtenir 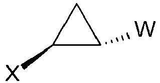 et son énantiomère à partir de 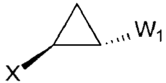 et son énantiomère.

Selon un mode de réalisation avantageux, la présente description concerne un procédé de préparation d'un composé de formule (I), sous la forme d'un composé *cis* racémique de formule (I-2) : 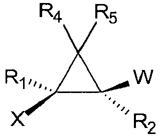 et son énantiomère, (I-2) dans laquelle X, R₁, R₂, R₄, R₅ et W sont tels que définis précédemment, ledit procédé comprenant :
- une étape de réaction entre :
   ▪ un dérivé diazoïque de formule suivante : 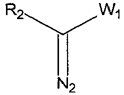 dans laquelle R₂ est tel que défini ci-dessus, W₁ étant choisi dans le groupe constitué de -COR^{a}, -COOR^{a}, -CONH₂, -CONHR^{a}, et -CONR^{a}R^{b},
      et
   ▪ un composé vinyltrifluoroborate de formule suivante : 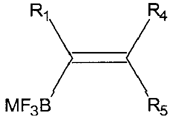 dans laquelle R₁, R₄, R₅ et M sont tels que définis ci-dessus,
   en présence d'un catalyseur contenant un métal de transition, pour obtenir un composé de formule suivante : 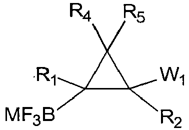 dans laquelle R₁, R₂, R₄, R₅, W₁ et M sont tels que définis ci-dessus, sous la forme d'un mélange comprenant les couples d'énantiomères de formules suivantes : 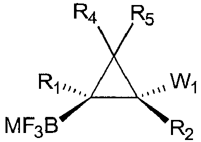 et son énantiomère, et 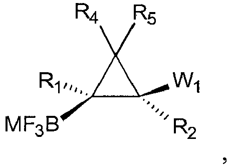 et son énantiomère,
- une étape de séparation desdits couples d'énantiomères, en particulier par recristallisation, pour obtenir le couple d'énantiomères *trans* suivant : 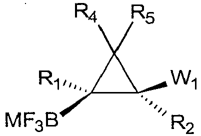 et son énantiomère,
si W est différent de W₁ et/ou X est différent de MF₃B, ledit procédé comprenant en outre les étapes suivantes :
- une étape de conversion de W₁ en W permettant d'obtenir 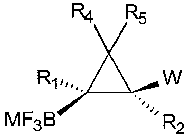 et son énantiomère à partir de 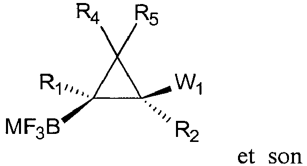 énantiomère, et
- une étape de conversion de -BF₃M en -X permettant d'obtenir 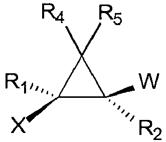 et son énantiomère à partir de 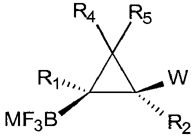 et son énantiomère,
   ou
- une étape de conversion de -BF₃M en -X permettant d'obtenir 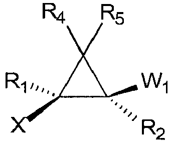 et son énantiomère à partir de 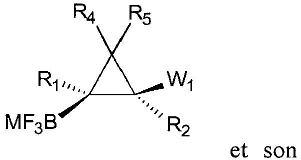 énantiomère,
   et
- une étape de conversion de W₁ en W permettant d'obtenir 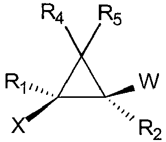 et son énantiomère à partir de 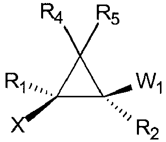 et son énantiomère.

Le terme « *cis* » s'entend relativement au groupe X et au groupe W, et signifie que lesdits groupes X et W sont dans le même demi-espace, par rapport au plan défini par le cyclopropyle.

### Par exemple le composé [(1S,2R)-2-éthoxycarbonylcyclopropyl]-trifluoroborate de potassium de formule suivante :

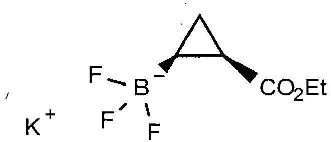

est *cis*.

Le terme « *cis* » s'entend pour un couple d'énantiomères. Les deux composés suivants : 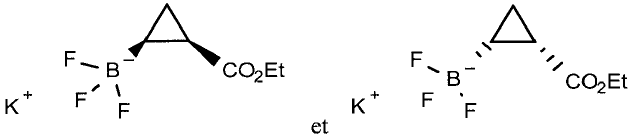 sont *cis*.

Ainsi, lorsqu'est mentionné un composé « *cis* » de formule suivante : 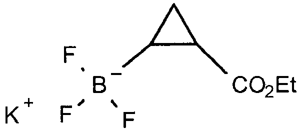 il est fait référence au couple d'énantiomères suivant :

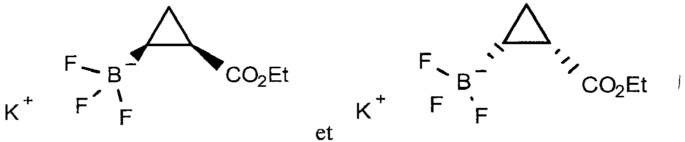

Lorsque R₂ = W, par exemple R₂ = W = -COOR^{a}, la notion de « *cis* » ne peut être définie, et on considère le couple d'énantiomère suivant :

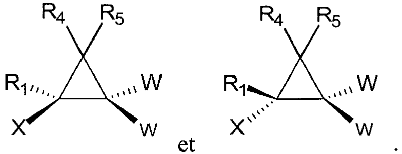

Selon un mode de réalisation avantageux, la présente invention concerne un procédé de préparation d'un composé de formule (I), dans lequel R₁, R₂, R₄ et R₅ représentent H, sous la forme d'un composé *cis* racémique de formule (I-2a) : 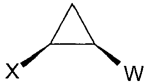 et son énantiomère, (I-2a) dans laquelle X et W sont tels que définis précédemment, ledit procédé comprenant :
- une étape de réaction entre :
   un dérivé diazoïque de formule suivante : 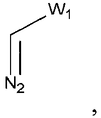 W₁ étant choisi dans le groupe constitué de -COR^{a}, -COOR^{a}, -CONH₂, -CONHR^{a}, et -CONR^{a}R^{b}, W₁ représentant en particulier -COOR^{a},
      et
   un composé vinyltrifluoroborate de formule suivante : 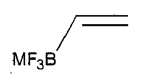
   dans laquelle M est tel que défini ci-dessus,
   en présence d'un catalyseur contenant un métal de transition, pour obtenir un composé de formule suivante : 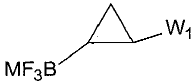 dans laquelle W₁ et M sont tels que définis ci-dessus, sous la forme d'un mélange comprenant les couples d'énantiomères de formules suivantes : 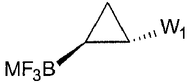 et son énantiomère, et 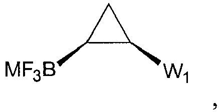 et son énantiomère,
- une étape de séparation desdits couples d'énantiomères, en particulier par recristallisation, pour obtenir le couple d'énantiomères *cis* suivant : 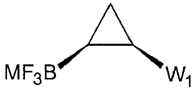 et son énantiomère,
si W est différent de W₁ et/ou X est différent de MF₃B, ledit procédé comprenant en outre les étapes suivantes :
- une étape de conversion de W₁ en W permettant d'obtenir 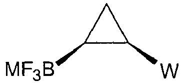 et son énantiomère à partir de 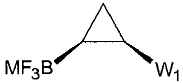 et son énantiomère,
   et
- une étape de conversion de -BF₃M en -X permettant d'obtenir 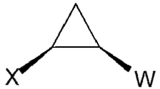 et son énantiomère à partir de 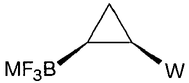 et son énantiomère,
   ou
- une étape de conversion de -BF₃M en -X permettant d'obtenir 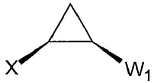 et son énantiomère à partir de 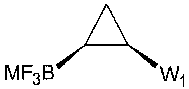 et son énantiomère,
   et
- une étape de conversion de W₁ en W permettant d'obtenir 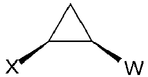 et son énantiomère à partir de 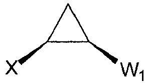 et son énantiomère.

Selon un mode de réalisation avantageux, la présente description concerne un procédé de préparation d'un composé répondant à la formule (I), dans laquelle W représente un groupe fonctionnel choisi parmi -OH, -OR^{a}, -OZ', -NH₂, -NHR^{a}, -NR^{a}R^{b}, -NHZ et -NZZ₂, en particulier parmi -OH, -OR^{a} et -OZ', ledit procédé comprenant :
- le traitement d'un composé de formule suivante : 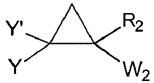 dans laquelle R₂ est tel que défini précédemment, R₂ représentant en particulier H, et dans laquelle W₂ représente un groupe fonctionnel choisi parmi -OR^{a}, -OZ', -NR^{a}R^{b} et -NZZ₂, en particulier parmi -OR^{a} et -OZ', Y étant un halogénure, en particulier -Br, Y' étant un halogénure, en particulier -Br, ou H,
   par :
   - une base forte, en particulier un alkyl lithium, plus particulièrement le n-butyllithium ou *sec*-butyllithium, puis
   - un composé de formule X"-B(OR)₂, R étant tel que défini ci-dessus, X" représentant H, un groupe O-alkyle comportant de 1 à 14 atomes de carbone ou un groupe O-aryle éventuellement substitués,
   pour obtenir un composé de formule suivante : 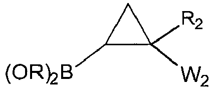 dans laquelle R, R₂ et W₂ sont tels que définis ci-dessus,
   ou
- une réaction de type Simmons-Smith à partir d'un composé de formule suivante : 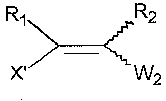 dans laquelle R₁ et R₂ sont tels que définis précédemment, R₁ et R₂ représentant en particulier H, W₂ représentant un groupe fonctionnel choisi parmi -OR^{a}, -OZ', -NR^{a}R^{b} et -NZZ₂, en particulier parmi -OR^{a} et -OZ', X' représentant B(OR)₂ ou BF₃M, en particulier B(OR)₂, dans lesquels R et M sont tels que définis précédemment,
   pour obtenir un composé de formule suivante : 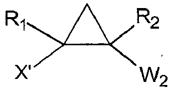 dans laquelle R₁, R₂, X' et W₂ sont tels que définis ci-dessus,
si W représente -OH ou -NH₂, et/ou X est différent de B(OR)₂, ledit procédé comprenant en outre les étapes suivantes, R₁ représentant en particulier H:
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 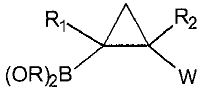 à partir de 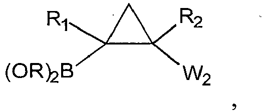 et
- une étape de conversion de B(OR)₂ en -X permettant d'obtenir 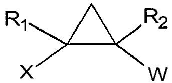 à partir de 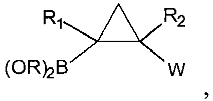 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   ou
- une étape de conversion de B(OR)₂ en -X permettant d'obtenir 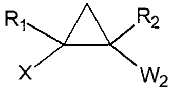 à partir de 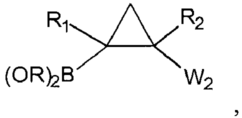 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   et
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 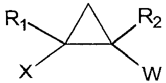 à partir de 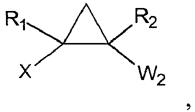 ou, lorsque X' représente BF₃M, W représentant -OH ou NH₂,
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 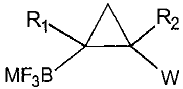 à partir de

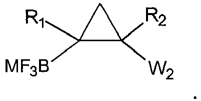

Selon un mode de réalisation avantageux, la présente description concerne un procédé de préparation d'un composé répondant à la formule (I), dans laquelle W représente un groupe fonctionnel W₂ choisi parmi -OR^{a}, -OZ', -NR^{a}R^{b} et -NZZ₂, en particulier parmi -OR^{a} et - OZ', ledit procédé comprenant :
- le traitement d'un composé de formule suivante : 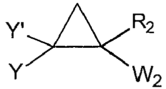
   dans laquelle R₂ est tel que défini précédemment,
   et dans laquelle W₂ est tel que décrit ci-dessus, Y étant un halogénure, en particulier - Br, Y' étant un halogénure, en particulier-Br, ou H,
   par :
   - une base forte, en particulier le butyllithium, plus particulièrement le n-butyllithium, puis
   - un composé de formule X"-B(OR)₂, R étant tel que défini ci-dessus, X" représentant H, un groupe O-alkyle comportant de 1 à 14 atomes de carbone ou un groupe O-aryle éventuellement substitués,
   pour obtenir un composé de formule suivante : 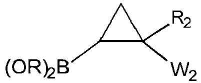 dans laquelle R et W₂ sont tels que définis ci-dessus,
   ou
- une réaction de type Simmons-Smith à partir d'un composé de formule suivante : 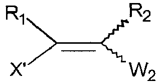 dans laquelle W₂, R₁ et R₂ sont tels que définis précédemment, X' représentant B(OR)₂ ou BF₃M, en particulier B(OR)₂, dans lesquels R et M sont tels que définis précédemment,
   pour obtenir un composé de formule suivante : 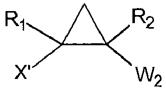 dans laquelle R₁, R₂, X' et W₂ sont tels que définis ci-dessus.

Selon un mode de réalisation avantageux, la présente description concerne un procédé de préparation d'un composé répondant à la formule (I), dans laquelle W représente un groupe fonctionnel -OH ou -NH₂, en particulier -OH:
- le traitement d'un composé de formule suivante : 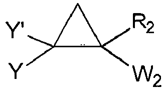
   dans laquelle R₂ est tel que défini précédemment,
   et dans laquelle W₂ représente un groupe fonctionnel choisi parmi -OR^{a}, -OZ' et - NZZ₂, en particulier parmi -OR^{a} et -OZ', W₂ représentant en particulier -OZ', Y étant un halogénure, en particulier -Br, Y' étant un halogénure, en particulier -Br, ou H,
   par :
   - une base forte, en particulier un alkyl lithium, plus particulièrement le n-butyllithium ou *sec*-butyllithium, puis
   - un composé de formule X"-B(OR)₂, R étant tel que défini ci-dessus, X" représentant H, un groupe O-alkyle comportant de 1 à 14 atomes de carbone ou un groupe O-aryle éventuellement substitués,
   pour obtenir un composé de formule suivante : 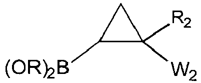 dans laquelle R et W₂ sont tels que définis ci-dessus,
   ou
- une réaction de type Simmons-Smith à partir d'un composé de formule suivante : 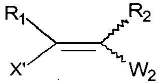 dans laquelle R₁ et R₂ sont tels que définis précédemment, W₂ représentant un groupe fonctionnel choisi parmi -OR^{a}, -OZ' et -NZZ₂, en particulier parmi -OR^{a} et -OZ', W₂ représentant en particulier -OZ', X' représentant B(OR)₂ ou BF₃M, en particulier B(OR)₂, dans lesquels R et M sont tels que définis précédemment,
   pour obtenir un composé de formule suivante : 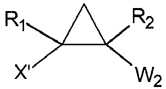 dans laquelle R₁, R₂, X' et W₂ sont tels que définis ci-dessus,
ledit procédé comprenant en outre l'étape suivante, R₁ représentant en particulier H :
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 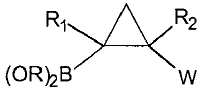 à partir de 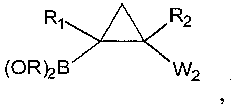 ou, lorsque X' représente BF₃M,
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 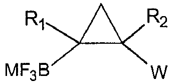 à partir de

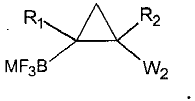

Selon un mode de réalisation avantageux, la présente description concerne un procédé de préparation d'un composé répondant à la formule (I), dans laquelle W représente un groupe fonctionnel W₂ choisi parmi -OR^{a}, -OZ', -NR^{a}R^{b} et -NZZ₂, en particulier parmi -OR^{a} et - OZ', ledit procédé comprenant :
- le traitement d'un composé de formule suivante : 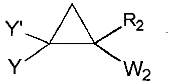
   dans laquelle R₂ est tel que défini précédemment,
   et dans laquelle W₂ est tel que décrit ci-dessus, Y étant un halogénure, en particulier - Br, Y' étant un halogénure, en particulier -Br, ou H, par :
      - une base forte, en particulier un alkyl lithium, plus particulièrement le n-butyllithium ou *sec*-butyllithium puis
      - un composé de formule X"-B(OR)₂, R étant tel que défini ci-dessus, X" représentant H, un groupe O-alkyle comportant de 1 à 14 atomes de carbone ou un groupe O-aryle éventuellement substitués,
      pour obtenir un composé de formule suivante : 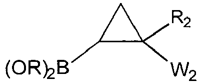 dans laquelle R et W₂ sont tels que définis ci-dessus,
   ou
- une réaction de type Simmons-Smith à partir d'un composé de formule suivante : 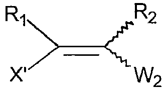 dans laquelle W₂, R₁ et R₂ sont tels que définis précédemment, X' représentant B(OR)₂ ou BF₃M, en particulier B(OR)₂, dans lesquels R et M sont tels que définis précédemment,
   pour obtenir un composé de formule suivante : 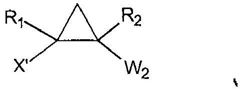 dans laquelle R₁, R₂, X' et W₂ sont tels que définis ci-dessus,
ledit procédé comprenant en outre l'étape suivante, R₁ représentant en particulier H :
- une étape de conversion de B(OR)₂ en X, X représentant B(OH)₂, BF₃M ou B(OR')₃M, permettant d'obtenir 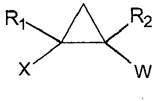 à partir de 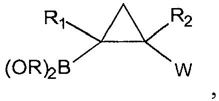 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂.

Selon un mode de réalisation avantageux, la présente description concerne un procédé de préparation d'un composé répondant à la formule (I), dans laquelle W représente un groupe fonctionnel -OH ou -NH₂, en particulier -OH ledit procédé comprenant :
- le traitement d'un composé de formule suivante : 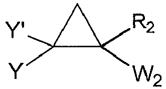
   dans laquelle R₂ est tel que défini précédemment,
   et dans laquelle W₂ représente un groupe fonctionnel choisi parmi -OR^{a}, -OZ' et - NZZ₂, en particulier parmi -OR^{a} et -OZ', W₂ représentant en particulier -OZ', Y étant un halogénure, en particulier -Br, Y' étant un halogénure, en particulier -Br, ou H, par :
      - une base forte, en particulier le butyllithium, plus particulièrement le n-butyllithium, puis
      - un composé de formule X"-B(OR)₂, R étant tel que défini ci-dessus, X" représentant H, un groupe O-alkyle comportant de 1 à 14 atomes de carbone ou un groupe O-aryle éventuellement substitués,
   pour obtenir un composé de formule suivante : 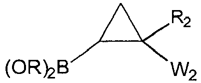 dans laquelle R et W₂ sont tels que définis ci-dessus,
   ou
- une réaction de type Simmons-Smith à partir d'un composé de formule suivante : 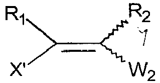 dans laquelle R₁ et R₂ sont tels que définis précédemment, W₂ représentant un groupe fonctionnel choisi parmi -OR^{a}, -OZ' et -NZZ₂, en particulier parmi -OR^{a} et -OZ', W₂ représentant en particulier -OZ', X' représentant B(OR)₂ ou BF₃M, en particulier B(OR)₂, dans lesquels R et M sont tels que définis précédemment,
   pour obtenir un composé de formule suivante : 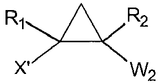 dans laquelle R₁, R₂, X' et W₂ sont tels que définis ci-dessus,
ledit procédé comprenant en outre les étapes suivantes, R₁ représentant en particulier H :
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 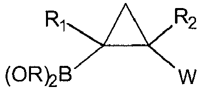 à partir de 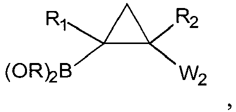 et
- une étape de conversion de B(OR)₂ en X, X représentant B(OH)₂, BF₃M ou B(OR')₃M, permettant d'obtenir 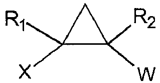 à partir de 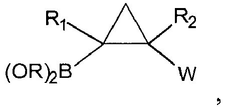 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   ou
- une étape de conversion de B(OR)₂ en X, X représentant B(OH)₂, BF₃M ou B(OR')₃M, permettant d'obtenir 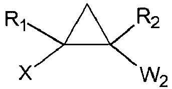 à partir de 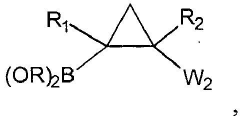 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   et
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 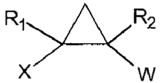 à partir de 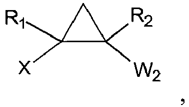 ou, lorsque X' représente BF₃M, W représentant -OH ou -NH₂,
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 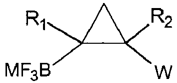 à partir de

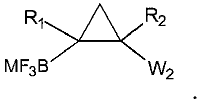

Selon un mode de réalisation avantageux, la présente description concerne un procédé de préparation d'un composé répondant à la formule (I), dans laquelle W représente un groupe fonctionnel choisi parmi -OH, -OR^{a}, -OZ', -NH₂, -NHR^{a}, -NR^{a}R^{b}, -NHZ et -NZZ₂, en particulier parmi -OH, -OR^{a} et -OZ', ledit procédé comprenant :
- le traitement d'un composé de formule suivante : 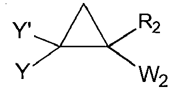
   dans laquelle R₂ est tel que défini précédemment,
   et dans laquelle W₂ représente un groupe fonctionnel choisi parmi -OR^{a}, -OZ', -NR^{a}R^{b} et -NZZ₂, en particulier parmi -OR^{a} et -OZ', Y étant un halogénure, en particulier -Br,
   Y' étant un halogénure, en particulier -Br, ou H,
      par :
      - une base forte, en particulier en particulier un alkyl lithium, plus particulièrement le n-butyllithium ou *sec*-butyllithium puis
      - un composé de formule X"-B(OR)₂, R étant tel que défini ci-dessus, X" représentant H, un groupe O-alkyle comportant de 1 à 14 atomes de carbone ou un groupe O-aryle éventuellement substitués,
   pour obtenir un composé de formule suivante : 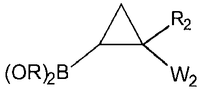 dans laquelle R, R₂ et W₂ sont tels que définis ci-dessus,
si W représente -OH ou -NH₂, et/ou X est différent de B(OR)₂, ledit procédé comprenant en outre les étapes suivantes:
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 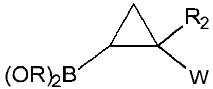 à partir de 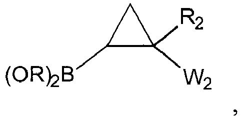 et
- une étape de conversion de B(OR)₂ en -X permettant d'obtenir 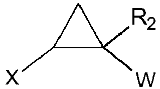 à partir de 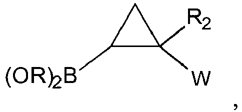 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   ou
- une étape de conversion de B(OR)₂ en -X permettant d'obtenir 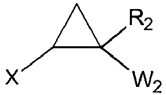 à partir de 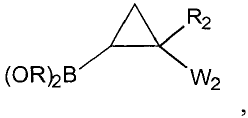 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   et
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 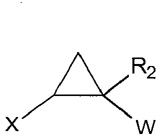 à partir de

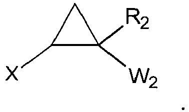

Selon un mode de réalisation avantageux, la présente description concerne un procédé de préparation, dans lequel R₂ représente H.

Selon un mode de réalisation avantageux, la présente description concerne un procédé de préparation, dans lequel R₂ représente un groupe alkyle linéaire, cyclique ou ramifié comportant de 1 à 15 atomes de carbone, éventuellement substitués.

Selon un mode de réalisation avantageux, la présente description concerne un procédé de préparation d'un composé répondant à la formule (I), dans laquelle W représente un groupe fonctionnel choisi parmi -OH, -OR^{a}, -OZ', -NH₂, -NHR^{a}, -NR^{a}R^{b}, -NHZ et -NZZ₂, en particulier parmi -OH, -OR^{a} et -OZ', ledit procédé comprenant :
- une réaction de type Simmons-Smith à partir d'un composé de formule suivante : 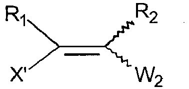 dans laquelle R₁ et R₂ sont tels que définis précédemment, W₂ représentant un groupe fonctionnel choisi parmi -OR^{a}, -OZ', -NR^{a}R^{b} et -NZZ₂, en particulier parmi -OR^{a} et-OZ', X' représentant B(OR)₂ ou BF₃M, en particulier B(OR)₂, dans lesquels R et M sont tels que définis précédemment,
   pour obtenir un composé de formule suivante : 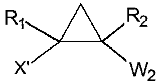 dans laquelle R₁, R₂, X' et W₂ sont tels que définis ci-dessus,
si W représente -OH ou -NH₂, et/ou X est différent de B(OR)₂, ledit procédé comprenant en outre les étapes suivantes:
lorsque X' représente B(OR)₂,
   - une étape de conversion de W₂ en W, en particulier la déprotection du groupement Z' lorsque W₂ représente -OZ' ou des groupements Z et Z₂ lorsque W₂ représente -NZZ₂, permettant d'obtenir 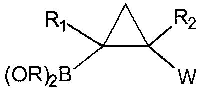 à partir de 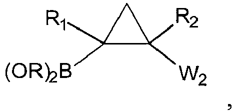 et
   - une étape de conversion de B(OR)₂ en -X permettant d'obtenir 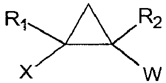 à partir de 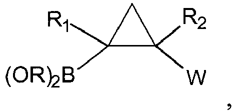 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
      ou
   - une étape de conversion de B(OR)₂ en -X permettant d'obtenir 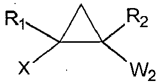 à partir de 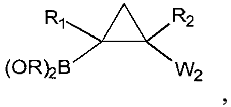 ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
      et
   - une étape de conversion de W₂ en W, en particulier la déprotection du groupement Z' lorsque W₂ représente -OZ' ou des groupements Z et Z₂ lorsque W₂ représente -NZZ₂, permettant d'obtenir 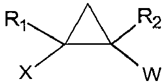 à partir de 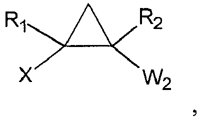 ou, lorsque X' représente BF₃M, W représentant -OH ou -NH₂,
   - une étape de conversion de W₂ en W, en particulier la déprotection du groupement Z' lorsque W₂ représente -OZ' ou des groupements Z et Z₂ lorsque W₂ représente -NZZ₂, permettant d'obtenir 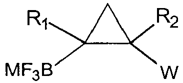 à partir de

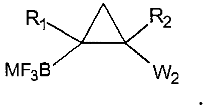

Selon un mode de réalisation avantageux, la présente description concerne un procédé de préparation, dans lequel R₁ et R₂ représentent H.

Selon un mode de réalisation avantageux, la présente description concerne un procédé de préparation, dans lequel R₁ et R₂ représentent indépendamment l'un de l'autre H ou un groupe alkyle linéaire, cyclique ou ramifié comportant de 1 à 15 atomes de carbone, éventuellement substitués.

Selon un mode de réalisation avantageux, la présente description concerne un procédé de préparation d'un composé répondant à la formule (I), dans laquelle W représente un groupe fonctionnel choisi parmi -OH, -OR^{a},-OZ', -NH₂, -NHR^{a}, -NR^{a}R^{b}, -NHZ et -NZZ₂, en particulier parmi -OH, -OR^{a} et -OZ',
sous la forme d'un composé *trans* racémique de formule (I-1c) : 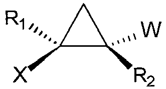 et son énantiomère, (I-1c) dans laquelle X et W sont tels que définis précédemment, ledit procédé comprenant :
- le traitement d'un composé de formule suivante : 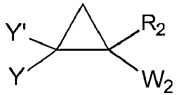
   dans laquelle R₂ est tel que défini précédemment,
   et dans laquelle W₂ représente un groupe fonctionnel choisi parmi -OR^{a}, -OZ', -NR^{a}R^{b} et -NZZ₂, en particulier parmi -OR^{a} et -OZ', Y étant un halogénure, en particulier -Br,
   Y' étant un halogénure, en particulier -Br,
   par :
   - une base forte, en particulier un alkyl lithium, plus particulièrement le n-butyllithium ou *sec*-butyllithium puis
   - un composé de formule X"-B(OR)₂, R étant tel que défini ci-dessus, X" représentant H, un groupe O-alkyle comportant de 1 à 14 atomes de carbone ou un groupe O-aryle éventuellement substitués,
   pour obtenir un composé de formule suivante : 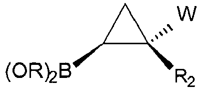 et son énantiomère, dans laquelle R et W sont tels que définis ci-dessus,
ou les étapes suivantes :
- le traitement d'un composé de formule suivante : 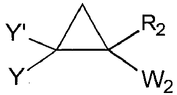
   dans laquelle R₂ est tel que défini précédemment,
   et dans laquelle W₂ représente un groupe fonctionnel choisi parmi -OR^{a}, -OZ', -NR^{a}R^{b} et -NHZ, en particulier parmi -OR^{a} et -OZ', Y étant un halogénure, en particulier -Br,
   Y' étant un halogénure, en particulier -Br,
   par une base forte, en particulier le butyllithium, plus particulièrement le n-butyllithium, pour obtenir un composé de formule suivante : 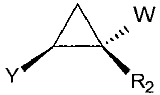 et son énantiomère, dans laquelle W est tel que défini ci-dessus,
- le traitement du composé de formule suivante : 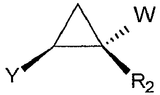 et son énantiomère, dans laquelle W est tel que défini ci-dessus,
   par :
   - une base forte, en particulier un alkyl lithium, plus particulièrement le n-butyllithium ou *sec*-butyllithium, puis
   - un composé de formule X"-B(OR)₂, R étant tel que défini ci-dessus, X" représentant H, un groupe O-alkyle comportant de 1 à 14 atomes de carbone ou un groupe O-aryle éventuellement substitués,
   pour obtenir un composé de formule suivante : 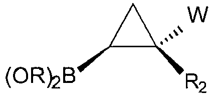 et son énantiomère, dans laquelle R et W sont tels que définis ci-dessus,
ou l'étape suivante :
- une réaction de type Simmons-Smith à partir d'un composé de formule suivante : 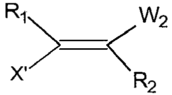 dans laquelle R₁ et R₂ sont tels que définis précédemment, W₂ représentant un groupe fonctionnel choisi parmi -OR^{a}, -OZ', -NR^{a}R^{b} et -NZZ₂, en particulier parmi -OR^{a} et-OZ',X' représentant B(O.R)₂ ou BF₃M, en particulier B(OR)₂, dans lesquels R et M sont tels que définis précédemment,
   pour obtenir un composé de formule suivante : 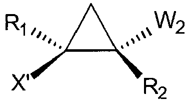 et son énantiomère, dans laquelle R₁, R₂, X' et W₂ sont tels que définis ci-dessus,
si W représente -OH ou NH₂, et/ou X est différent de B(OR)₂, ledit procédé comprenant en outre les étapes suivantes, R₁ représentant en particulier H:
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 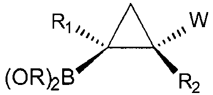 et son énantiomère à partir de 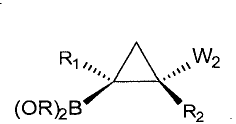 et son énantiomère,
   et
- une étape de conversion de B(OR)₂ en -X permettant d'obtenir 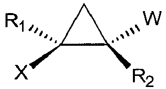 et son énantiomère à partir de 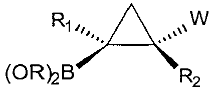 et son énantiomère,
   ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   ou
- une étape de conversion de B(OR)₂ en -X permettant d'obtenir 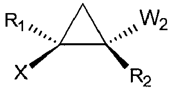 et son énantiomère à partir de 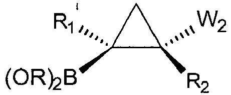 et son énantiomère,
   ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   et
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 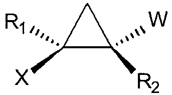 et son énantiomère à partir de 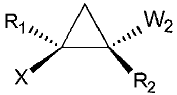 et son énantiomère, ou, lorsque X' représente BF₃M, W représentant -OH ou -NH₂,
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 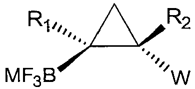 et son énantiomère à partir de 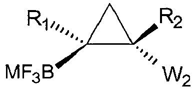 et son énantiomère.

Selon un mode de réalisation avantageux, la présente description concerne un procédé de préparation d'un composé répondant à la formule (I), dans laquelle W représente un groupe fonctionnel choisi parmi -OH, -OR^{a},-OZ', -NH₂, -NHR^{a}, -NR^{a}R^{b}, -NHZ et -NZZ₂, en particulier parmi -OH, -OR^{a} et -OZ', sous la forme d'un composé *trans* racémique de formule (I-1b) : 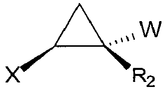 et son énantiomère, (I-1b) dans laquelle X et W sont tels que définis précédemment, ledit procédé comprenant :
- le traitement d'un composé de formule suivante : 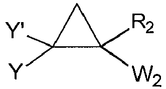
   dans laquelle R₂ est tel que défini précédemment,
   et dans laquelle W₂ représente un groupe fonctionnel choisi parmi -OR^{a}, -OZ', -NR^{a}R^{b} et -NZZ₂, en particulier parmi -OR^{a} et -OZ', Y étant un halogénure, en particulier -Br,
   Y' étant un halogénure, en particulier -Br,
      par :
      - une base forte, en particulier un alkyl lithium, plus particulièrement le n-butyllithium ou *sec*-butyllithium, puis
      - un composé de formule X"-B(OR)₂, R étant tel que défini ci-dessus, X" représentant H, un groupe O-alkyle comportant de 1 à 14 atomes de carbone ou un groupe O-aryle éventuellement substitués,
   pour obtenir un composé de formule suivante : 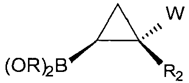 et son énantiomère, dans laquelle R et W sont tels que définis ci-dessus,
ou les étapes suivantes :
- le traitement d'un composé de formule suivante : 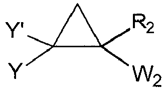
   dans laquelle R₂ est tel que défini précédemment,
   et dans laquelle W₂ représente un groupe fonctionnel choisi parmi -OR^{a}, -OZ', -NR^{a}R^{b} et -NZZ₂, en particulier parmi -OR^{a} et -OZ', Y étant un halogénure, en particulier -Br,
   Y' étant un halogénure, en particulier -Br,
   par une base forte, en particulier un alkyl lithium, plus particulièrement le n-butyllithium ou *sec*-butyllithium, pour obtenir un composé de formule suivante : 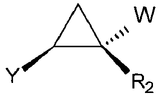 et son énantiomère, dans laquelle W est tel que défini ci-dessus,
- le traitement du composé de formule suivante : 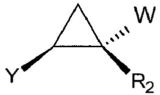 et son énantiomère, dans laquelle W est tel que défini ci-dessus,
   par :
   - une base forte, en particulier un alkyl lithium, plus particulièrement le n-butyllithium ou *sec*-butyllithium, puis
   - un composé de formule X"-B(OR)₂, R étant tel que défini ci-dessus, X" représentant H, un groupe O-alkyle comportant de 1 à 14 atomes de carbone ou un groupe O-aryle éventuellement substitués,
   pour obtenir un composé de formule suivante : 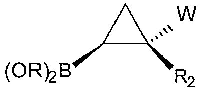 et son énantiomère, dans laquelle R et W sont tels que définis ci-dessus,
si W représente -OH ou -NH₂, et/ou X est différent de B(OR)₂, ledit procédé comprenant en outre les étapes suivantes:
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 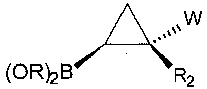 et son énantiomère à partir de 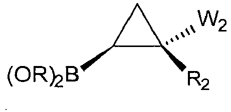 et son énantiomère,
   et
- une étape de conversion de B(OR)₂ en -X permettant d'obtenir 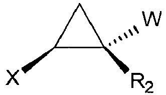 et son énantiomère à partir de 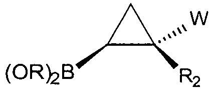 et son énantiomère,
   ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   ou
- une étape de conversion de B(OR)₂ en -X permettant d'obtenir 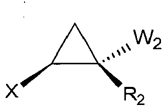 et son énantiomère à partir de 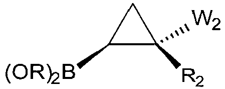 et son énantiomère, ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   et
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 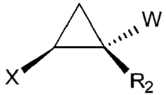 et son énantiomère à partir de 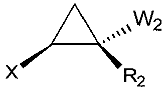 et son énantiomère,
   ou, lorsque X' représente BF₃M, W représentant -OH ou -NH₂,
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 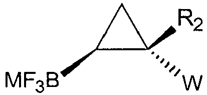 et son énantiomère à partir de 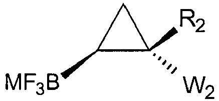 et son énantiomère.

Selon un mode de réalisation avantageux, la présente description concerne un procédé de préparation d'un composé répondant à la formule (I), dans laquelle W représente un groupe fonctionnel choisi parmi -OH, -OR^{a}, -OZ', -NH₂, -NHR^{a}, -NR^{a}R^{b}, -NHZ et -NZZ₂, en particulier parmi -OH, -OR^{a} et -OZ', sous la forme d'un composé *trans* racémique de formule (I-1c) : 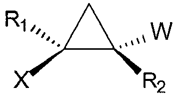 et son énantiomère, (I-1c) dans laquelle X et W sont tels que définis précédemment, ledit procédé comprenant :
- une étape de réaction entre un composé de formule suivante : 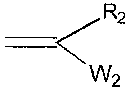 R₂ étant tel que défini précédemment, W₂ représentant un groupe fonctionnel choisi parmi -OR^{a}, -OZ', -NR^{a}R^{b} et -NZZ₂, en particulier parmi -OR^{a} et -OZ',
   et un composé de formule suivante: 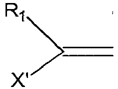 R₁ étant tel que défini précédemment, X' représentant B(OR)₂ ou BF₃M, en particulier B(OR)₂,
   en présence d'un catalyseur comprenant un métal de transition choisi dans le groupe constitué par le nickel, le molybdène, le tungstène, le ruthénium, en particulier le molybdène ou le ruthénium, ledit catalyseur étant plus particulièrement un catalyseur de Shrock, un catalyseur de Grubbs 1^{ère} génération, un catalyseur de Grubbs 2^{ème} génération, ou un catalyseur de formule suivante: 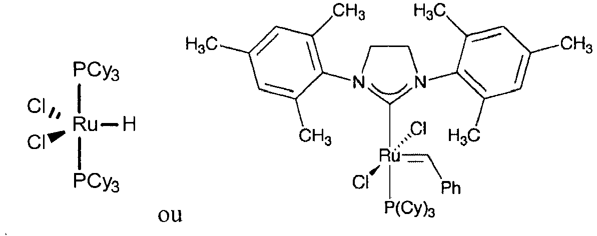 pour former un composé de formule suivante : 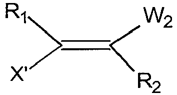 dans laquelle R₁, R₂, X' et W₂ sont tels que définis ci-dessus,
- une réaction de type Simmons-Smith à partir d'un composé de formule suivante : 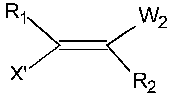 dans laquelle R₁, R₂, X' et W₂ sont tels que définis ci-dessus, pour obtenir un composé de formule suivante : 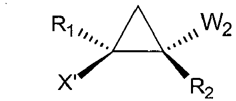 et son énantiomère, dans laquelle R₁, R₂, X' et W₂ sont tels que définis ci-dessus,
si W représente -OH ou -NH₂, et/ou X est différent de B(OR)₂, ledit procédé comprenant en outre les étapes suivantes :
lorsque X' représente B(OR)₂,
   - une étape de conversion de W₂ en W, en particulier la déprotection du groupement Z' lorsque W₂ représente -OZ' ou des groupements Z et Z₂ lorsque W₂ représente -NZZ₂, permettant d'obtenir 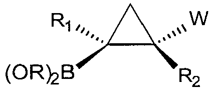 et son énantiomère à partir de 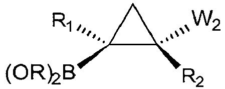 et son énantiomère,
      et
   - une étape de conversion de B(OR)₂ en -X permettant d'obtenir 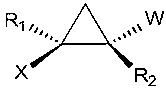 et son énantiomère à partir de 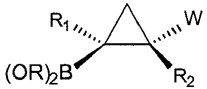 et son énantiomère,
      ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
      ou
   - une étape de conversion de B(OR)₂ en -X permettant d'obtenir 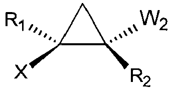 et son énantiomère à partir de 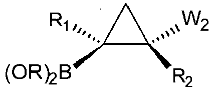 et son énantiomère,
      ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
      et
   - une étape de conversion de W₂ en W, en particulier la déprotection du groupement Z' lorsque W₂ représente -OZ' ou des groupements Z et Z₂ lorsque W₂ représente -NZZ₂, permettant d'obtenir 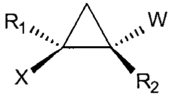 et son énantiomère à partir de 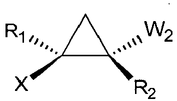 et son énantiomère,
      ou, lorsque X' représente BF₃M, W représentant -OH ou -NH₂,
   - une étape de conversion de W₂ en W, en particulier la déprotection du groupement Z' lorsque W₂ représente -OZ' ou des groupements Z et Z₂ lorsque W₂ représente -NZZ₂, permettant d'obtenir 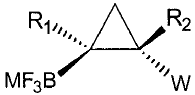 et son énantiomère à partir de 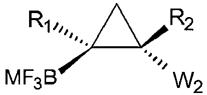 et son énantiomère.

Selon un mode de réalisation avantageux, la présente description concerne un procédé de préparation d'un composé répondant à la formule (I), dans laquelle W représente un groupe fonctionnel choisi parmi -OH, -OR^{a}, -OZ', -NH₂, -NHR^{a}, -NR^{a}R^{b}, -NHZ et -NZZ₂, en particulier parmi -OH, -OR^{a} et -OZ',
sous la forme d'un composé *cis* racémique de formule (I-2b) : 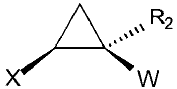 et son énantiomère, (I-2b) dans laquelle X et W sont tels que définis précédemment, ledit procédé comprenant :
- une réaction de type Simmons-Smith à partir d'un composé de formule suivante : 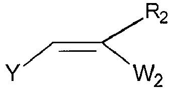 dans laquelle Y est tel que défini précédemment, W₂ représentant un groupe fonctionnel choisi parmi -OR^{a}, -OZ', -NR^{a}R^{b} et -NZZ₂, en particulier parmi -OR^{a} et -OZ',
   pour obtenir un composé de formule suivante : 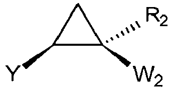 et son énantiomère, dans laquelle Y et W₂ sont tels que définis ci-dessus, et
- le traitement d'un composé de formule suivante : 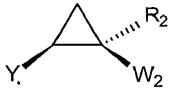 et son énantiomère, dans laquelle Y et W₂ sont tels que définis ci-dessus,
   par :
   - une base forte, en particulier un alkyl lithium, plus particulièrement le n-butyllithium ou *sec*-butyllithium, puis
   - un composé de formule X"-B(OR)₂, R étant tel que défini ci-dessus, X" représentant H, un groupe O-alkyle comportant de 1 à 14 atomes de carbone ou un groupe O-aryle éventuellement substitués,
   pour obtenir un composé de formule suivante : 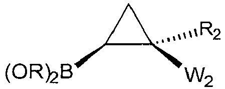 et son énantiomère, dans laquelle R et W sont tels que définis ci-dessus,
   ou
- le traitement d'un composé de formule suivante : 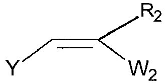 dans laquelle Y et W₂ sont tels que définis ci-dessus,
   par :
   - une base forte, en particulier un alkyl lithium, plus particulièrement le n-butyllithium ou *sec*-butyllithium, puis
   - un composé de formule X'-B(OR)₂, R étant tel que défini ci-dessus, X' représentant H, un groupe O-alkyle comportant de 1 à 14 atomes de carbone ou un groupe O-aryle éventuellement substitués,
   pour obtenir un composé de formule suivante : 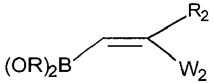 dans laquelle R et W₂ sont tels que définis ci-dessus, et
- une réaction de type Simmons-Smith à partir d'un composé de formule suivante : 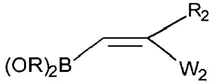 dans laquelle Y est tel que défini précédemment, W₂ représentant un groupe fonctionnel choisi parmi -OR^{a}, -OZ', -NR^{a}R^{b} et -NZZ₂,
   pour obtenir un composé de formule suivante : 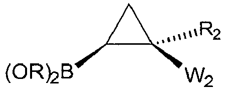 et son énantiomère, dans laquelle R et W₂ sont tels que définis ci-dessus ;
si W représente -OH ou -NH₂, et/ou X est différent de B(OR)₂, ledit procédé comprenant en outre les étapes suivantes :
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 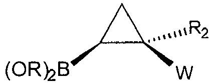 et son énantiomère à partir de 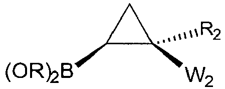 et son énantiomère, et
- une étape de conversion de B(OR)₂ en -X permettant d'obtenir 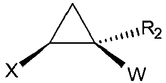 et son énantiomère à partir de 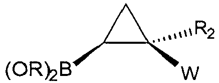 et son énantiomère,
   ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   ou
- une étape de conversion de B(OR)₂ en -X permettant d'obtenir 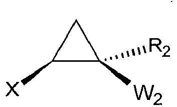 et son énantiomère à partir de 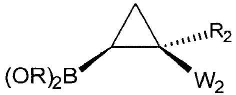 et son énantiomère,
   ladite étape de conversion étant en particulier en présence de MHF₂ lorsque X représente BF₃M, ou en particulier une hydrolyse, plus particulièrement en présence d'une base minérale, organique ou en présence d'un acide de Lewis, lorsque X représente B(OH)₂,
   et
- une étape de conversion de W₂ en W, en particulier la déprotection du groupement Z' lorsque W₂ représente -OZ' ou des groupements Z et Z₂ lorsque W₂ représente -NZZ₂, permettant d'obtenir 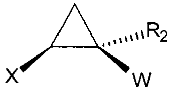 et son énantiomère à partir de 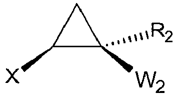 et son énantiomère.

La description concerne également un composé répondant à la formule (I) suivante 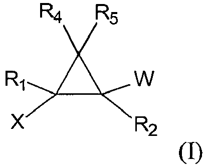 dans laquelle :
▪ X représente un atome de bore substitué choisi dans le groupe comprenant B(OH)₂, B(OR)₂, BF₃M, B(OR')₃M dans lesquels :
   ∘ R est un groupe alkyle comportant de 1 à 14 atomes de carbone, un groupe aryle éventuellement substitués, ou est tel que (OR)₂ forme un cycle entre les deux atomes d'oxygène, (OR)₂ étant notamment choisi dans le groupe comprenant les radicaux bivalents dérivant de diols, tels que O-CH₂-CH₂-O, O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH2-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O et ses esters, et les radicaux bivalents dérivant de diacides, tels que OCO-CH₂-N(CH₃)-CH₂-COO,
   ∘ R' est un groupe alkyle comportant de 1 à 14 atomes de carbone ou est tel que :
      - (OR')₃ forme un cycle entre deux des atomes d'oxygène, (OR')₃ étant alors sous la forme OR'(OR)₂, où R' est un groupe alkyle comportant de 1 à 14 atomes de carbone et (OR)₂ est tel que défini ci-dessus, ou
      - (OR')₃ forme un bicycle entre les trois atomes d'oxygène, (OR')₃ étant notamment choisi dans le groupe comprenant les radicaux trivalents dérivant de triols, tels que H₃C-C-(CH₂-O)₃,
   ∘ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} R^{e}, R^{f} sont choisis parmi H ou une chaîne carbonée saturée comportant notamment 1 à 6 atomes de carbone choisis indépendamment les uns des autres,
      et notamment X représente B(OH)₂, B(OR)₂ ou BF₃K,
   R₁, R₄ et R₅ identiques ou différents sont choisis dans le groupe constitué:
   1. de H
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   R₁ et R₄, ou R₁ et R₅ pouvant former un cycle à 5, 6, ou 7 chainons comportant éventuellement un hétéroatome choisi parmi l'oxygène, de l'azote et le soufre, ledit cycle pouvant être substitué ;
▪ R₂ est choisi dans le groupe constitué par les groupes pouvant être représentés par R₁, R₄ ou R₅, ainsi que -COR^{a}, -COOR^{a}, -CONH₂, -CONHR^{a}, -CONR^{a}R^{b}, -CN et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CH₂OH, -CH₂OR^{a}, -CHR^{b}OH, -CHR^{b}OR^{a}, -CR^{b}R^{b'}OH, - CR^{b}R^{b'}OR^{a}, -CH₂NH₂, -CH₂NHZ, -CHR^{a}NHZ, -OH, -OR^{a}, -OZ', -NH₂, -NHR^{a}, -NR^{a}R^{b},-NHZ et -NZZ₂,
   dans lesquels Z et Z₂ représentent un groupe protecteur d'une fonction amine, et Z' représente un groupe protecteur d'une fonction alcool, et
   dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
sous réserve que :
▪ lorsque R₁, R₂, R₄ et R₅ représentent H et que B représente B(OH)₂, B(OR)₂, ou B(OR')₃M, alors W est choisi parmi -COR^{a}, -CONH₂, -CONHR^{a}, -CONR^{a}R^{b}, -OH, -OR^{a} et-OZ',
▪ lorsque R₁, R₂, R₄ et R₅ représentent H et que B représente BF₃M, alors W est choisi parmi -COOH, -COOR^{a}, -CHO, -COR^{a} -CONH₂, -CONHR^{a}, -CONR^{a}R^{b}, -OH, -OR^{a} et -OZ',
▪ lorsque W représente CH₂OH, -CH₂OR^{a}, -CHR^{b}OH, -CHR^{b}OR^{a}, -CR^{b}R^{b'}OH, ou-CR^{b}R^{b'}OR^{a}, et que B représente B(OH)₂, B(OR)₂, ou B(OR')₃M, alors :
   ∘ R₁ ne représente pas H, ou
   ∘ R₂ et R₄ ne représentent pas H, ou
   ∘ R₂ et R₅ ne représentent pas H.
▪ lorsque W représente CH₂OH, -CH₂OR^{a}, -CHR^{b}OH, -CHR^{b}OR^{a}, -CR^{b}R^{b'}OH, ou-CR^{b}R^{b'}OR^{a}, et que B représente BF₃M, alors R₁ ou R₂ ne représente pas H.
▪ lorsque W représente -COOH ou -COOR^{a}, et que B représente B(OH)₂, B(OR)₂, ou B(OR')₃M, alors :
   ∘ R₁ ne représente pas H, ou
   ∘ R₂ et R₄ ne représentent pas H, ou
   ∘ R₂ et R₅ ne représentent pas H.

Selon un mode de réalisation avantageux, la présente invention concerne un composé dans lequel W représente un groupe fonctionnel choisi parmi -CHO, -COR^{a}, -COOH,-COOR^{a}, -CONH₂, -CONHR^{a}, -CONR^{a}R^{b}, -CH₂OH, -CH₂OR^{a}, -CHR^{b}OH, -CHR^{b}OR^{a},-CR^{b}R^{b'}OH, -CR^{b}R^{b'}OR^{a}, -CH₂NH₂, -CH₂NHZ, -CHR^{a}NHZ.

Selon un mode de réalisation avantageux, la présente invention concerne un composé dans lequel W représente un groupe fonctionnel choisi parmi -CHO, -COR^{a}, -COOH,-COOR^{a}, -CONH₂, -CONHR^{a}, -CONR^{a}R^{b}, -CH₂OH, -CH₂OR^{a}, -CHR^{b}OH, -CHR^{b}OR^{a}, - CR^{b}R^{b'}OH, -CR^{b}R^{b'}OR^{a}_{.}

Selon un mode de réalisation avantageux, la présente invention concerne un composé dans lequel W représente un groupe fonctionnel choisi parmi -CHO, -COR^{a}, -COOH,-COOR^{a}, -CONH₂, -CONHR^{a}, -CONR^{a}R^{b}.

Selon un mode de réalisation avantageux, la présente invention concerne un composé dans lequel W représente un groupe fonctionnel choisi parmi -CONH-SO₂-cyclopropyle,-CH₂-NH-CO-CH₂-CH₃ et le groupe de formule suivante :

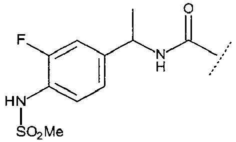

Selon un mode de réalisation avantageux, la présente invention concerne un composé dans lequel R₂ représente H.

Selon un mode de réalisation avantageux, la présente invention concerne un composé dans lequel R₂ est choisi dans le groupe constitué par les groupes -COR^{a}, -COOR^{a}, -CONH₂, -CONHR^{a}, -CONR^{a}R^{b}, -CN et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un composé dans lequel R₁, R₄ et R₅ représentent H, un aryle, un hétérocycle, un hétéroaryle ou un alkyle tels que définis précédemment.

Selon un mode de réalisation avantageux, la présente invention concerne un composé dans lequel R₁, R₂, R₄ et R₅ représentent H.

Selon un mode de réalisation avantageux, la présente description concerne un composé dans lequel W représente un groupe fonctionnel choisi parmi -OH, -OR^{a}, -OZ', - NH₂,-NHR^{a}, -NR^{a}R^{b}, -NHZ et -NZZ₂,
R₁, R₂, R₄ et R₅ représentant en particulier H.

Selon un mode de réalisation avantageux, la présente description concerne un composé dans lequel R₄ et R₅ représentent H.

Selon un mode de réalisation avantageux, la présente description concerne un composé dans lequel R₁, R₄ et R₅ représentent H.

Selon un mode de réalisation avantageux, la présente description concerne un composé dans lequel R₂, R₄ et R₅ représentent H.

Selon un mode de réalisation avantageux, la présente description concerne un composé dans lequel R₁, R₂, R₄ et R₅ représentent H.

Selon un mode de réalisation avantageux, la présente description concerne un composé dans lequel R₁ et R₂ représentent indépendamment l'un de l'autre H ou un groupe alkyle linéaire, cyclique ou ramifié comportant de 1 à 15 atomes de carbone, éventuellement substitués.

Selon un mode de réalisation avantageux, la présente description concerne un composé dans lequel R₂ représente H ou un groupe alkyle linéaire, cyclique ou ramifié comportant de 1 à 15 atomes de carbone, éventuellement substitués.

Selon un mode de réalisation avantageux, la présente invention concerne un composé répondant à la formule (I-A) suivante 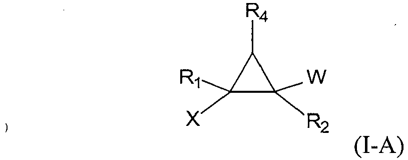 dans laquelle :
▪ X représente un atome de bore substitué choisi dans le groupe comprenant B(OH)₂, B(OR)₂, BF₃M, B(OR')₃M dans lesquels :
   ∘ R est un groupe alkyle comportant de 1 à 14 atomes de carbone, un groupe aryle éventuellement substitués, ou est tel que (OR)₂ forme un cycle entre les deux atomes d'oxygène, (OR)₂ étant notamment choisi dans le groupe comprenant les radicaux bivalents dérivant de diols, tels que O-CH₂-CH₂-O, O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O et ses esters, et les radicaux bivalents dérivant de diacides, tels que OCO-CH₂-N(CH₃)-CH₂-COO,
   ∘ R' est un groupe alkyle comportant de 1 à 14 atomes de carbone ou est tel que :
      - (OR')₃ forme un cycle entre deux des atomes d'oxygène, (OR')₃ étant alors sous la forme OR'(OR)₂, où R' est un groupe alkyle comportant de 1 à 14 atomes de carbone et (OR)₂ est tel que défini ci-dessus, ou
      - (OR')₃ forme un bicycle entre les trois atomes d'oxygène, (OR')₃ étant notamment choisi dans le groupe comprenant les radicaux trivalents dérivant de triols, tels que H₃C-C-(CH₂-O)₃,
   ∘ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} R^{e}, R^{f} sont choisis parmi H ou une chaîne carbonée saturée comportant notamment 1 à 6 atomes de carbone choisis indépendamment les uns des autres,
      et notamment X représente B(OH)₂, B(OR)₂ ou BF₃K,
   R₁ et R₄ identiques ou différents sont choisis dans le groupe constitué:
   1. de H
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   l'un au moins des groupes R₁ et R₄ représentant H,
▪ R₂ est choisi dans le groupe constitué par les groupes pouvant être représentés par R₁ ou R₄, ainsi que -COR^{a}, -COOR^{a}, -CONH₂, -CONHR^{a}, -CONR^{a}R^{b}, -CN et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CONH-SO₂-R^{a}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a}, dans lesquels Z représente un groupe protecteur d'une fonction amine, et dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
sous réserve que :
▪ lorsque R₁, R₂ et R₄ représentent H et que B représente B(OH)₂, B(OR)₂, ou B(OR')₃M, alors W est choisi parmi -COR^{a}, -CONH₂, -CONHR^{a}, -CONR^{a}R^{b},
▪ lorsque R₁, R₂ et R₄ représentent H et que B représente BF₃M, alors W est choisi parmi - COOH, -COOR^{a}, -CHO, -COR^{a}-CONH₂, -CONHR^{a}, -CONR^{a}R^{b},
▪ lorsque W représente CH₂OH, -CH₂OR^{a}, -CHR^{b}OH, -CHR^{b}OR^{a}, -CR^{b}R^{b'}OH, ou-CR^{b}R^{b'}OR^{a}, et que B représente B(OH)₂, B(OR)₂, ou B(OR')₃M, alors :
   ∘ R₁ ne représente pas H, ou
   ∘ R₂ et R₄ ne représentent pas H,
▪ lorsque W représente CH₂OH, -CH₂OR^{a}, -CHR^{b}OH, -CHR^{b}OR^{a}, -CR^{b}R^{b'}OH, ou-CR^{b}R^{b'}OR^{a}, et que B représente BF₃M, alors R₁ ou R₂ ne représente pas H.
▪ lorsque W représente -COOH ou -COOR^{a}, et que B représente B(OH)₂, B(OR)₂, ou B(OR')₃M, alors :
   ∘ R₁ ne représente pas H, ou
   ∘ R₂ et R₄ ne représentent pas H.

Selon un mode de réalisation avantageux, la présente invention concerne un composé répondant à la formule (I-B) suivante 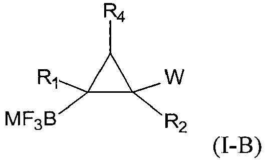 dans laquelle :
▪ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} R^{e}, R^{f} sont choisis parmi H ou une chaîne carbonée saturée comportant notamment 1 à 6 atomes de carbone choisis indépendamment les uns des autres,
   BF₃M représentant notamment BF₃K,
   R₁ et R₄ identiques ou différents sont choisis dans le groupe constitué:
   1. de H
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   l'un au moins des groupes R₁ et R₄ représentant H,
▪ R₂ est choisi dans le groupe constitué par les groupes pouvant être représentés par R₁ ou R₄, ainsi que -COR^{a}, -COOR^{a}, -CONH₂, -CONHR^{a}, -CONR^{a}R^{b}, -CN et -NO₂, dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CONH-SO₂-R^{a}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a}, dans lesquels Z représente un groupe protecteur d'une fonction amine, et
   dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
sous réserve que :
▪ lorsque R₁, R₂ et R₄ représentent H, alors W est choisi parmi -COOH, -COOR^{a}, -CHO,-COR^{a} -CONH₂, -CONHR^{a}, -CONR^{a}R^{b},
▪ lorsque W représente CH₂OH, -CH₂OR^{a}, -CHR^{b}OH, -CHR^{b}OR^{a}, -CR^{b}R^{b'}OH, ou-CR^{b}R^{b'}OR^{a}, alors R₁ ou R₂ ne représente pas H.

Selon un mode de réalisation avantageux, la présente invention concerne un composé de formule (I-A) ou (I-B) dans lequel W représente un groupe fonctionnel choisi -CHO,-COR^{a}, -COOH, -COOR^{a}, -CONH₂, -CONHR^{a}, -CONR^{a}R^{b}, -CONH-SO₂₋R^{a}.

Selon un mode de réalisation avantageux, la présente invention concerne un composé de formule (I-A) ou (I-B) dans lequel W représente un groupe fonctionnel choisi parmi-CHO, -COR^{a}, -COOH et -COOR^{a}, W représentant en particulier -COOR^{a}.

Selon un mode de réalisation avantageux, la présente invention concerne un composé de formule (I-A) ou (I-B) dans lequel W représente un groupe fonctionnel choisi parmi-CONH-SO₂-cyclopropyle, -CH₂-NH-CO-CH₂-CH₃ et le groupe de formule suivante :

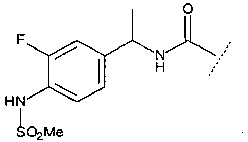

Selon un mode de réalisation avantageux, la présente invention concerne un composé de formule (I-A) ou (I-B) dans lequel R₂ représente H.

Selon un mode de réalisation avantageux, la présente invention concerne un composé de formule (I-A) ou (I-B) dans lequel R₂ est choisi dans le groupe constitué par les groupes-COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un composé de formule (I-A) ou (I-B) dans lequel R₁, R₂ et R₄ représentent H.

Selon un mode de réalisation avantageux, la présente invention concerne un composé de formule (I-A) ou (I-B) dans lequel R₁ représente H.

Selon un mode de réalisation avantageux, la présente invention concerne un composé de formule (I-A) ou (I-B) dans lequel R₁ représente H et R₄ représente un aryle, un hétérocycle, un hétéroaryle ou un alkyle tels que définis précédemment.

Selon un mode de réalisation avantageux, la présente invention concerne un composé de formule (I-A) ou (I-B) dans lequel R₄ représente H.

Selon un mode de réalisation avantageux, la présente invention concerne un composé de formule (I-A) ou (I-B) dans lequel R₄ représente H et R1 représente un aryle, un hétérocycle, un hétéroaryle ou un alkyle tels que définis précédemment.

Selon un mode de réalisation avantageux, la présente invention concerne un composé de formule (I-A) ou (I-B) dans lequel R₁ et R₂ représentent H et R₄ représente un aryle, un hétérocycle, un hétéroaryle ou un alkyle tels que définis précédemment.

Selon un mode de réalisation avantageux, la présente invention concerne un composé de formule (I-A) ou (I-B) dans lequel R₂ et R₄ représentent H et R₁ représente un aryle, un hétérocycle, un hétéroaryle ou un alkyle tels que définis précédemment.

Selon un mode de réalisation avantageux, la présente invention concerne un composé de formule (I-A) ou (I-B) dans lequel R₁ représente H, R₄ représente un aryle, un hétérocycle, un hétéroaryle ou un alkyle tels que définis précédemment, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un composé de formule (I-A) ou (I-B) dans lequel R₄ représente H, R₁ représente un aryle, un hétérocycle, un hétéroaryle ou un alkyle tels que définis précédemment, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un composé de formule (I-A) ou (I-B) dans lequel R₁ et R₄ représentent H, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un composé répondant à la formule (I-C) suivante 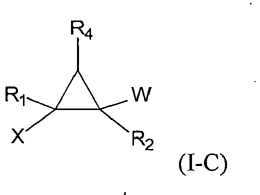 dans laquelle :
▪ X représente un atome de bore substitué choisi dans le groupe comprenant B(OH)₂, B(OR)₂, BF₃M, B(OR')₃M dans lesquels :
   ∘ R est un groupe alkyle comportant de 1 à 14 atomes de carbone, un groupe aryle éventuellement substitués, ou est tel que (OR)₂ forme un cycle entre les deux atomes d'oxygène, (OR)₂ étant notamment choisi dans le groupe comprenant les radicaux bivalents dérivant de diols, tels que O-CH₂-CH₂-O, O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O et ses esters, et les radicaux bivalents dérivant de diacides, tels que OCO-CH₂-N(CH₃)-CH₂-COO,
   ∘ R' est un groupe alkyle comportant de 1 à 14 atomes de carbone ou est tel que :
      - (OR')₃ forme un cycle entre deux des atomes d'oxygène, (OR')₃ étant alors sous la forme OR'(OR)₂, où R' est un groupe alkyle comportant de 1 à 14 atomes de carbone et (OR)₂ est tel que défini ci-dessus, ou
      - (OR')₃ forme un bicycle entre les trois atomes d'oxygène, (OR')₃ étant notamment choisi dans le groupe comprenant les radicaux trivalents dérivant de triols, tels que H₃C-C-(CH₂-O)₃,
   ∘ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} R^{e}, R^{f} sont choisis parmi H ou une chaîne carbonée saturée comportant notamment 1 à 6 atomes de carbone choisis indépendamment les uns des autres,
      et notamment X représente B(OH)₂, B(OR)₂ ou BF₃K,
   R₁ et R₄ identiques ou différents sont choisis dans le groupe constitué:
   1. de H
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   l'un au moins des groupes R₁ et R₄ représentant H,
▪ R₂ est choisi dans le groupe constitué par H et les groupes -COR^{a}, -COOR^{a}, -CONH₂,-CONHR^{a}, -CONR^{a}R^{b} et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b},
   dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
sous réserve que :
▪ lorsque R₁, R₂ et R₄ représentent H et que B représente B(OH)₂, B(OR)₂, ou B(OR')₃M, alors W est choisi parmi -COR^{a}, -CONH₂, -CONHR^{a}, -CONR^{a}R^{b},
▪ lorsque W représente -COOH ou -COOR^{a}, et que B représente B(OH)₂, B(OR)₂, ou B(OR')₃M, alors :
   ∘ R₁ ne représente pas H, ou
   ∘ R₂ et R₄ ne représentent pas H.

Selon un mode de réalisation avantageux, la présente invention concerne un composé répondant à la formule (I-D) suivante 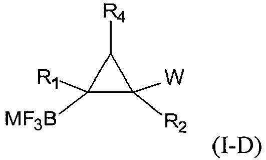 dans laquelle :
▪ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} Re, R^{f} sont choisis parmi H ou une chaîne carbonée saturée comportant notamment 1 à 6 atomes de carbone choisis indépendamment les uns des autres,
   BF₃M représentant notamment BF₃K,
   R₁ et R₄ identiques ou différents sont choisis dans le groupe constitué:
   1. de H
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   l'un au moins des groupes R₁ et R₄ représentant H,
▪ R₂ est choisi dans le groupe constitué par H et les groupes -COR^{a}, -COOR^{a}, -CONH₂,-CONHR^{a}, -CONR^{a}R^{b} et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b},
   dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués.

Selon un mode de réalisation avantageux, la présente invention concerne un composé de formule (I-C) ou (I-D) dans lequel W représente un groupe fonctionnel choisi parmi-CHO, -COR^{a}, -COOH et -COOR^{a}, W représentant en particulier -COOR^{a}.

Selon un mode de réalisation avantageux, la présente invention concerne un composé de formule (I-C) ou (I-D) dans lequel R₂ représente H.

Selon un mode de réalisation avantageux, la présente invention concerne un procédé de préparation d'un composé de formule (I-C) ou (I-D) dans lequel R₂ est choisi dans le groupe constitué par les groupes -COOR^{a}, -CONH₂, -CONHR^{a} et -CONR^{a}R^{b},
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un composé de formule (I-C) ou (I-D) dans lequel R₁, R₂ et R₄ représentent H.

Selon un mode de réalisation avantageux, la présente invention concerne un composé de formule (I-C) ou (I-D) dans lequel R₁ représente H.

Selon un mode de réalisation avantageux, la présente invention concerne un composé de formule (I-C) ou (I-D) dans lequel R₁ représente H et R₄ représente un aryle, un hétérocycle, un hétéroaryle ou un alkyle tels que définis précédemment.

Selon un mode de réalisation avantageux, la présente invention concerne un composé de formule (I-C) ou (I-D) dans lequel R₄ représente H.

Selon un mode de réalisation avantageux, la présente invention concerne un composé de formule (I-C) ou (I-D) dans lequel R₄ représente H et R1 représente un aryle, un hétérocycle, un hétéroaryle ou un alkyle tels que définis précédemment.

Selon un mode de réalisation avantageux, la présente invention concerne un composé de formule (I-C) ou (I-D) dans lequel R₁ et R₂ représentent H et R₄ représente un aryle, un hétérocycle, un hétéroaryle ou un alkyle tels que définis précédemment.

Selon un mode de réalisation avantageux, la présente invention concerne un composé de formule (I-C) ou (I-D) dans lequel R₂ et R₄ représentent H et R₁ représente un aryle, un hétérocycle, un hétéroaryle ou un alkyle tels que définis précédemment.

Selon un mode de réalisation avantageux, la présente invention concerne un composé de formule (I-C) ou (I-D) dans lequel R₁ représente H, R₄ représente un aryle, un hétérocycle, un hétéroaryle ou un alkyle tels que définis précédemment, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un composé de formule (I-C) ou (I-D) dans lequel R₄ représente H, R₁ représente un aryle, un hétérocycle, un hétéroaryle ou un alkyle tels que définis précédemment, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un composé de formule (I-C) ou (I-D) dans lequel R₁ et R₄ représentent H, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne un composé choisi dans le groupe constitué des composés de formule suivante et leur énantiomère:

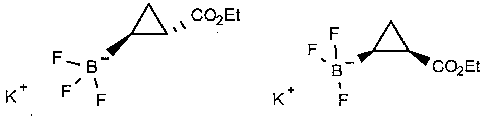

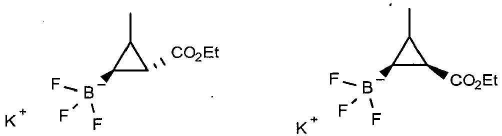

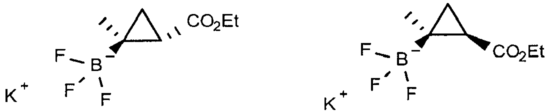

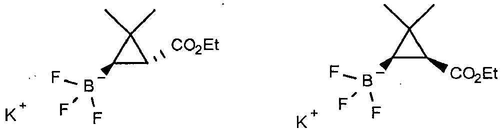

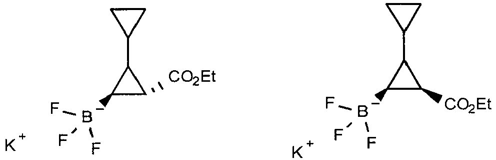

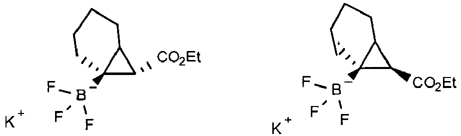

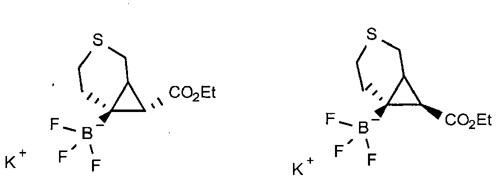

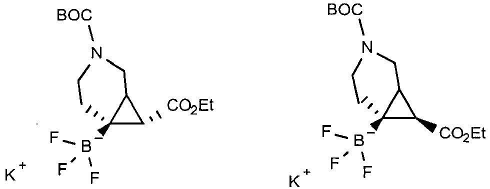

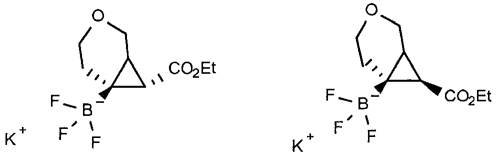

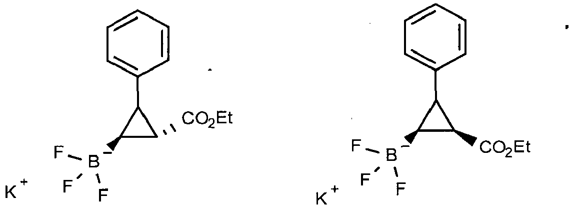

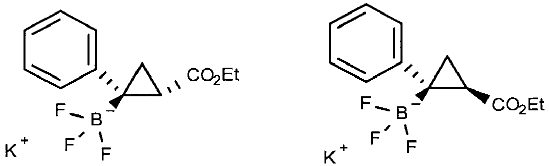

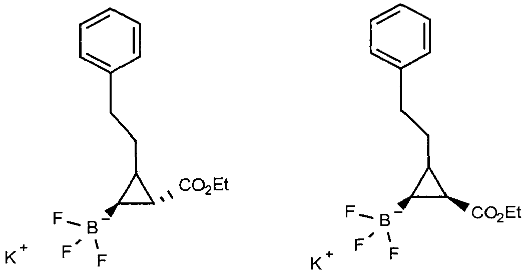

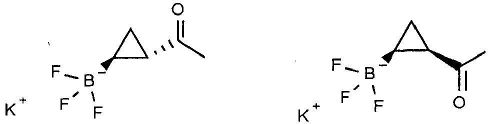

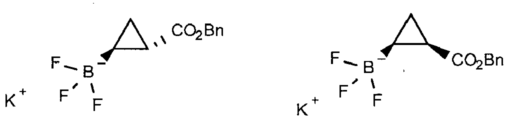

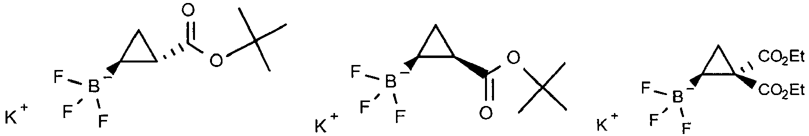

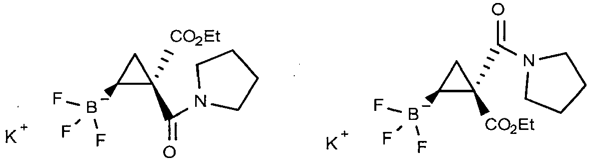

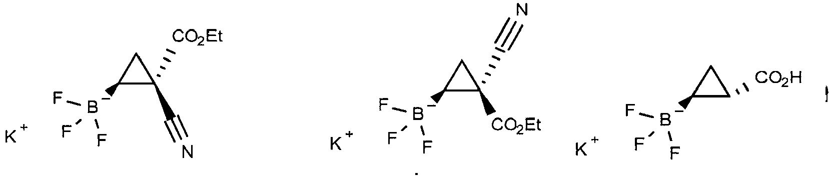

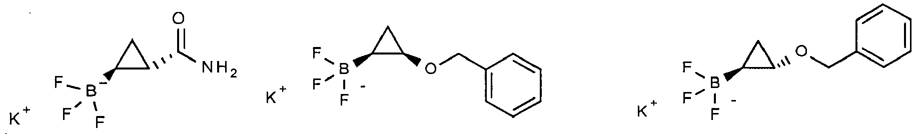

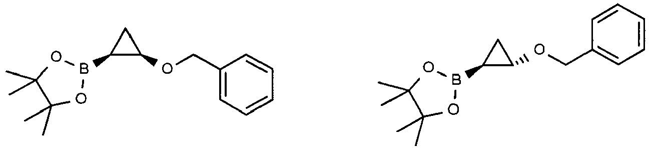

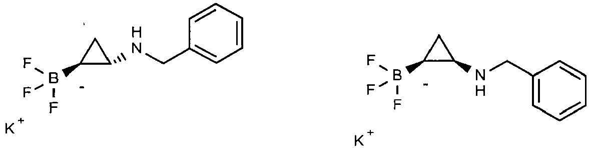

Selon un mode de réalisation avantageux, la présente invention concerne un composé choisi dans le groupe constitué des composés de formule suivante et leur énantiomère:

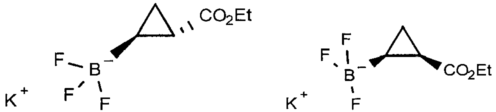

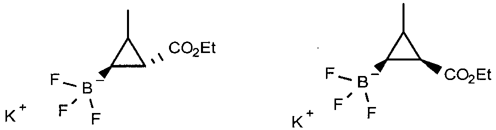

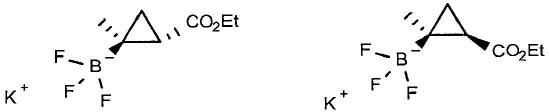

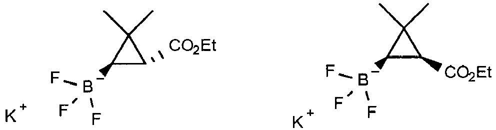

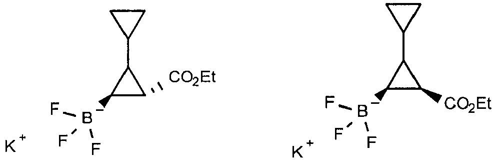

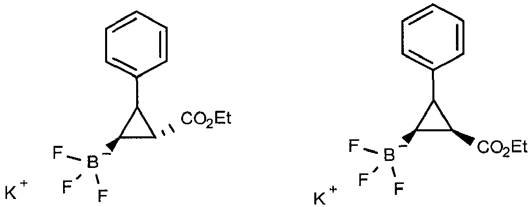

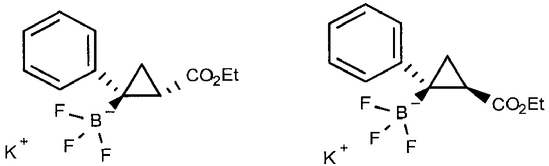

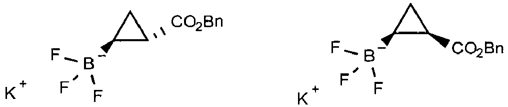

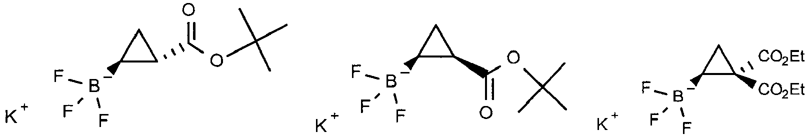

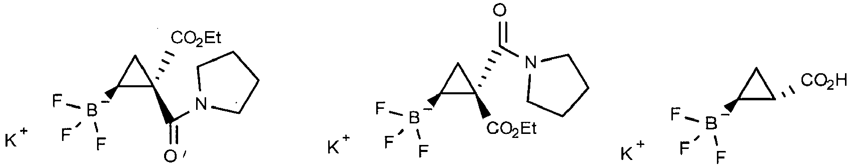

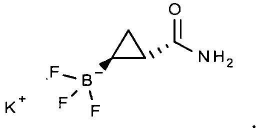

La description concerne également l'utilisation d'un composé de formule (I), pour la préparation de composés de formule (II) suivante : 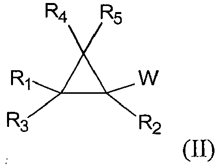 dans laquelle :
▪ R₁, R₃, R₄ et R₅ identiques ou différents sont choisis dans le groupe constitué:
   1. de H, sous réserve que R₃ ne représente pas H,
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   R₁ et R₄, ou R₁ et R₅ pouvant former un cycle à 5, 6, ou 7 chainons comportant éventuellement un hétéroatome choisi parmi l'oxygène, de l'azote et le soufre, ledit cycle pouvant être substitué ;
▪ R₂ est choisi dans le groupe constitué par les groupes pouvant être représentés par R₁, R₄ ou R₅, ainsi que -COR^{a}, -COOR^{a}, -CONH₂, -CONHR^{a}, -CONR^{a}R^{b}, -CN et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CH₂OH, -CH₂OR^{a}, -CHR^{b}OH, -CHR^{b}OR^{a}, -CR^{b}R^{b'}OH,-CR^{b}R^{b'}OR^{a}, -CH₂NH₂, -CH₂NHZ, -CHR^{a}NHZ, -OH, -OR^{a}, -OZ', - NH₂, -NHR^{a}, -NR^{a}R^{b},-NHZ et NZZ₂,
   dans lesquels Z représente un groupe protecteur d'une fonction amine, Z' représente un groupe protecteur d'une fonction alcool, et
   dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
   sous réserve que :
   ▪ lorsque R₁, R₂, R₄ et R₅ représentent H et que B représente B(OH)₂, B(OR)₂, ou B(OR')₃M, alors W est choisi parmi -COR^{a}, -CONH₂, -CONHR^{a}, et -CONR^{a}R^{b},
   ▪ lorsque R₁, R₂, R₄ et R₅ représentent H et que B représente BF₃M, alors W est choisi parmi -COOH, -COOR^{a}, -CHO, -COR^{a} -CONH₂, -CONHR^{a}, -CONR^{a}R^{b},
   ▪ lorsque W représente CH₂OH, -CH₂OR^{a}, -CHR^{b}OH, -CHR^{b}OR^{a}, -CR^{b}R^{b'}OH, ou-CR^{b}R^{b'}OR^{a}, et que B représente B(OH)₂, B(OR)₂, ou B(OR')₃M, alors :
      ∘ R₁ ne représente pas H, ou
      ∘ R₂ et R₄ ne représentent pas H, ou
      ∘ R₂ et R₅ ne représentent pas H.
   ▪ lorsque W représente CH₂OH, -CH₂OR^{a}, -CHR^{b}OH, -CHR^{b}OR^{a}, -CR^{b}R^{b'}OH, ou-CR^{b}R^{b}'OR^{a}, et que B représente BF₃M, alors R₁ ou R₂ ne représente pas H.
   ▪ lorsque W représente -COOH ou -COOR^{a}, et que B représente B(OH)₂, B(OR)₂, ou B(OR')₃M, alors :
      ∘ R₁ ne représente pas H, ou
      ∘ R₂ et R₄ ne représentent pas H, ou
      ∘ R₂ et R₅ ne représentent pas H,
par réaction dudit composé de formule (I) avec un composé de formule (III) suivante :

R₃-X₂,

dans laquelle R₃ est tel que défini ci-dessus, et X₂ est choisi dans le groupe constitué des halogénures, en particulier l'iode, le brome et le chlore, et du triflate (OTf), en présence d'un catalyseur contenant un métal de transition, et, éventuellement, d'un ligand.

Selon un mode de réalisation avantageux, la présente invention concerne une utilisation dans laquelle ledit métal de transition est le palladium (0) ou le palladium (II), ledit catalyseur étant en particulier choisi dans le groupe constitué de Pd(PPh₃)₄, Pd(OAc)₂, PdCl₂(dppf), PdCl₂ et PdCl₂(CN)₂.

Selon un mode de réalisation avantageux, la présente invention concerne une utilisation, ladite utilisation étant en présence d'un ligand choisi dans le groupe constitué de PPh₃, P(*t*Bu)₃, *n*-BuPAd₂, 1,2-*bis*(diphénylphosphine)propane (dpp), tricyclohexylphosphane (PCy₃), S-Phos et Xantphos.

Selon un mode de réalisation avantageux, la présente invention concerne une utilisation dans laquelle W représente un groupe fonctionnel choisi parmi -CHO, -COR^{a},-COOH, -COOR^{a}, -CONH₂, -CONHR^{a}, -CONR^{a}R^{b}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ.

Selon un mode de réalisation avantageux, la présente invention concerne une utilisation dans lequel W représente un groupe fonctionnel choisi parmi -CONH-SO₂- cyclopropyle, -CH₂-NH-CO-CH₂-CH₃, et le groupe de formule suivante :

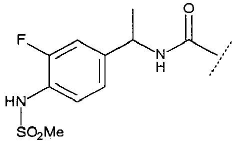

Selon un mode de réalisation avantageux, la présente invention concerne une utilisation dans laquelle R₂ représente H.

Selon un mode de réalisation avantageux, la présente invention concerne une utilisation dans laquelle R₂ est choisi dans le groupe constitué par les groupes -COR^{a},-COOR^{a}, -CONH₂, -CONHR^{a}, -CONR^{a}R^{b}, -CN et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne une utilisation dans laquelle R₁, R₄ et R₅ représentent H, un aryle, un hétérocycle, un hétéroaryle ou un alkyle tels que définis précédemment.

Selon un mode de réalisation avantageux, la présente invention concerne une utilisation dans laquelle R₁, R₂, R₄ et R₅ représentent H.

Selon un mode de réalisation avantageux, la présente description concerne une utilisation dans laquelle W représente un groupe fonctionnel choisi parmi -OH, -OR^{a}, -OZ',-NH₂, -NHR^{a}, -NR^{a}R^{b}, -NHZ et -NZZ₂,
R₁, R₂, R₄ et R₅ représentant en particulier H.

Selon un mode de réalisation avantageux; la présente description concerne une utilisation dans laquelle R₁ et R₂ représentent indépendamment l'un de l'autre H ou un groupe alkyle linéaire, cyclique ou ramifié comportant de 1 à 15 atomes de carbone, éventuellement substitués.

Selon un mode de réalisation avantageux, la présente description concerne une utilisation dans laquelle R₂ représente H ou un groupe alkyle linéaire, cyclique ou ramifié comportant de 1 à 15 atomes de carbone, éventuellement substitués.

Selon un mode de réalisation avantageux, la présente invention concerne l'utilisation d'un composé de formule (I-A) telle que décrite précédemment, pour la préparation de composés de formule (II-A) suivante : 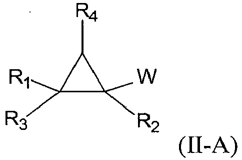 dans laquelle :
▪ R₁, R₃ et R₄ identiques ou différents sont choisis dans le groupe constitué:
   1. de H, sous réserve que R₃ ne représente pas H,
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   l'un au moins des groupes R₁ et R₄ représentant H,
▪ R₂ est choisi dans le groupe constitué par les groupes pouvant être représentés par R₁, R₄ ou R₅, ainsi que -COR^{a}, -COOR^{a}, -CONH₂, -CONHR^{a}, -CONR^{a}R^{b}, -CN et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CONH-SO₂-R^{a}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a}, dans lesquels Z représente un groupe protecteur d'une fonction amine, et
   dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
sous réserve que :
▪ lorsque R₁, R₂ et R₄ représentent H et que B représente B(OH)₂, B(OR)₂, ou B(OR')₃M, alors W est choisi parmi -COR^{a}, -CONH₂, -CONHR^{a}, -CONR^{a}R^{b},
▪ lorsque R₁, R₂ et R₄ représentent H et que B représente BF₃M, alors W est choisi parmi - COOH, -COOR^{a}, -CHO, -COR^{a} -CONH₂, -CONHR^{a}, -CONR^{a}R^{b},
▪ lorsque W représente CH₂OH, -CH₂OR^{a}, -CHR^{b}OH, -CHR^{b}OR^{a}, -CR^{b}R^{b'}OH, ou-CR^{b}R^{b'}OR^{a}, et que B représente B(OH)₂, B(OR)₂, ou B(OR')₃M, alors :
   ∘ R₁ ne représente pas H, ou
   ∘ R₂ et R₄ ne représentent pas H,
▪ lorsque W représente CH₂OH, -CH₂OR^{a}, -CHR^{b}OH, -CHR^{b}OR^{a}, -CR^{b}R^{b'}OH, ou - CR^{b}R^{b'}OR^{a}, et que B représente BF₃M, alors R₁ ou R₂ ne représente pas H.
▪ lorsque W représente -COOH ou -COOR^{a}, et que B représente B(OH)₂, B(OR)₂, ou B(OR')₃M, alors :
   ∘ R₁ ne représente pas H, ou
   ∘ R₂ et R₄ ne représentent pas H,
par réaction dudit composé de formule (I) avec un composé de formule (III) suivante :

R₃-X₂,

dans laquelle R₃ est tel que défini ci-dessus, et X₂ est choisi dans le groupe constitué des halogénures, en particulier l'iode, le brome et le chlore, et du triflate (OTf), en présence d'un catalyseur contenant un métal de transition, et, éventuellement, d'un ligand.

Selon un mode de réalisation avantageux, la présente invention concerne l'utilisation d'un composé de formule (I-B) telle que décrite précédemment, pour la préparation de composés de formule (II-A) suivante : 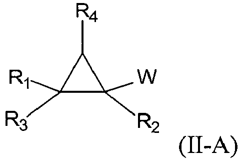 dans laquelle :
▪ R₁, R₃ et R₄ identiques ou différents sont choisis dans le groupe constitué:
   1. de H, sous réserve que R₃ ne représente pas H,
   2. des aryles comportant des cycles de 6 à 15 atomes de carbone éventuellement substitués ;
   3. des hétérocycles ou des hétéroaryles comportant des cycles de 2 à 15 atomes de carbone éventuellement substitués ;
   4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées ;
   5. des alcynyles linéaires ou ramifiés comportant de 1 à 15 atomes de carbone éventuellement substitués ;
   6. des groupes alkyles linéaires, cycliques ou ramifiés comportant de 1 à 15 atomes de carbone éventuellement substitués ;
   l'un au moins des groupes R₁ et R₄ représentant H,
▪ R₂ est choisi dans le groupe constitué par les groupes pouvant être représentés par R₁, R₄ ou R₅, ainsi que -COR^{a}, -COOR^{a}, -CONH₂, -CONHR^{a}, -CONR^{a}R^{b}, -CN et -NO₂,
   dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
   R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CONH-SO₂₋R^{a}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a}, dans lesquels Z représente un groupe protecteur d'une fonction amine, et
   dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
sous réserve que :
▪ lorsque R₁, R₂ et R₄ représentent H et que B représente BF₃M, alors W est choisi parmi-COOH, -COOR^{a}, -CHO, -COR^{a} -CONH₂, -CONHR^{a}, -CONR^{a}R^{b},
▪ lorsque W représente CH₂OH, -CH₂OR^{a}, -CHR^{b}OH, -CHR^{b}OR^{a}, -CR^{b}R^{b'}OH, ou - CR^{b}R^{b'}OR^{a}, et que B représente BF₃M, alors R₁ ou R₂ ne représente pas H,
par réaction dudit composé de formule (I) avec un composé de formule (III) suivante :

R₃-X₂,

dans laquelle R₃ est tel que défini ci-dessus, et X₂ est choisi dans le groupe constitué des halogénures, en particulier l'iode, le brome et le chlore, et du triflate (OTf), en présence d'un catalyseur contenant un métal de transition, et, éventuellement, d'un ligand.

Selon un mode de réalisation avantageux, la présente invention concerne l'utilisation d'un composé de formule (I-A) ou (I-B) dans laquelle ledit métal de transition est le palladium (0) ou le palladium (II), ledit catalyseur étant en particulier choisi dans le groupe constitué de Pd(PPh₃)₄, Pd(OAc)₂, PdCl₂(dppf), PdCl₂ et PdCl₂(CN)₂.

Selon un mode de réalisation avantageux, la présente invention concerne l'utilisation d'un composé de formule (I-A) ou (I-B), ladite utilisation étant en présence d'un ligand choisi dans le groupe constitué de PPh₃, P(*t*Bu)₃, *n*-BuPAd₂, 1,2-*bis*(diphénylphosphine)propane (dpp), tricyclohexylphosphane (PCy₃), S-Phos et Xantphos.

Selon un mode de réalisation avantageux, la présente invention concerne l'utilisation d'un composé de formule (I-A) ou (I-B) dans laquelle W représente un groupe fonctionnel choisi parmi -CHO, -COR^{a}, -COOH, -COOR^{a}, -CH₂OH, -CH₂OR^{a}, -CHR^{b}OH, -CHR^{b}OR^{a},-CR^{b}R^{b'}OH et -CR^{b}R^{b'}OR^{a}.

Selon un mode de réalisation avantageux, la présente invention concerne l'utilisation d'un composé de formule (I-A) ou (I-B) dans laquelle W représente un groupe fonctionnel choisi -CHO, -COR^{a}, -COOH et -COOR^{a}, W représentant en particulier -COOR^{a}.

Selon un mode de réalisation avantageux, la présente invention concerne l'utilisation d'un composé de formule (I-A) ou (I-B) dans lequel W représente un groupe fonctionnel choisi parmi -CONH-SO₂-cyclopropyle, -CH₂-NH-CO-CH₂-CH₃, et le groupe de formule suivante :

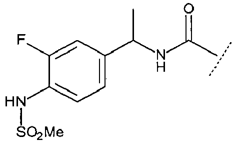

Selon un mode de réalisation avantageux, la présente invention concerne l'utilisation d'un composé de formule (I-A) ou (I-B) dans laquelle R₂ représente H.

Selon un mode de réalisation avantageux, la présente invention concerne l'utilisation d'un composé de formule (I-A) ou (I-B) dans laquelle R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne l'utilisation d'un composé de formule (I-A) ou (I-B) dans lequel R₁, R₂ et R₄ représentent H.

Selon un mode de réalisation avantageux, la présente invention concerne l'utilisation d'un composé de formule (I-A) ou (I-B) dans lequel R₁ représente H.

Selon un mode de réalisation avantageux, la présente invention concerne l'utilisation d'un composé de formule (I-A) ou (I-B) dans lequel R₁ représente H et R₄ représente un aryle, un hétérocycle, un hétéroaryle ou un alkyle tels que définis précédemment.

Selon un mode de réalisation avantageux, la présente invention concerne l'utilisation d'un composé de formule (I-A) ou (I-B) dans lequel R₄ représente H.

Selon un mode de réalisation avantageux, la présente invention concerne l'utilisation d'un composé de formule (I-A) ou (I-B) dans lequel R₄ représente H et R1 représente un aryle, un hétérocycle, un hétéroaryle ou un alkyle tels que définis précédemment.

Selon un mode de réalisation avantageux, la présente invention concerne l'utilisation d'un composé de formule (I-A) ou (I-B) dans lequel R₁ et R₂ représentent H et R₄ représente un aryle, un hétérocycle, un hétéroaryle ou un alkyle tels que définis précédemment.

Selon un mode de réalisation avantageux, la présente invention concerne l'utilisation d'un composé de formule (I-A) ou (I-B) dans lequel R₂ et R₄ représentent H et R₁ représente un aryle, un hétérocycle, un hétéroaryle ou un alkyle tels que définis précédemment.

Selon un mode de réalisation avantageux, la présente invention concerne l'utilisation d'un composé de formule (I-A) ou (I-B) dans lequel R₁ représente H, R₄ représente un aryle, un hétérocycle, un hétéroaryle ou un alkyle tels que définis précédemment, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne l'utilisation d'un composé de formule (I-A) ou (I-B) dans lequel R₄ représente H, R₁ représente un aryle, un hétérocycle, un hétéroaryle ou un alkyle tels que définis précédemment, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne l'utilisation d'un composé de formule (I-A) ou (I-B) dans lequel R₁ et R₄ représentent H, et R₂ est choisi dans le groupe constitué par les groupes -COR^{a}, -COOR^{a}, -CONH₂, -CONHR^{a}, -CONR^{a}R^{b} et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone, éventuellement substitués ;
R^{a} et R^{b} pouvant être reliés pour former un cycle, éventuellement substitué.

Selon un mode de réalisation avantageux, la présente invention concerne l'utilisation d'un composé de formule (I-A) ou (I-B), respectivement de formule particulière (I-C) ou (I-D) telles que définies précédemment.

Selon un mode de réalisation avantageux, la présente invention concerne une utilisation dans laquelle R₃ représente un groupe choisi parmi :

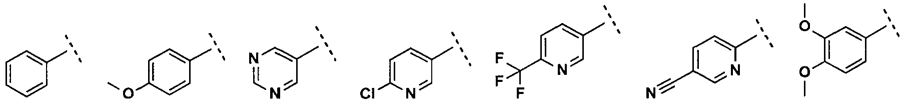

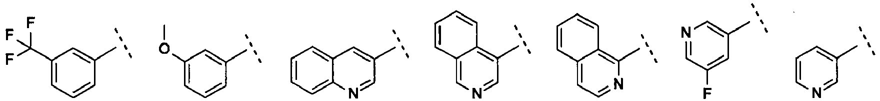

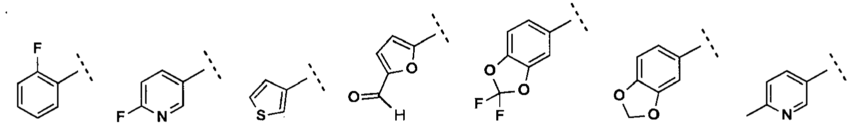

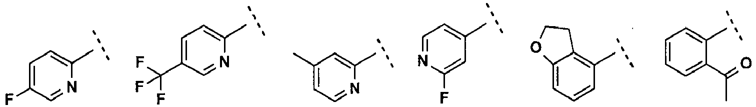

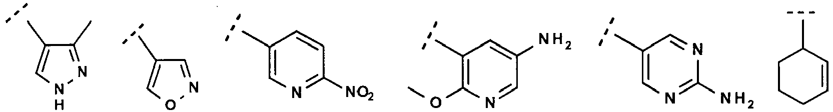

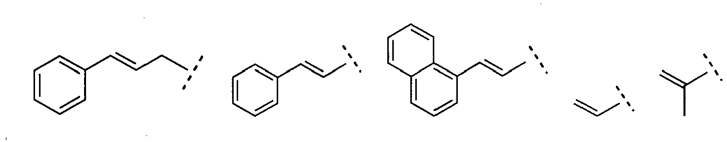

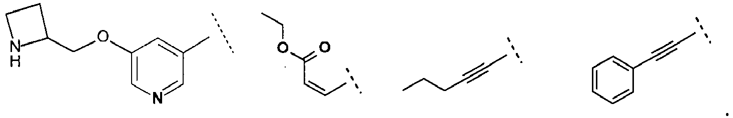

Selon un mode de réalisation avantageux, la présente invention concerne une utilisation dans laquelle R₃ représente un groupe choisi parmi :

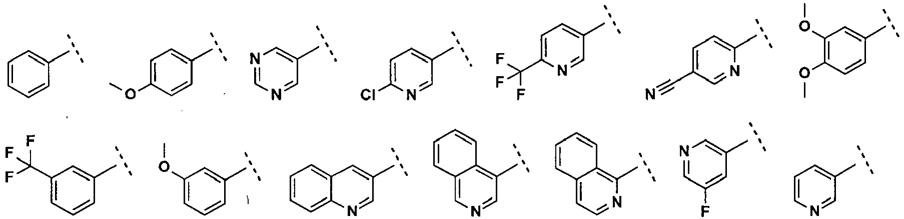

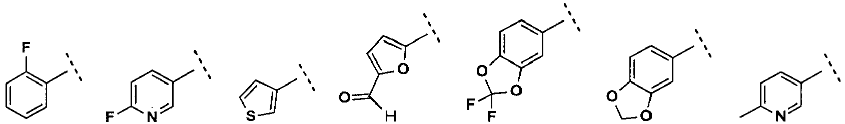

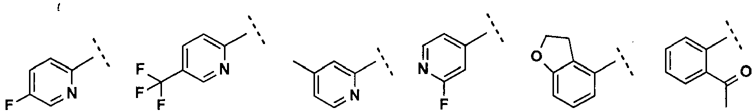

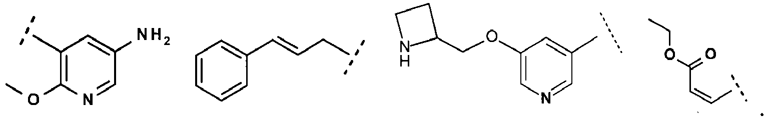

Selon un mode de réalisation avantageux, la présente invention concerne l'utilisation d'un composé choisi dans le groupe constitué des composés de formule suivante et leur énantiomère:

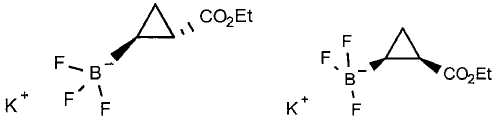

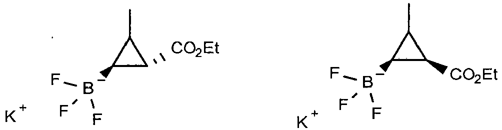

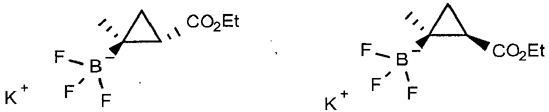

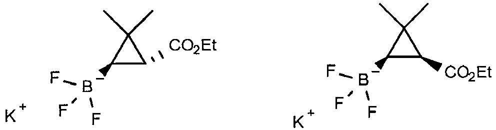

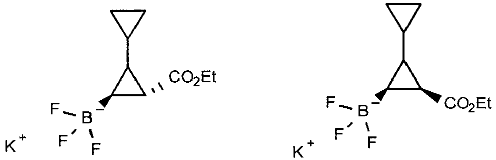

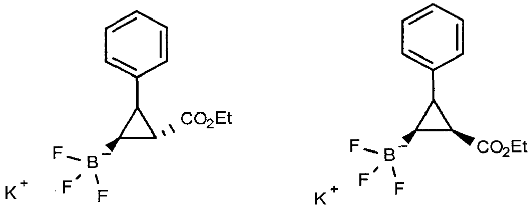

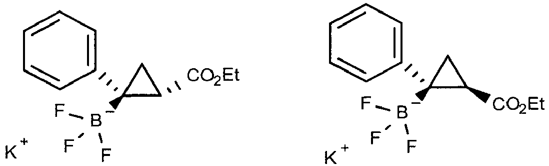

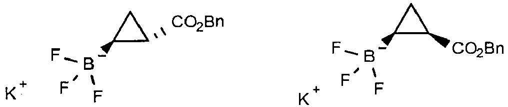

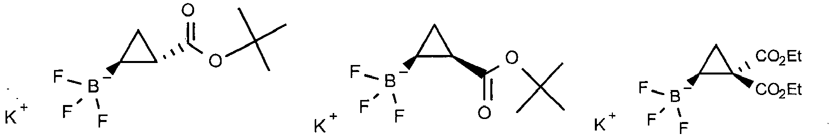

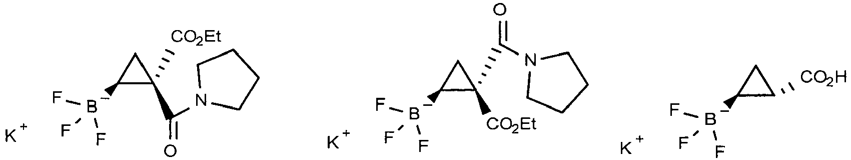

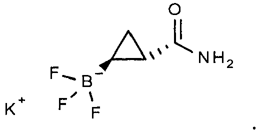

Les exemples qui suivent illustrent l'invention.

### EXEMPLES

### Exemple 1 : Synthèse du [2-éthoxycarbonylcyclopropyl]-trifluoroborate de potassium

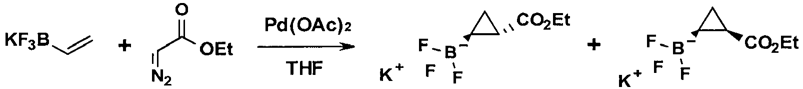

Du vinyle trifluoroborate de potassium (888g, 6.63 mole) est mis en solution dans du THF (9L). L'acétate de palladium (14.88g, 66 mmol) est additionné. Le mélange est chauffé à 35 °C et une solution de diazo acétate d'éthyle (902 ml, 7.72 mole) dans le THF (IL) est additionné goutte à goutte sur 3 heures 40 (exothermie). Le mélange est agité pendant 30 minutes supplémentaires. De l'heptane est ajouté à la solution. La suspension ainsi obtenue est agitée pendant 30 minutes, puis filtrée. Le solide est cristallisé dans 9L d'acétone à - 18 °C. Le solide est filtré et séché pour conduire au produit attendu *cis* (335g, 1.52 mole, 23%). Le filtrat est lavé au noir animal, filtré et concentré à sec. Le produit est cristallisé dans 10L d'éthanol pour conduire au produit attendu *trans* (903g, 4.1 mmol, 62%). 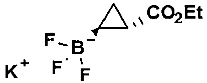 ¹H-RMN (DMSO-d₆, 300 MHz) δ 3.96 (q, J=7.1Hz, 2H), 1.15 (t, J=7.1Hz, 3H), 1.10 (m, 1H), 0.63 (m, 1H), 0.47 (m, 1H), -0.06 (m, 1H). 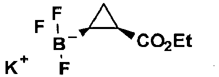 ¹H-RMN (DMSO-d₆, 300 MHz) δ 3.90 (q, J=7.0Hz, 2H), 1.25 (m, 1H), 1.12 (t, J=7.0Hz, 3H), 0.75 (m, 1H), 0.54 (m, 1H), -0.15 (m, 1H).

### Exemple 2 : Synthèse de [2-éthoxycarbonylcyclopropyl]-trifluoroborates de potassium substitués

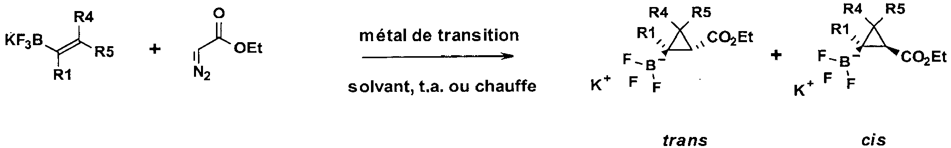

### Procédure générale :

*Méthode A :* A une solution de d'alkenyltrifluoroborate de potassium (1 éq.) dans du tétrahydrofurane est additionné l'acétate de palladium (0,01-0,2 éq.) sous azote. Le mélange est chauffé à 35-70°C et une solution de diazoacétate d'éthyle (1,2 à 5 éq.) dans du tétrahydrofurane est additionné goutte à goutte très lentement. Le milieu réactionnel est agité à cette température pendant 1 à 10 h jusqu'à conversion complète. De l'heptane est ajouté à la solution. La suspension ainsi obtenue est agitée pendant 30 minutes, puis filtrée. Lorsque le dérivé *cis* est suffisamment abondant dans le brut réactionnel, celui-ci peut-être cristallisé dans l'acétone à basse température (-20°C). Le composé *trans* est quant à lui cristallisé dans un mélange acétonitrile / éther diéthylique.

*Méthode B :* A une solution d'alkenyltrifluoroborate de potassium (1 éq.) dans un mélange toluène/dioxane 1/1 est additionné le cuivre acétoacétate (0,2 éq.) sous azote. Le mélange est chauffé à 35-70°C et une solution de diazoacétate d'éthyle (1,2 à 5 éq.) dans du toluène est additionné goutte à goutte très lentement. Le milieu réactionnel est agité à cette température pendant 1 à 10 h jusqu'à conversion complète. Après retour à température ambiante, de l'heptane est ajouté à la solution. La suspension ainsi obtenue est agitée pendant 30 minutes, puis filtrée. Lorsque le dérivé *cis* est suffisamment abondant dans le brut réactionnel, celui-ci peut-être cristallisé dans l'acétone à basse température (-20°C). Le composé *trans* est quant à lui cristallisé dans un mélange acétonitrile / éther diéthylique ou méthanol.

### • Synthèse du [2-éthoxycarbonyl-3-méthyl-cyclopropyl]-trifluoroborate de potassium

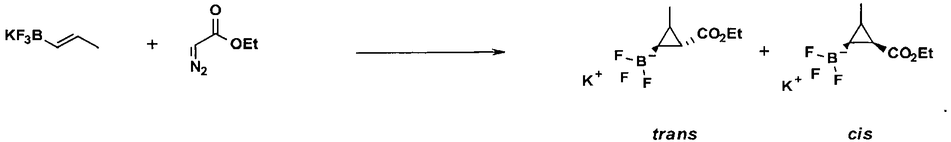

Le produit est obtenu selon la procédure générale.
¹H-RMN (DMSO-d₆, 300 MHz) (composé ***trans***) : δ 3.87 (q, J=7.1Hz, 2H), 1.3-1.0 (m, 2H), 1.09 (t, J=7.1Hz, 3H), 0.95 (d, J=7.9 Hz, 3H), -0.30 (m, 1H).
¹H-RMN (DMSO-d₆, 300 MHz) (composé ***cis***) : δ 3.96 (q, J=7.05Hz, 2H), 1.3-1.0 (m, 2H), 1.15 (t, J=7.05Hz, 3H), 0.90 (d, J=5.3 Hz, 3H), -0.16 (m, 1H).

### • Synthèse du [2-éthoxycarbonyl-1-méthyl-cyclopropyl]-trifluoroborate de potassium

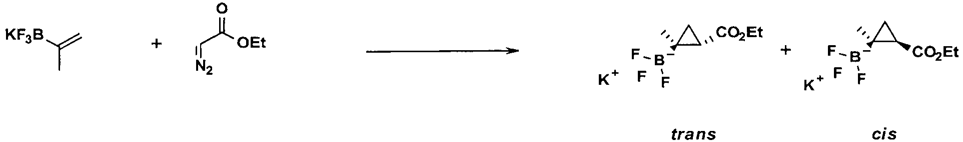

Le produit est obtenu selon la procédure générale.
¹H-RMN (DMSO-d₆, 300 MHz) (Composé ***trans*)** : δ 3.9 (m, 2H), 1.28 (dd, J=4.7Hz, J=7.1Hz, 1H), 1.14 (t, J=7.1Hz, 3H), 0.87 (s, 3H), 0.63 (dd, J=1.9Hz, J=7.1Hz, 1H), 0.45 (m, 1H).
Pas de composé ***cis*** visible.

### • Synthèse du [2-éthoxycarbonyl-3-cyclopropyl-cyclopropyl]-trifluoroborate de potassium

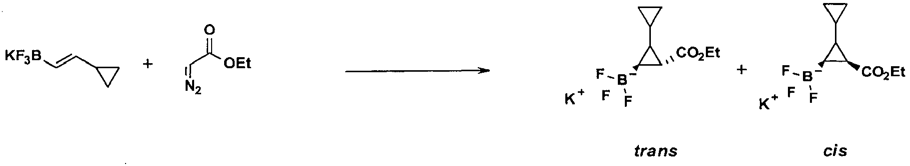

Le produit est obtenu selon la procédure générale.

### • Synthèse du [2-éthoxycarbonyl-3-propyl-cyclopropyl]-trifluoroborate de potassium

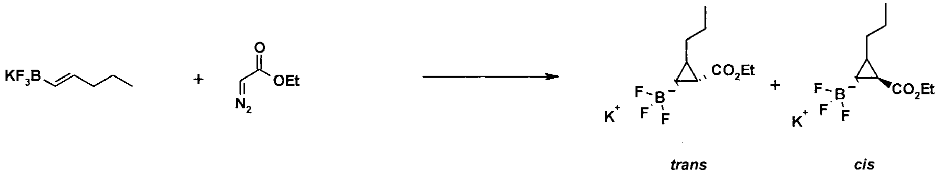

¹H-RMN (DMSO-d₆, 300 MHz) (composé ***trans***) : δ 3.95 (q, J=6.6Hz, 2H), 1.5-1.0 (m, 9H), 0.83 (t, J=6.6Hz, 3H), -0.11 (m, 1H).
¹H-RMN (DMSO-d₆, 300 MHz) (composé ***cis***) : δ 3.88 (q, J=7.1Hz, 2H), 1.5-1.0 (m, 9H), 0.86 (d, J=7.1 Hz, 3H), -0.26 (m, 1H).

### • Synthèse du [2-éthoxycarbonyl-3-phényl-cyclopropyl]-trifluoroborate de potassium

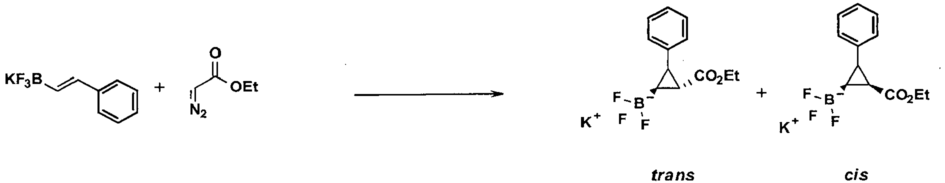

Le produit est obtenu selon la procédure générale.
¹H-RMN (DMSO-d₆, 300 MHz) (composé ***trans***) : δ 7.20-7.0 (m, 5H), 3.73 (m, 2H), 2.18 (t, J=8.6Hz, 1H), 1.56 (dd, J=8.4Hz, J=6.6Hz, 1H), 0.92 (t, J=8.8Hz, 3H), 0.71 (m, 1H). ¹H-RMN (DMSO-d₆, 300 MHz) (composé ***cis***) : δ 7.20-7.0 (m, 5H), 3.94 (q, J=6.4Hz, 2H), 2.29 (m, 1H), 1.59 (m, 1H), 1.09 (t, J=6.4Hz, 3H), 0.37 (m, 1H).

### • Synthèse du [2-éthoxycarbonyl-1-phényl-cyclopropyl]-trifluoroborate de potassium

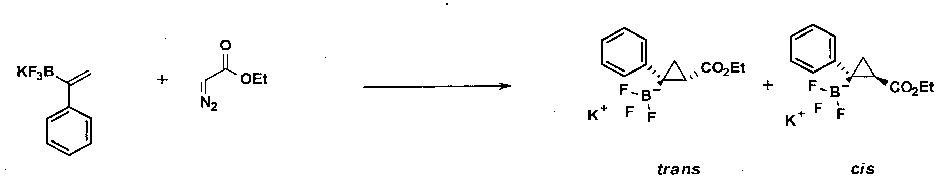

Le produit est obtenu selon la procédure générale.
MS (ES-, MeOH) : 257.2 [M]-

### Exemple 3 : Synthèse de cyclopropyltrifluoroborates de potassium substitués en position 2

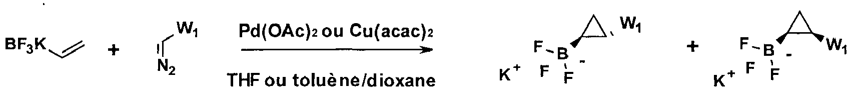

### Méthode générale :

A une solution de vinyltrifluoroborate de potassium (1 éq) dans le THF ou un mélange toluène/dioxane est additionné le catalyseur Pd(OAc)₂ ou Cu(acac)₂ (0,02-0,2 éq.). Le milieu réactionnel est chauffé à 40-70 °C puis le dérivé diazo (2-5 éq.) est additionné au goutte à goutte lent. Le milieu est agité à cette température pendant 1-10 h jusqu'à conversion complète. Après retour à température ambiante, de l'heptane est ajouté à la solution. La suspension ainsi obtenue est agitée pendant 30 minutes, puis filtrée. Lorsque le dérivé *cis* est suffisamment abondant dans le brut réactionnel, celui-ci peut-être cristallisé dans l'acétone à basse température (-20°C). Le composé *trans* est quant à lui cristallisé dans un mélange acétonitrile / éther diéthylique ou méthanol.

### • Synthèse du [2-benzyloxycarbonylcyclopropyl]-trifluoroborate de potassium

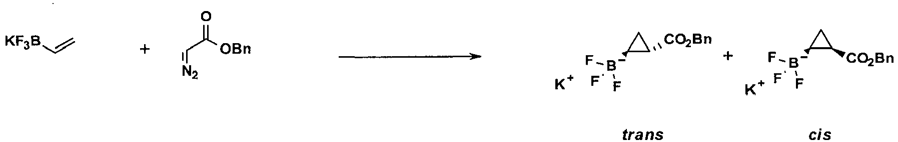

Le produit est obtenu selon la procédure générale.
¹H-RMN (DMSO-d₆, 300 MHz) (composé ***trans***) δ 7.41-7.25 (m, 5H), 5.0 (d, J=1.8Hz, 2H), 1.16 (m, 1H), 0.69 (dm, J=9.7Hz, 1H), 0.53 (td, J=7.3Hz, J=1.8 Hz, 1H), 0.00 (m, 1H). ¹H-RMN (DMSO-d₆, 300 MHz) (composé ***cis***) δ 7.41-7.25 (m, 5H), 4.94 (d, J=12.1Hz, 2H), 1.34 (m, 1H), 0.82 (m, 1H), 0.22 (tm, J=7.9Hz, 1H), -0.08 (m, 1H).

### • Synthèse du [2-tert-butoxycarbonylcyclopropyl]-trifluoroborate de potassium

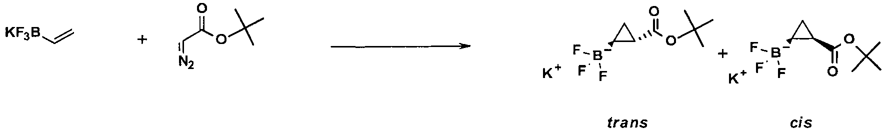

Le produit est obtenu selon la procédure générale.
¹H-RMN (DMSO-d₆, 300 MHz) (composé ***trans***) δ 1.36 (s, 9H), 1.01 (m, 1H), 0.54 (dm, J=9.9Hz, 1H), 0.40 (tm, J=6.6Hz, 1H), -0.11 (m, 1 H).
¹H-RMN (DMSO-d₆, 300 MHz) (composé ***cis***) δ 1.33 (s, 9H), 1.15 (m, 1H), 0.68 (m, 1H), 0.47 (m, 1H), -0.21 (m, 1H).

### • Synthèse du [2,2-bis(éthoxycarbonyl)cyclopropyl]-trifluoroborate de potassium

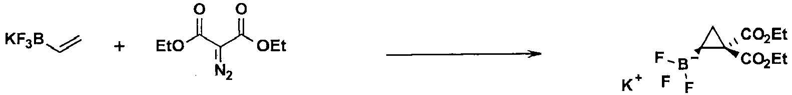

Le produit est obtenu selon la procédure générale.
¹H-RMN (DMSO-d₆, 300 MHz) δ 4.2 (q, 2H, J=7.1Hz), 3.99 (q, 2H, J=8Hz), 1.22 (t, 3H, J=8Hz), 1.13 (t, 3H, J=7.1Hz), 1.02 (dd, 1H, J=9.5Hz, J=1.7Hz), 0.83 (dm, H, J=10.3Hz), 0.54 (m, 1H).

### • Synthèse du [2-éthoxycarbonyl-2-(pyrrolidine-1-carbonyl)cyclopropyl]-trifluoroborate de potassium

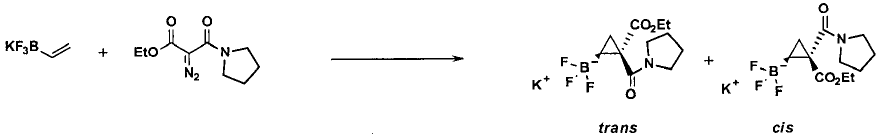

Le produit est obtenu selon la procédure générale.
¹H-RMN (DMSO-d₆, 300 MHz) mélange *cis*/*trans* δ 3.97 (m, 4H), 3.27-3.49 (m, 8H), 0.98-1.24 (m, 12H), 1.09 (t, 3H, J=8,5Hz), 0.77 (m, 2H), 0.58 (m, 1H), 0.39 (m, 1H).

### Exemple 4 : Synthèse de l'acide (trans)-2-(4,4,5,5-tetraméthyl-1,3,2-dioxaborolan-2-yl)cyclopropanecarboxylique

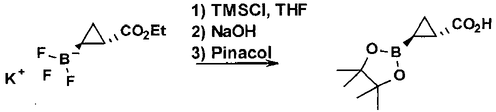

Le dérivé trifluoroborate de potassium (exemple 1, 15g, 68.2 mmol) est mis en solution dans du THF (150 ml). Du chlorure de triméthylsilane (18.2g, 143.2 mmol) est ajouté goutte à goutte. Le milieu est agité à température ambiante pendant 30 minutes. Le milieu réactionnel est versé dans 600 ml d'eau. Le THF est concentré au rota et une solution d'un molaire d'hydroxyde de sodium est ajouté jusqu'a pH basique (environ 50 ml). Le milieu est agité à température ambiante pendant la nuit. Du NaOH en pastilles (2g) et une solution d'hydroxyde de sodium (6N) sont additionnés et le milieu est agité pendant la nuit. Le milieu est concentré à sec. On reprend dans 700 ml de toluène et du pinacol (8.46g, 71.6 mmol) sont additionnés. Le milieu est filtré sur Célite et concentré à sec. L'huile amorphe est trituré dans du pentane (50 ml) pour obtenir un solide blanc (2.85g, 13.4 mmol). Le filtrat est placé au congélateur pour conduire à un deuxième lot de produit (5.8g, 27.4 mmol, rendement global 60%). ¹H-RMN (DMSO-d₆, 300 MHz) δ 12.25 (bs, 1H), 1.51 (m, 1H), 1.17 (s, 12H), 1.04 (m, 1H), 0.84 (m, 1H), 0.30 (m, 1 H).

### Exemple 5 : Synthèse du [(trans)-2-méthylcyclopropyl]trifluoroborate de potassium

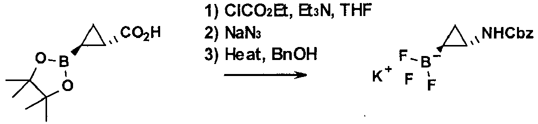

L'acide carboxylique (**exemple 4,** 8.65g, 40.8 mmol) est mis en solution dans du THF (216 ml). Le milieu est refroidi à 0 °C on ajoute de la triéthylamine (8.5 ml, 61.2 mmol). Ensuite sont ajoutés goutte à goutte de l'éthylchloroformate (6.64g, 61.2 mmol). Le milieu est agité à température ambiante pendant 1 heure. On refroidi à 0°C et une solution de azoture de sodium (132.6g, 2.04 mole) dans de l'eau (325 ml) sont additionnés goutte à goutte. Le milieu est agité à température ambiante pendant la nuit. De l'eau (200 ml) est ajouté et le produit attendu est extrait 2 fois par de l'éther isopropylique. La phase organique est séchée sur sulfate de magnésium et filtrée. Du toluène (50 ml) et de l'alcool benzylique (4.3 ml) sont additionnés et le milieu est chauffé à 105 °C. Le THF et l'éther isopropylique sont distillés du milieu. Après une nuit à 105 °C, le milieu est concentré à sec. Le brut réactionnel est purifié 2 fois par chromatographie sur gel de silice ce qui ne permets pas de supprimer l'excès d'alcool benzylique. Le produit (6.48 g) est alors remis dans un mélange eau (16.2 ml)/méthanol (78 ml) et du KHF₂ (11.17g) est ajouté et le milieu est agité à température ambiante pendant la nuit. La suspension est concentrée à sec et le solide est trituré dans de l'acétonitrile à 90 °C pendant 4 heures. Le filtrat est concentré pour conduire à un solide blanc. ¹H-RMN (DMSO-d₆, 300 MHz) δ 7.33 (m, 5H), 6.99 (bd, 0.8H), 6.7 (bs, 0.2H), 4.96 (s, 2H), 2.16 (m, 1 H), 0.12 (m, 1H), 0.0 (m, 1H), -0.52 (m, 1H).

### Exemple 6 : Synthèse du [(trans)-2-carboxycyclopropyl]-trifluoroborate de potassium

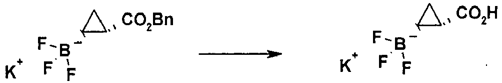

A une solution d'ester (**exemple 3**, 1 mmol) dans 5 ml de méthanol, est additionné sous atmosphère inerte, du palladium sur charbon (0,2 mmol, 0,2 éq.). Le milieu réactionnel est placé sous atmosphère d'hydrogène (1 atm) pendant 5 heures, filtré sur fritté puis concentré à sec pour conduire à l'acide carboxylique attendu.
¹H-RMN (DMSO-d₆, 300 MHz) (composé ***trans***) δ 1.12 (m, 1H), 0.68 (d, J=10.0Hz, 1H), 0.52 (t, J=7.3Hz, 1H), 0.00 (m, 1H).

### Exemple 10 : Synthèse de l'acide [(trans)-2-éthoxycarbonylcyclopropyl]boronique

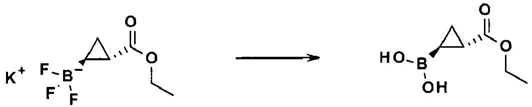

A une solution de dérivé trifluoroborate de potassium (**exemple 1**, 1 éq.) dans l'eau est additionné du silica gel (1 éq.). Le milieu réactionnel est agité à température ambiante ou à chaud pendant 4 h puis filtré sur fritté. La phase aqueuse est extraite 3 fois par de l'acétate d'éthyle. Les phases organiques combinées sont lavées par de la saumure, séchées sur sulfate de magnésium et concentrées à sec pour conduire au dérivé acide boronique attendu.
¹H-RMN (DMSO-d₆, 300 MHz) (composé ***trans***) 5 4.03 (t, J=7.4Hz, 2H), 1.58 (m, 1H), 1.17 (t, J=7.4Hz, 3H), 0.97 (m, 1H), 0.87 (td, J=7.5Hz, J=2.7Hz, 1H), 0.30 (m, 1H).

### Exemple 11 : Synthèse de l'ester MIDA de l'acide [(trans)-2-éthoxycarbonylcyclopropyl]boronique

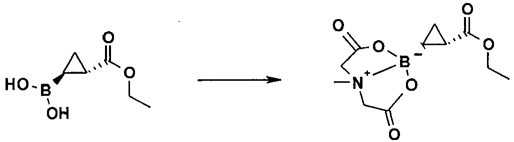

A une solution de dérivé boronique acide (**exemple 10**, 1 éq.) dans un mélange toluène/DMSO est additionné l'acide N-méthyliminodiacétique (1 éq.). Le milieu réactionnel est chauffé à reflux avec un Dean-Stark jusqu'à disparition complète du produit de départ. Après retour à température ambiante, le milieu est hydrolysé par ajout d'eau et extrait 3 fois par un mélange THF/Et₂O 2/1. Les phases organiques sont séchées sur sulfate de magnésium, filtrées sur fritté et concentrées à sec pour conduire à un brut réactionnel. Une purification par chromatographie sur silice (éluant : Heptane / AcOEt) conduit au dérivé attendu.
¹H-RMN (DMSO-d₆, 300 MHz) (composé ***trans***) δ 4.21 (dd, J=16.9Hz, J=4.7Hz, 2H), 4.03 (m, 4H), 2.95 (s, 3H), 1.37 (m, 1H), 1.16 (t, J=7.1Hz, 3H), 0.96 (m, 1H), 0.68 (td, J=7.6Hz, J=3.1Hz, 1H), 0.42 (m, 1 H).

### Exemple 13 : Synthèse du vinyloxymethylbenzene

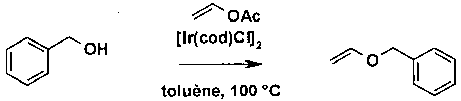

A une suspension de [Ir(cod)Cl]₂ (336 mg, 0.500 mmol, 1 mol %) et Na₂CO₃ (3.18 g, 30.0 mmol, 0.6 équiv) dans du toluène (50 mL) sont ajoutés de l'alcool benzylique (5.18 mL, 50.0 mmol) et de l'acétate de vinyle (9.22 mL, 100 mmol, 2.0 équiv). Après 2 heures d'agitation à 100°C, le mélange obtenu est refroidi à température ambiante et filtré sur Célite. Le filtrat est concentré sous pression réduite et le résidu purifié par chromatographie flash sur gel de silice (éther de pétrol/EtOAc: 98/2) pour donner une huile jaune (5.15 g, 77%).
¹H-RMN (CDCl₃, 400 MHz) *δ* 7.39-7.29 (m, 5H), 6.57 (dd, *J* = 14.3 Hz and *J* = 6.8 Hz, 1H), 4.76 (s, 2H), 4.30 (dd, *J* = 14.3 Hz and *J* = 2.0 Hz, 1H), 4.08 (dd, *J* = 6.8 Hz and *J* = 2.0 Hz, 1H).

### Exemple 14: synthèse du 2-[(E)-2-benzyloxy)éthenyl]-4,4,5,5-tetraméthyl-1,3,2-dioxaborolane (2).

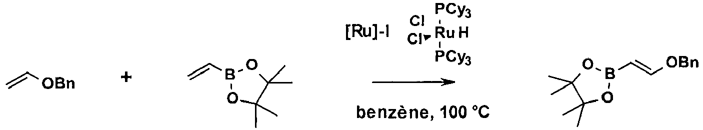

A une solution du benzyle vinyle éther (**exemple 13**, 201 mg, 1.50 mmol, 3.0 equiv) dans le benzène (5.0 mL) sont ajouté le 4,4,5,5-tetramethyl-2-vinyl-1,3,2-dioxaborolane (84.8 µL, 0.500 mmol) et le catalyseur au ruthénium **[Ru]-1** (préparé par réaction du catalyseur Grubbs I avec du 1-propanol et Et₃N dans du toluène à 75 °C pendant 16 h, selon Dinger, M. B.; Mol, J. C. *Organometallics* **2003**, *22*, 1089-1095) (3.63 mg, 5.00 µmol, 1 mol %). Après 24 heures d'agitation à 100°C, le mélange obtenu est refroidi à température ambiante, concentré sous pression réduite et le résidu purifié par chromatographie flash sur gel de silice (éther de pétrol/EtOAc: 90/10) pour donner une huile jaune.
**IR** 1632, 1607, 1367, 1309, 1124, 1104, 970, 851, 814, 736, 696, 656 cm⁻¹; **¹H-RMN (CDCl₃, 400 MHz)** *δ* 7.36-7.29 (m, 5H), 7.15 (d, *J* = 14.4 Hz, 1H), 4.83 (s, 2H), 4.56 (d, *J* = 14.3 Hz, 1H), 1.26 (s, 12H); **¹³C**-**RMN** (100 MHz, CDCl₃) *δ* 162.7 (d), 136.4 (s), 128.5 (d), 128.0 (d), 127.6 (d), 82.7 (s, 2C), 70.6 (t), 24.7 (q, 4C); **EI-MS** *m*/*z* (intensité relative) 260 (M^{+•}, 0.3), 160 (2), 133 (M-Bpin⁺, 2), 117 (2), 116 (13), 92 (11), 91 (100), 85 (3), 84 (6), 83 (8), 69 (2), 65 (8), 59 (2), 57 (2), 55 (2). **HRMS** calcd for C₁₅H₂₁BO₃Na (M+Na⁺): 283.14760. Found: 283.14798.

### Exemple 15 : Synthèse du (2R*)-2-(benzyloxy)-1,1-dibromocyclopropane

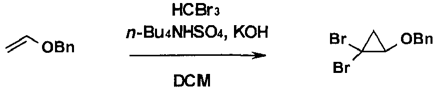

A une solution de benzoate de vinyle (**exemple 13**, 4.12 g, 30.7 mmol) dans le dichlorométhane (250 mL) à 0°C, sont additionnés successivement de la potasse (24.8 g, 442 mmol, 14.4 équivalents), du sulfate de tetrabutylammonium (3.13 g, 9.21 mmol, 0.3 équivalents) puis du tribromométhane (24.7 mL, 282 mmol, 9.2 équivalents). Après 2 heures d'agitation à température ambiante, le milieu réactionnel est filtré sur célite, rincé au dichloromethane, puis 400 ml d'eau sont ajoutés au filtrat. Les phases sont séparées et la phase aqueuse est extraite par de l'éther diéthylique. Les phases organiques combinées sont séchées sur sulfate de magnésium, filtrées puis évaporées à sec sous pression réduite. Le brut réactionnel est purifié par chromatographie sur gel de silice (Eluant : Ether de pétrole/AcOEt: 98/2) pour conduire à 8.73 g (93%) du composé **4** sous la forme d'une huile orange.
**¹H RMN** (400 MHz, CDCl₃) *δ* 7.44-7.31 (m, 5H), 4.90 (d, AB syst, *J* = 11.2 Hz, 1H), 4.68 (d, AB syst, *J* = 11.2 Hz, 1H), 3.63 (dd, *J* = 8.1 Hz et *J* = 5.0 Hz, 1H), 1.87 (dd, *J* = 8.9 Hz et *J* = 8.2 Hz, 1H), 1.75 (dd, *J* = 8.9 Hz et *J* = 5.0 Hz, 1H); **¹³C RMN** (100 MHz, CDCl₃) *δ* 136.4 (s), 128.6 (d, 2C), 128.4 (d, 2C), 128.3 (d), 73.4 (t), 62.9 (d), 29.8 (t), 26.8 (s).

### Exemple 16 : Synthèse du ((trans)-1-(benzyloxy)-2-bromocyclopropane

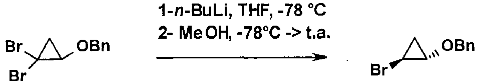

A une solution de dibromo-cyclopropane (**exemple 15**, 546 mg, 1.78 mmol) dans le THF (10 mL) à -78 °C, est additionné goutte à goute le *n*-butyllithium (860 µL, 2.5 M dans l'hexane, 2.14 mmol, 1.2 équivalents). Après 10 minutes d'agitation à -78 °C, 4 ml de méthanol sont additionnés. Le milieu réactionnel est agité 15 minutes puis laissé remonter à température ambiante. Après 30 minutes d'agitation à cette température, de l'eau (10 mL) et de l'éther diéthylique (10 mL) sont additionnés, puis la phase aqueuse est extraite par de l'éther diéthylique. Les phases organiques combinées sont séchées sur sulfate de magnésium, filtrées puis évaporées à sec sous pression réduite. Le brut réactionnel est purifié par chromatographie sur gel de silice (Ether de pétrole/AcOEt : 98/2) pour conduire à 334 mg (82%) du composé **6** sous la forme d'une huile jaune.
**¹H RMN** (400 MHz, CDCl₃) *δ* 7.39-7.30 (m, 5H), 4.61 (d, AB syst, *J* = 11.7 Hz, 1H), 4.57 (d, AB syst, *J* = 11.7 Hz, 1H), 3.56 (ddd, *J* = 7.4 Hz, *J* = 3.9 Hz et *J* = 1.7 Hz, 1H), 2.93 (ddd, *J* = 8.9 Hz, *J* = 5.0 Hz et *J* = 1.7 Hz, 1H), 1.38 (ddd, *J* = 8.9 Hz, *J* = 7.8 Hz et *J* = 3.9 Hz, 1H), 1.09 (ddd, apparent td, *J* = 7.5 Hz et *J* = 5.0 Hz, 1H); **¹³C RMN** (100 MHz, CDCl₃) *δ* 137.1 (s), 128.5 (d, 2C), 128.1 (d, 2C), 128.0 (d), 73.0 (t), 59.9 (d), 17.8 (d), 17.7 (t)

### Exemple 17 : Synthèse du 2-[(trans)-2-(benzyloxy)cyclopropyl]-4,4,5,5-tetraméthyl-1,3,2-dioxaborolane

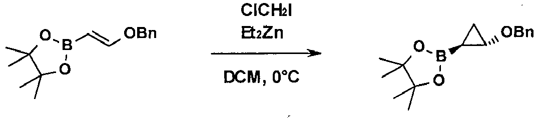

A partir de **l'exemple 14**: A une solution de vinyléther (**exemple 14**, 58.3 mg, 0.224 mmol) dans le dichlorométhane (2.5 mL) à 0 °C, sont additionnés le diéthylzinc (450 µL, 1 M dans l'hexane, 0.448 mmol, 2.0 équivalents) puis le chloroiodométhane (65.3 µL, 0.896 mmol, 4.0 équivalents). Après 1,5 heures à 0 °C, la réaction est hydrolysée par ajout d'une solution aqueuse saturée de chlorure d'ammonium (10 mL) puis la phase aqueuse est extraite par du dichlorométhane (10 mL). Les phases organiques combinées sont séchées sur sulfate de magnésium, filtrées puis évaporées à sec sous pression réduite. Le résidu est purifié par chromatographie sur gel de silice (Eluant : éther de pétrole/AcOEt : 90/10) pour conduire à 39.7 mg (65%) de composé **3** sous la forme d'une huile incolore.

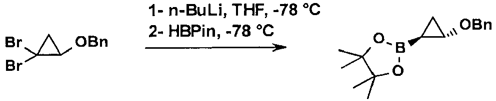

A partir de **l'exemple 15**: A une solution de dibromocyclopropane (**exemple 15**, 91.8 mg, 0.300 mmol) dans le THF (3.0 mL) à -78 °C, est additionné le *n*-butyllithium (144 µL, 2.5 M dans l'hexane, 0.360 mmol, 1.2 équivalents). Après 15 minutes à -78 °C, le pinacolborane (600 µL, 1 M dans le THF, 2.0 équivalents) est additionné et le mélange réactionnel est chauffé à 50 °C pendant 16 heures. La réaction est ensuite refroidie à température ambiante puis hydrolysée par ajout d'une solution aqueuse saturée en chlorure d'ammonium. La phase aqueuse est extraite par de l'éther diéthylique puis les phases organiques combinées sont séchées sur sulfate de magnésium, filtrées et évaporées à sec sous pression réduite. Le résidu est purifié par chromatographie sur gel de silice (Eluant : Ether de pétrole/AcOEt : 90/10) pour conduire au composé **3** sous la forme d'une huile jaune pâle.

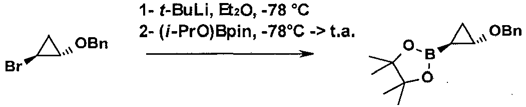

A partir de **l'exemple 16**: A une solution de bromocyclopropane (**exemple 16**, 4.50 g, 19.8 mmol) dans l'éther diéthylique (120 mL) à -78 °C, est additionné le *t*-butyllithium (23.3 mL, 1.7 M dans l'hexane, 39.6 mmol, 2.0 équivalents). Après 30 minutes à cette température, l'isopropoxyboronate de pinacol (12.1 mL, 59.4 mmol, 3.0 équivalents) est additionné au goutte à goutte puis le milieu réactionnel est laissé remonter doucement à température ambiante pendant 2 heures. Après 30 minutes à cette température, le milieu est hydrolysé par ajout d'une solution aqueuse saturée en chlorure d'ammonium (150 mL), les phases sont séparées puis la phase aqueuse est extraite par de l'éther diéthylique. Les phases organiques combinées sont séchées sur sulfate de magnésium, filtrées puis évaporées à sec sous pression réduite. Le résidu est purifié par chromatographie sur gel de silice (Eluant : éther de pétrole/AcOEt: 90/10) pour conduire à 4.51 g (83%) de composé **3** sous la forme d'une huile jaune.
**IR** 1435, 1411, 1380, 1371, 1317, 1199, 1142, 1089, 1067, 854, 735, 697, 700 cm⁻¹; **¹H RMN** (400 MHz, CDCl₃) *δ* 7.34-7.25 (m, 5H), 4.57 (d, AB syst, *J* = 11.5 Hz, 1H), 4.51 (d, AB syst, *J* = 11.5 Hz, 1H), 3.46 (ddd, apparent dt, *J* = 5.9 Hz et *J* = 3.3 Hz, 1H), 1.21 (s, 6H), 1.20 (s, 6H), 0.99 (ddd, *J* = 11.3 Hz, *J* = 4.6 Hz et *J* = 3.3 Hz, 1H), 0.73 (ddd, *J* = 7.5 Hz, *J* = 5.9 Hz et *J* = 4.6 Hz, 1 H), 0.19 (ddd, *J* = 11.3 Hz, *J* = 7.5 Hz et *J* = 3.3 Hz, 1H); **¹³C RMN** (100 MHz, CDCl₃) *δ* 137.8 (s), 128.3 (d, 2C), 128.1 (d, 2C), 127.6 (d), 83.0 (s, 2C), 73.1 (t), 58.1 (d), 24.64 (q, 2C), 24.59 (q, 2C), 11.7 (t); **EI-MS** *m*/*z* (intensité relative) 183 (M-Bn⁺, 5), 174 (2), 145 (2), 144 (3), 131 (2), 130 (8), 129 (4), 104 (3), 101 (3), 92 (11), 91 (100), 85 (3), 84 (7), 83 (10), 79 (2), 69 (2), 67 (2), 65 (7), 59 (2), 57 (2), 55 (5). **HRMS** calculé pour C₁₆H₂₃BO₃Na (M+Na⁺): 297.16325. Trouvé: 297.16352.

### Exemple 18: Synthèse du ((trans)-2-(benzyloxy)cyclopropyltrifluoroborate de potassium

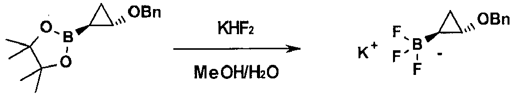

A une solution de dioxaborolane (**exemple 17**, 4.48 g, 16.3 mmol) dans le méthanol (70 mL) à température ambiante, sont additionnés successivement le difluorure de potassium (8.93 g, 114 mmol, 7.0 équivalents) puis l'eau (14 mL). Après 3 heures d'agitation vigoureuse à température ambiante, le milieu réactionnel est concentré sous pression réduite. 100 ml d'acétonitrile sont ajoutés au milieu puis évaporé à sec. Cette opération est réalisée plusieurs fois jusqu'à élimination complète de l'eau. Le résidu pâteux est mis en suspension dans l'acétonitrile (50 mL) puis filtré sur coton pour éliminer l'excès de KHF₂. Le filtrat est évaporé sous pression réduite pour conduire à un solide qui est lavé à l'éther diéthylique. Après filtration, 3.44 g (83%) de composé **8** sont obtenus sous la forme d'un solide blanc.
**IR** 1440, 1364, 1299, 1210, 1090, 1035, 930, 909, 871, 794, 724, 693 cm⁻¹; **¹H RMN** (400 MHz, D₆-acetone) *δ* 7.37-7.30 (m, 4H), 7.28-7.23 (m, 1H), 4.51 (s, 2H), 3.20 (ddd, *J* = 5.3 Hz, *J* = 3.5 Hz et *J* = 2.3 Hz, 1H), 0.35 (m, 1H), 0.25 (ddd, *J* = 7.5 Hz, *J* = 5.3 Hz et *J* = 3.6 Hz, 1H), -0.16 (ddd, apparent m, 1H); **¹³C RMN** (100 MHz, D₆-acetone) *δ* 140.3 (s), 128.4 (d, 2C), 127.9 (d, 2C), 127.3 (d), 72.5 (t), 58.4 (d, J²_{13C-B} = 3.3 Hz), 8.6 (t, J²_{13C-B} = 3.3 Hz), mp > 172 °C (dec.).

### Exemple 20 : synthèse du benzyl (2,2,2-tribromoéthyl) éther

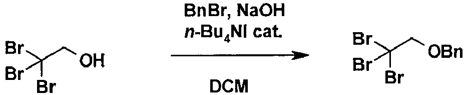

A une solution de 2,2,2-tribromoéthanol (10.0 g, 35.3 mmol) dans le dichlorométhane (25 mL) à 0 °C, sont additionnés le bromure de benzyle (8.41 mL, 70.7 mmol, 2.0 équivalents), le iodure de *tetra*butylammonium (653 mg, 1.77 mmol, 5 mol %) puis 25 ml d'une solution aqueuse de soude (2.12 g, 53.0 mmol, 1.5 équivalents). Après 3 heures d'agitation à température ambiante, les phases sont séparées et la phase aqueuse est extraite par du dichloromethane. Les phases organiques combinées sont séchées sur sulfate de magnésium, filtrées puis évaporées à sec sous pression réduite. Le résidu est purifié par chromatographie sur gel de silice (Eluant : éther de pétrole/AcOEt: 98/2 à 95/5) pour conduire à 12.1 g (92%) de composé **9** sous la forme d'un solide blanc de bas point de fusion.
**IR** 1453, 1401, 1348, 1111, 1077, 991, 973, 913, 755, 726, 696, 629, 602 cm⁻¹; **¹H RMN** (400 MHz, CDCl₃) *δ* 7.45-7.31 (m, 5H), 4.90 (s, 2H), 4.24 (s, 2H); **¹³C RMN** (100 MHz, CDCl₃) *δ* 136.9 (s), 128.6 (d, 2C), 128.2 (d), 127.8 (d, 2C), 84.8 (t), 74.0 (t), 40.2 (s); **EI-MS** *m*/*z* (intensité relative) 376 (0.3), 374 (0.8), 372 (0.8) et 372 (0.3) (M^{+•}), 265 (2), 263 (4) et 261 (2) (M-OBn⁺), 121 (7), 107 (2), 106 (2), 105 (4), 92 (10), 91 (100), 90 (2), 89 (2), 79 (7), 78 (3), 77 (8), 65 (10), 63 (2), 51 (5), 50 (2).

### Exemple 21 : synthèse du (Z)-2-benzyloxy-1-bromoéthylène

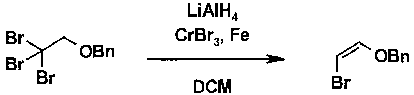

A une suspension de tribromure de chrome anhydre (préparé par séchage sous vide à 90°C d'une poudre finement divisée de tribromure de chrome hexahydraté) (948 mg, 3.22 mmol, 1.2 équivalents) et de fer en poudre (599 mg, 10.7 mmol, 4.0 équivalents) dans le THF (40 mL) à 0°C, est additionné l'aluminohydrure de lithium (61.1 mg, 1.61 mmol, 0.6 équivalents). Après 30 minutes d'agitation à température ambiante, l'éther de 2,2,2-tribromoéthyle (**exemple 20**, 1.00 g, 2.68 mmol) est ajouté en une portion. Le milieu réactionnel est agité pendant 2.5 heures avant d'être filtré sur un pad de gel de silice puis rincé à l'éther diéthylique. 200 ml d'eau sont ajoutés au filtrat, les phases sont séparées et la phase aqueuse est extraite par de l'éther diéthylique. Les phases organiques combinées sont séchées sur sulfate de magnésium, filtrées puis évaporées à sec sous pression réduite. Le résidu est purifié par chromatographie sur gel de silice (Eluant : éther de pétrole/AcOEt: 96/4) pour conduire à 487 mg (85%) de composé **10** sous la forme d'une huile jaune pâle.
**¹H RMN** (400 MHz, CDCl₃) *δ* 7.41-7.31 (m, 5H), 6.65 (d, *J* = 4.2 Hz, 1H), 5.16 (d, *J* = 4.2 Hz, 1H), 4.97 (s, 2H); **¹³C RMN** (100 MHz, CDCl₃) *δ* 147.0 (d), 136.4 (s), 128.6 (d, 2C), 128.3 (d), 127.4 (d, 2C), 83.3 (d), 74.4 (t)

### Exemple 22 : synthèse du (cis)-1-(benzyloxy)-2-bromocyclopropane

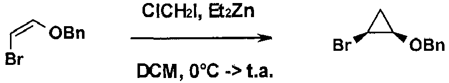

A une solution d'éther vinylique (**exemple 21**, 480 mg, 2.25 mmol) dans le dichlorométhane (15 mL) à 0°C, sont additionnées goutte à goutte le diéthylzinc (4.51 mL, 1 M dans l'hexane, 4.51 mmol, 2.0 équivalents) puis le chloroiodométhane (677 µL, 9.01 mmol, 4.0 équivalents). Après 9 heures d'agitation à température ambiante, une quantité supplémentaire de diéthylzinc (4.51 mL, 1 M dans l'hexane, 4.51 mmol, 2.0 équivalents) et de chloroiodométhane (677 µL, 9.01 mmol, 4.0 équivalents) sont ajoutés et le milieu réactionnel est agité 15 heures supplémentaires. Le milieu est hydrolysé par ajout, à 0°C, d'une solution aqueuse saturée en chlorure d'ammonium (15 mL), les phases sont séparées et la phase aqueuse est extraite par du dichloromethane. Les phases organiques combinées sont séchées sur sulfate de magnésium, filtrées puis évaporées à sec sous pression réduite. Le résidu est purifié par chromatographie sur gel de silice (Eluant : éther de pétrole/AcOEt : 95/5 à 90/10) pour conduire au composé attendu sous la forme d'une huile jaune pâle.
**IR** 1454, 1347, 1259, 1207, 1140, 1093, 1047, 1029, 973, 804, 780, 735, 696 cm⁻¹; **¹H RMN** (400 MHz, CDCl₃) *δ* 7.43-7.28 (m, 5H), 4.79 (d, AB syst, *J* = 11.3 Hz, 1H), 4.59 (d, AB syst, *J* = 11.3 Hz, 1H), 3.27 (ddd, dt apparent, *J* = 7.3 Hz et *J* = 4.8 Hz, 1H), 2.88 (ddd, dt apparent, *J* = 8.3 Hz et *J* = 5.2 Hz, 1H), 1.30 (ddd, q apparent, *J* = 7.8 Hz, 1H), 1.07 (ddd, *J* = 7.8 Hz, *J* = 5.4 Hz et *J* = 4.5 Hz, 1H); **¹³C RMN** (100 MHz, CDCl₃) *δ* 137.3 (s), 128.4 (d, 2C), 128.1 (d, 2C), 127.9 (d), 72.9 (t), 53.7 (d), 20.5 (d), 16.0 (t); **EI-MS** *m*/*z* (intensité relative) 147 (M-Br⁺, 2), 117 (4), 92 (8), 91 (100), 89 (3), 77 (2), 65 (13), 63 (2), 51 (3).

### Exemple 23 : synthèse du 2-[(cis)-2-(benzyloxy)cyclopropyl]-4,4,5,5-tetraméthyl-1,3,2-dioxaborolane

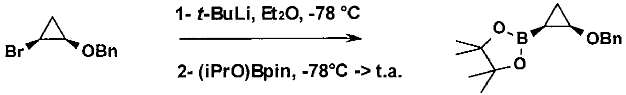

A une solution de bromocyclopropane (**exemple 22**, 351 mg, 1.55 mmol) dans l'éther diéthylique (10 mL) à -78 °C, est additionné goutte à goutte le *tert*-butyllithium (1.82 mL, 1.7 M dans l'hexane, 3.09 mmol, 2.0 équivalents). Après 30 minutes d'agitation à cette température, l'isopropoxyborate de pinacol (946 µL, 4.64 mmol, 3.0 équivalents) est ajouté goutte à goutte et le milieu réactionnel est laissé remonter doucement à température ambiante pendant 2 heures. Après 30 minutes d'agitation à cette température, le milieu est hydrolysé par ajout d'une solution aqueuse saturée en chlorure d'ammonium (20 mL). Les phases sont séparées et la phase aqueuse est extraite par de l'éther diéthylique. Les phases organiques combinées sont séchées sur sulfate de magnésium, filtrées puis évaporées à sec sous pression réduite. Le résidu est purifié par chromatographie sur gel de silice (Eluant : éther de pétrole /AcOEt : 90/10) pour conduire à 254 mg (60%) de composé attendu sous la forme d'une huile jaune pâle.
**IR** 1435, 1404, 1350, 1319, 1213, 1143, 1046, 1027, 951, 863, 851, 735, 697, 670, 611 cm⁻¹; **¹H RMN** (400 MHz, CDCl₃) *δ* 7.39-7.24 (m, 5H), 4.57 (d, AB syst, *J* = 11.7 Hz, 1H), 4.46 (d, AB syst, *J* = 11.7 Hz, 1H), 3.60 (ddd, *J* = 6.8 Hz, *J* = 6.1 Hz et *J* = 3.6 Hz, 1H), 1.25 (s, 6H), 1.23 (s, 6H), 1.05 (ddd, *J* = 8.1 Hz, *J* = 4.7 Hz et *J* = 3.6 Hz, 1H), 0.84 (ddd, *J* = 10.3 Hz, *J* = 6.1 Hz et *J* = 4.8 Hz, 1 H), 0.05 (ddd, *J* = 10.3 Hz, *J* = 8.1 Hz et *J* = 6.8 Hz, 1H); **¹³C RMN** (100 MHz, CDCl₃) *δ* 138.2 (s), 128.1 (d, 2C), 127.8 (d, 2C), 127.4 (d), 83.1 (s, 2C), 72.9 (t), 58.2 (d), 24.8 (q, 2C), 24.7 (q, 2C), 11.3 (t); **EI-MS** *m*/*z* (intensité relative) 183 (M-Bn⁺, 5), 174 (2), 144 (3), 130 (8), 129 (5), 125 (4), 104 (3), 101 (3), 92 (11), 91 (100), 85 (4), 84 (8), 83 (10), 81 (6), 79 (4), 65 (8), 57 (3), 55 (7). **HRMS** calculé pour C₁₆H₂₃O₃BNa (M+Na⁺): 297.16325. Trouvé : 297.16348.

### Exemple 24 : synthèse du ((cis)-2-(benzyloxy)cyclopropyltrifluoroborate de potassium

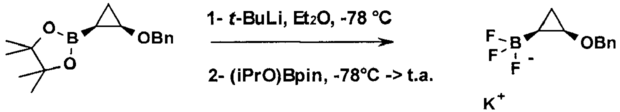

A une solution de dioxaborolane (**exemple 23**, 250 mg, 0.912 mmol) dans le méthanol (5 mL) à température ambiante, sont additionnés du difluorure de potassium (499 g, 6.38 mmol, 7.0 équivalents) puis de l'eau (1.0 mL). Après 3 heures d'agitation vigoureuse à température ambiante, le milieu réactionnel est concentré sous pression réduite. 10 ml d'acétonitrile sont ajoutés au milieu puis évaporés à sec. Cette opération est réalisée plusieurs fois jusqu'à élimination complète de l'eau. Le résidu pâteux est mis en suspension dans l'acétonitrile (10 mL) puis filtré sur coton pour éliminer l'excès de KHF₂. Le filtrat est évaporé sous pression réduite pour conduire à un solide qui est lavé à l'éther diéthylique. Après filtration, 129 mg (56%) de composé sont obtenus sous la forme d'un solide blanc.
**IR** 1356, 1338, 1207, 1098, 1029, 1010, 951, 934, 922, 905, 851, 779, 741, 698, 642 cm⁻¹; **¹H RMN** (400 MHz, D₆-DMSO) *δ* 7.35-7.28 (m, 4H), 7.26-7.21 (m, 1H), 4.59 (d, AB syst, *J* = 11.6 Hz, 1H), 4.37 (d, AB syst, *J* = 11.6 Hz, 1H), 3.16 (m, 1H), 0.28-0.19 (m, 2H),-0.65 (m, 1H); **¹³C RMN** (100 MHz, D₆-DMSO) *δ* 140.7 (s), 128.8 (d, 2C), 128.7 (d, 2C), 127.7 (d), 72.4 (t), 58.6 (d), 8.8 (t), mp = 180 °C.

### Exemple 26 : Couplages de Suzuki-Miyaura sur des cyclopropyltrifluoroborates de potassium trans portant COOEt

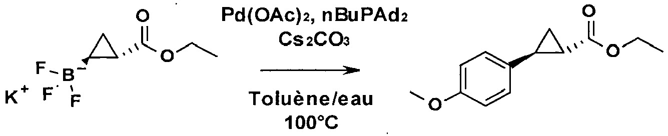

### • (trans)-2-(4-Methoxy-phenyl)-cyclopropyl acetic acid ethyl ester

Le réactif trifluoroborate de potassium (**exemple 1**, 2,5g, 11,4 mmol) est solubilisé dans un mélange toluène (62,5 ml) et eau (6,25 ml). Le 4-bromo-anisole (2,1 ml, 17 mmol), l'acétate de palladium (51 mg, 0,23 mmol) et le n-butyl-di-adamantylphosphine (122 mg, 0,34 mmol) sont additionnés. De l'azote est bullé dans le milieu pendant 10 minutes. Le carbonate de césium (10g, 30.7 mmol) est ajouté et le mélange est chauffé sous azote à 100 °C pendant la nuit. De l'eau est ajouté et le produit attendu est extrait 3 fois avec de l'acétate d'éthyle. Les phases organiques rassemblées sont séchées sur sulfate de magnésium, filtrées et concentrées pour conduire à 5,4g d'un solide marron. Le produit attendu est purifié par chromatographie sur gel de silice (gradient heptane/acétate d'éthyle). Le produit attendu est obtenu sous forme de solide orange (1,25g, 5.7 mmol, rendement 50%). ¹H-RMN (CDCl₃, 300 MHz) δ 7.03 (d, J=8.6Hz, 2H), 6.82 (d, J=8.7Hz, 2H), 4.16 (q, J=7.1Hz, 2H), 3.78 (s, 3H), 2.47 (m, 1H), 1.81 (m, 1H), 1,55 (m, 1H), 1.25 (m, 4H) ; LC/MS >99%, m/z (M+H)⁺ = 221.4.

Tous les autres produits sont obtenus suivant le même mode opératoire.

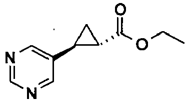

### • (trans)-2-Pyrimidin-5-yl-cyclopropanecarboxylic acid ethyl ester

¹H-RMN (CDCl₃, 300 MHz) δ 9.08 (s, 1H), 8.52 (s, 1H), 4.19 (q, J=7.1Hz, 2H), 2.48 (m, 1H), 1.98 (m, 1H), 1.70 (m, 1H), 1.37 (m, 1H), 1,29 (t, J=7.1Hz, 3H) ; LC/MS >99%, m/z (M+H)⁺ = 193.3.

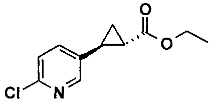

### • (trans)-2-(6-Chloropyridin-3-yl)-cyclopropanecarboxylic acid ethyl ester

¹H-RMN (CDCl₃, 300 MHz) δ 8.21 (d, J=2.5Hz, 1H), 7.32 (dd, J=2.5Hz, J=8.2Hz, 1H), 7.23 (d, J=8.2Hz, 1H), 4.18 (q, J=7.1Hz, 2H), 2.50 (m, 1H), 1.90 (m, 1H), 1.65 (m, 1H), 1.28(m, 4H) ; LC/MS >98%, m/z (M+H)⁺ = 226.3.

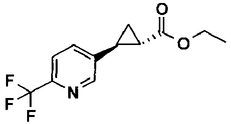

### • (trans)-2-(6-Trifluoromethyl-pyridin-3-yl)-cyclopropanecarboxylic acid ethyl ester

¹H-RMN (CDCl₃, 300 MHz) δ 8.54 (d, J=1.8Hz, 1H), 7.60 (d, J=8.1Hz, 1H), 7.50 (dd, J=1.8Hz, J=8.1Hz, 1H), 4.19 (q, J=7.1Hz, 2H), 2.59 (m, 1H), 1.98 (m, 1H), 1.73 (m, 1H), 1.37(m, 1H), 1.29 (t, J=7.1Hz, 3H) ; LC/MS >99%, m/z (M+H)⁺ = 260.2.

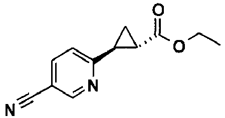

### • (trans)-2-(5-Cyano-pyridin-2-yl)-cyclopropanecarboxylic acid ethyl ester

¹H-RMN (CDCl₃, 300 MHz) δ 8.70 (dd, J=0.8Hz, J=2.2Hz, 1H), 7.82 (dd, J=2.2Hz, J=8.1Hz, 1H), 7.37 (dd, J=0.8Hz, J=8.1Hz, 1H), 4.17 (q, J=7.1Hz, 2H), 2.61 (m, 1H), 2.33 (m, 1H), 1.68 (m, 2H), 1.28 (t, J=7.1Hz, 3H) ; LC/MS >98%, m/z (M+H)⁺ = 217.3.

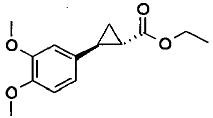

### • (trans)-2-(3,4-Dimethoxyphenyl)-cyclopropanecarboxylic acid ethyl ester

¹H-RMN (CDCl₃, 300 MHz) δ 6.77 (d, J=8.7Hz, 1H), 6.64 (m, 2H), 4.17 (q, J=7.1Hz, 2H), 3.87 (s, 3H), 3.85 (s, 3H), 2.49 (m, 1H), 1.83 (m, 1H), 1.55 (m, 1H), 1.28 (m, 4H) ; LC/MS >99%, m/z (M+H)⁺ = 251.3.

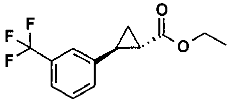

### • (trans)-2-(3-Trifluoromethyl-phenyl)-cyclopropanecarboxylic acid ethyl ester

¹H-RMN (CDCl₃, 300 MHz) δ 7.37 (m, 4H), 4.18 (q, J=7.1Hz, 2H), 2.57 (m, 1H), 1.93 (m, 1H), 1.65 (m, 1H), 1.34 (m, 1H), 1.28 (t, J=7.1Hz, 3H) ; LC/MS >99%, m/z (M+H)⁺ = 259.3.

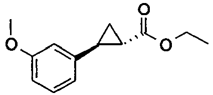

### • (trans)-2-(3-Methoxyphenyl)-cyclopropanecarboxylic acid ethyl ester

¹H-RMN (CDCl₃, 300 MHz) δ 7.19 (dd, J=7.9Hz, 1H), 6.76 (ddd, J=8.2Hz, J=2.6Hz, J=0.9Hz, 1H), 6.69 (m, 1H), 6.64 (dd, J=2.6Hz, 1H), 4.17 (q, J=7.1Hz, 2H), 3.79 (s, 3H), 2.49 (m, 1H), 1.90 (m, 1H), 1.59 (m, 1H), 1.29 (m, 4H) ; LC/MS >99%, m/z (M+H)⁺ = 291.2.

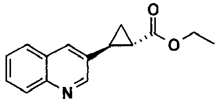

### • (trans)-2-Quinolin-3-yl-cyclopropanecarboxylic acid ethyl ester

¹H-RMN (CDCl₃, 300 MHz) δ 8.75 (d, J=2.1Hz, 1H), 8.06 (d, J=8.4Hz, 1H), 7.77 (d, J=2.1Hz, 2H), 7.73 (d, J=8.4 Hz, 1H), 7.65 (ddd, J=7.7Hz, J=7.7Hz, J=1.4Hz, 1H), 7.51 (ddd, J=7.7Hz, J=7.7Hz, J=1.4Hz, 1H), 4.19 (t, J=7.1Hz, 2H), 2.70 (m, 1H), 2.03 (m, 1H), 1.72 (m, 1H), 1.45 (m, 1H), 1.30 (t, J=7.1Hz, 3H) ; LC/MS >99%, m/z (M+H)⁺ = 242.3.

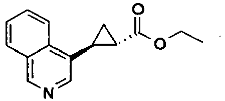

### • (trans)-2-Isoquinolin-4-yl-cyclopropanecarboxylic acid ethyl ester

¹H-RMN (CDCl₃, 300 MHz) δ 9.17 (bs, 1H), 8.33 (bs, 1H), 8.16 (d, J=8.4Hz, 1H), 8.0 (m, 1H), 7.79 (ddd, J=7.7Hz, J=7.7Hz, J=1.4Hz, 1H), 7.66 (ddd, J=7.7Hz, J=7.7Hz, J=1.4Hz, 1H), 4.27 (q, J=7.1Hz, 2H), 2.91 (m, 1H), 197 (m, 1H), 1.73 (m, 1H), 1.48 (m, 1H), 1.34 (t, J=7.1Hz, 3H) ; LC/MS >99%, m/z (M+H)⁺ = 242.3.

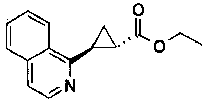

### • (trans)-2-Isoquinolin-1-yl-cyclopropanecarboxylic acid ethyl ester

¹H-RMN (CDCl₃, 300 MHz) δ 8.75 (m, 2H), 7.82 (m, 1H), 7.66 (m, 2H), 7.49 (d, J=5.7Hz, 1H), 4.21 (q, J=7.2Hz, 2H), 3.33 (m, 1H), 2.44 (m, 1H), 1.82 (m, 1H), 1.73 (m, 1H), 1.30 (t, J=7.2Hz, 3H) ; LC/MS >99%, m/z (M+H)⁺ = 242.3.

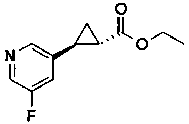

### • (trans)-2-(5-Fluoropyridin-3-yl)-cyclopropanecarboxylic acid ethyl ester

¹H-RMN (CDCl₃, 300 MHz) δ 8.31 (d, J=2.6Hz, 1H), 8.27 (bt, 1H), 7.05 (ddd, J=9.4Hz, J=2.2Hz, J=2.2Hz, 1H), 4.17 (q, J=7.1Hz, 2H), 2.52 (m, 1H), 1.93 (m, 1H), 1.67 (m, 1H), 1.32 (m, 1H), 1.28 (t, J=1.7Hz, 3H) ; LC/MS >99%, m/z (M+H)⁺ = 210.3.

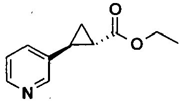

### • (trans)-2-Pyridin-3-yl-cyclopropanecarboxylic acid ethyl ester

¹H-RMN (CDCl₃, 300 MHz) δ 8.45 (m, 2H), 7.37 (ddd, J=7.9Hz, J=3.4Hz, J=3.4Hz, 1H), 7.23 (d, J=7.9Hz, 1H), 4.18 (q, J=7.1Hz, 2H), 2.52 (m, 1H), 1.93 (m, 1H), 1.65 (m, 1H), 1.32 (m, 1H), 1.29 (t, J=7.1Hz, 3H) ; LC/MS >99%, m/z (M+H)⁺ = 192.2.

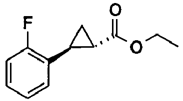

### • (trans)-2-(2-Fluorophenyl)-cyclopropanecarboxylic acid ethyl ester

¹H-RMN (CDCl₃, 300 MHz) δ 7.18 (m, 1H), 7.00 (m, 3H), 4.18 (q, J=7.0Hz, 2H), 2.66 (m, 1H), 1.94 (m, 1H), 1.60 (m, 1H), 1.35 (m, 1H), 1.29 (t, J=7.0 Hz, 3H) ; LC/MS >99%, m/z (M+H)⁺ = 209.3.

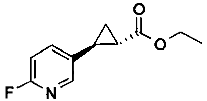

### • (trans)-2-(6-Fluoropyridin-3-yl)-cyclopropanecarboxylic acid ethyl ester

¹H-RMN (CDCl₃, 300 MHz) δ 8.01 (m, 1H), 7.44 (ddd, J=8.9Hz, J=2.5Hz, J=2.5Hz, 1H), 6.83 (dd, J=8.9Hz, J=2.5Hz, 1H), 4.16 (q, J=7.1Hz, 2H), 2.45 (m, 1H), 1.86 (m, 1H), 1.61 (m, 1H), 1.26 (m, 4H) ; LC/MS >99%, m/z (M+H)⁺ = 210.3.

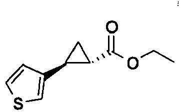

### • (trans)-2-Thiophen-3-yl-cyclopropanecarboxylic acid ethyl ester

¹H-RMN (CDCl₃, 300 MHz) δ 7.26 (dd, J=5.0Hz, J=3.0Hz, 1H), 6.97 (m, 1H), 6.84 (dd, J=5.0Hz, J=3.0Hz, 1H), 4.17 (q, J=7.1Hz, 2H), 2.55 (m, 1H), 1.87 (m, 1H), 1.57 (m, 1H), 1.29 (m, 4H) ; LC/MS >99%, m/z (M+H)⁺ = 197.1.

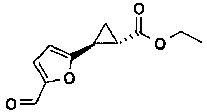

### • (trans)-2-(5-Formyl-furan-2-yl)-cyclopropanecarboxylic acid ethyl ester

¹H-RMN (CDCl₃, 300 MHz) δ 9.49 (s, 1H), 7.16 (d, J=3.6Hz, 1H), 6.32 (d, J=3.6Hz, 1H), 4.17 (q, J=7.2Hz, 2H), 2.58 (m, 1H), 2.18 (m, 1H), 1.59 (m, 2H), 1.28 (t, J=7.2Hz, 3H) ; LC/MS >99%, m/z (M+H)⁺ = 209.2.

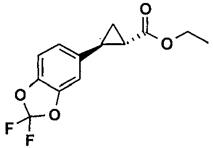

### • (trans)-2-(2,2-Difluoro-benzo[1,3]dioxol-5-yl)-cyclopropanecarboxylic acid ethyl ester

¹H-RMN (CDCl₃, 300 MHz) δ 6.95 (d, J=8.3Hz, 1H), 6.80 (dd, J=8.3Hz, J=1.7Hz, 1H), 6.84 (d, J=1.7Hz, 1H), 4.17 (q, J=7.1Hz, 2H), 2.51 (m, 1H), 1.84 (m, 1H), 1.60 (m, 1H), 1.27 (m, 4H) ; LC/MS >99%, m/z (M+H)⁺ = 271.2.

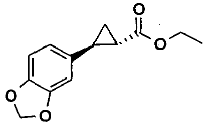

### • (trans)-2-Benzo[1,3]dioxol-5-yl-cyclopropanecarboxylic acid ethyl ester

¹H-RMN (CDCl₃, 300 MHz) δ 6.71 (d, J=7.98Hz, 1H), 6.60 (ddd, J=7.9Hz, J=1.8Hz, J=0.42Hz, 1H), 6.56 (d, J=1.8Hz, 1H), 4.16 (q, J=7.1Hz, 2H), 2.45 (m, 1H), 1.80 (m, 1H), 1.53 (m, 1H), 1.24 (m,4H) ; LC/MS >99%, m/z

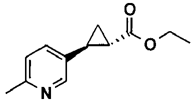

### • (trans)-2-(6-Methylpyridin-3-yl)-cyclopropanecarboxylic acid ethyl ester

¹H-RMN (CDCl₃, 300MHz) δ 8.34 (d, J=2.3Hz, 1H), 7.25 (dd, J=8.2Hz, J=2.3Hz, 1H), 7.07 (d, J=8.2Hz, 1H), 4.17 (q, J=7.1Hz, 2H), 2.50 (m, 1H), 1.89 (m, 1H), 1.62 (m, 1H), 1.30 (m, 4H); LC/MS >99%, m/z (M+H)⁺ = 206.3

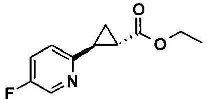

### • (trans)-2-(5-Fluoropyridin-2-yl)-cyclopropanecarboxylic acid ethyl ester

¹H-RMN (CDCl₃, 300MHz) δ 8.30 (d, J=2.7Hz, 1H), 7.27 (m, 2H), 4.17 (q, J=7.1Hz, 2H), 2.58 (m, 1H), 2.2 (m, 1H), 1.60 (m, 1H), 1.28 (m, 4H) ; LC/MS >99%, m/z (M+H)⁺= 210.3

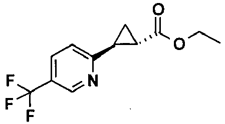

### • (trans)-2-(5-Trifluoromethyl-pyridin-2-yl)-cyclopropanecarboxylic acid ethyl ester

¹H-RMN (CDCl₃, 300MHz) δ 8.68 (s, 1H), 7.78 (dd, J=8.2Hz, J=1.8Hz, 1H), 7.35 (d, J=8.2Hz, 1H), 4.16 (q, J=7.2Hz, 2H), 2.62 (m, 1H), 2.30 (m, 1H), 1.65 (m, 2H), 1.27 (t, J=7.2Hz, 3H) ; LC/MS >99%, m/z (M+H)⁺= 260.3

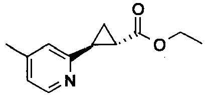

### • (trans)-2-(4-Methylpyridin-2-yl)-cyclopropanecarboxylic acid ethyl ester

¹H-RMN (CDCl₃, 300MHz) δ 8.31 (d, J=5.0Hz, 1H), 7.07 (d, J=0.7Hz, 1H), 6.93 (dd, J=5.0Hz, J=0.7Hz, 1H), 4.18 (q, J=7.2Hz, 2H), 2.55 (m, 1H), 2.23 (m, 1H), 1.6 (m, 2H), 1.29 (t, 7.2Hz, 3H) ; LC/MS >99%, m/z (M+H)⁺= 206.3

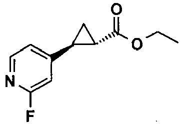

### • (trans)-2-(2-Fluoropyridin-4-yl)-cyclopropanecarboxylic acid ethyl ester

¹H-RMN (CDCl₃, 300MHz) δ 8.11 (d, J=5.3Hz, 1H), 6.89 (dt, J=5.3Hz, J=1.6Hz, 1H), 6.64 (s, 1H), 4.19 (q, J=7.1Hz, 2H), 2.52 (m, 1H), 2.02 (m, 1H), 1.72 (m, 1H), 1.39 (m, 1H), 1.35 (t, J=7.1Hz, 3H) ; LC/MS >99%, m/z (M+H)⁺=210.3

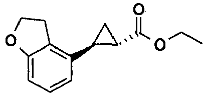

### • (trans)-2-(2,3-Dihydro-benzofuran-4-yl)-cyclopropanecarboxylic acid ethyl ester

¹H-RMN (CDCl₃, 300MHz) δ 7.04 (dd, J=7.8Hz, 1H), 6.65 (d, J=7.1Hz, 1H), 6.40 (d, J=7.8Hz, 1H), 4.60 (t, J=8.7Hz, 2H), 4.18 (q, 7.1Hz, 2H), 3.24 (d, J=7.8Hz, 2H), 2.42 (m, 1H), 1.90 (m, 1H), 1.58 (m, 1H), 1.30 (m, 4H).

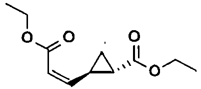

### • Ethyl-(trans)-2-[(Z)-3-éthoxy-3-oxo-prop-1-enyl]cyclopropanecarboxylate

¹H-RMN (CDCl₃, 300MHz) δ 5.77 (dm, J=11.4Hz, 1H), 5.44 (dd, J=11.4Hz, J=10.8Hz, 1H), 4.19 (q, J=7.0Hz, 2H), 4.13 (q, J=7.0Hz, 2H), 3.40 (m, 1H), 1.74 (m, 1H), 1.59 (m, 1H), 1.29 (t, J=7.0Hz, 3H), 1.26 (t, J=7.0Hz, 3H), 1.03 (m, 1H).

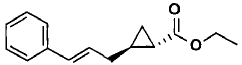

### • Ethyl-(trans)-2-[(E)-cinnamyl]cyclopropanecarboxylate

¹H-RMN (CDCl₃, 300MHz) δ 7.4-7.12 (m, 5H), 6.40 (d, J=15.9Hz, 1H), 6.17 (dt, J=15.9Hz, J=6.9Hz, 1H), 4.09 (q, J=7.0Hz, 2H), 2.21 (m, 2H), 1.43 (m, 2H), 1.23 (t, J=7.0Hz, 3H), 1.20 (m, 1H), 0.77 (m, 1H).

### Exemple 27 : Couplages de Suzuki-Miyaura sur des cyclopropyltrifluoroborates de potassium trans portant -CH₂-NHZ

Les produits suivants sont obtenus selon le mode opératoire présenté dans l'exemple 26, à partir du réactif trifluoroborate de potassium décrit dans l'exemple 9.

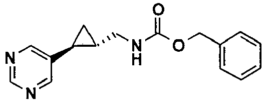

### • ((trans)-2-Pyrimidin-5-yl-cyclopropylmethyl)-carbamic acid benzyl ester

¹H-RMN (CDCl₃, 300 MHz) δ 9.04 (s, 1H); 8.46 (s, 2H); 7.35 (m, 5H); 5.13 (s, 2H); 5.02 (bs, 1H); 3.29 (m, 2H); 1.87 (m, 1H); 1.42 (m, 1H); 1.08 (m, 2H).

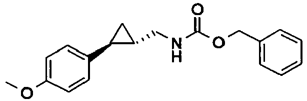

### • [(trans)-2-(4-Methoxy-phenyl)-cyclopropylmethyl]-carbamic acid benzyl ester

¹H-RMN (CDCl₃, 300 MHz) δ 7.34(m, 5H); 6.97 (d, J=8.5Hz, 2H); 6.80 (d, J=8.5Hz, 2H); 5.11 (s, 2H); 4.93 (bs, 1H); 3.78 (s 3H); 3.22 (m, 2H); 1.77 (m, 1H); 1.24 (m, 1H); 0.87 (m,2H).

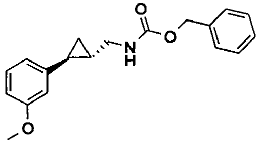

### • (trans)-2-(3-Methoxy-phenyl)-cyclopropylmethyl]-carbamic acid benzyl ester

¹H-RMN (CDCl₃, 300 MHz) δ 7.37 (m, 5H); 7.18 (dd, J=7.9Hz, J=7.9Hz, 1H); 6.72 (dd, J=2.5Hz, J=8.2Hz, 1H); 6.65 (d, J=8.2 Hz, 1H); 6.60 (m, 1H); 5.12 (s, 2H ; 4.92 (s, 1H) ; 3.80 (s, 3H); 3.25 (m, 2H ; 1.80 (m, 1H) ; 1.34 (m, 1H ; 0.95 (m, 2H).

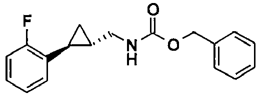

### • [(trans)-2-(2-Fluoro-phenyl)-cyclopropylmethyl]-carbamic acid benzyl ester

¹H-RMN (CDCl₃, 300 MHz) δ 7.36 (m, 5H); 7.16 (m, 1H); 7.03 (m, 2H); 6.92 (dd, J=7.0Hz, J=7.0Hz, 1H); 5.13 (s, 2H); 5.01 (bs, 1H); 3.43 (m, 1H); 3.14 (m, 1H); 1.98 (m, 1H); 1.31 (m, 1H); 0.98 (m, 2H).

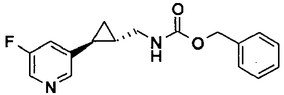

### • [(trans)-2-(5-Fluoro-pyridin-3-yl)-cyclopropylmethyl]-carbamic acid benzyl ester*

¹H-RMN (CDCl₃, 300 MHz) δ 8.25 (d, J=2.6Hz, 1H); 8.21 (s, 1H); 7.34 (m, 5H); 6.96 (d, J=9.4Hz, 1H); 5.11 (s, 2H); 5.00 (bs, 1H); 3.25 (m, 2H); 1.87 (m, 1H); 1.35 (m, 1H); 1.00 (m, 2H).

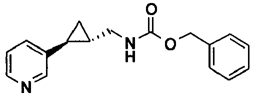

### • ((trans)-2-Pyridin-3-yl-cyclopropylmethyl)-carbamic acid benzyl ester

¹H-RMN (CDCl₃, 300 MHz) δ 8.40 (m, 2H); 7.34 (m, 6H); 7.21 (m, 1H); 5.10 (s, 2H); 4.96 (bs, 1H); 3.26 (m, 2H); 1.85 (m, 1H); 1.34 (m, 1H); 1.00 (m, 2H).

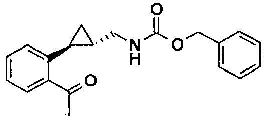

### • [(trans)-2-(2-Acetyl-phenyl)-cyclopropylmethyl]-carbamic acid benzyl ester

¹H-RMN (CDCl₃, 300 MHz) δ 7.68 (dd, J=1.2Hz, J=7.6Hz, 1H); 7.34 (m, 7H); 7.08 (d, J=7.6Hz, 1H); 5.13 (s, 2H); 3.79 (m, 1H); 2.76 (m, 1H); 2.63 (s, 3H); 2.26 (m, 1H); 1.10 (m, 2H); 0.85 (m, 1H).

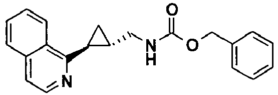

### • ((trans)-2-Isoquinolin-1-yl-cyclopropylmethyl)-carbamic acid benzyl ester

¹H-RMN (CDCl₃, 300 MHz) δ 8.39 (d, J=8.3Hz, 1H); 8.35 (d, J=5.7Hz, 1H); 7.82 (d, J=7.5Hz, 1H); 7.65 (m, 2H); 7.46 (dd, J=0.6Hz; J=5.7Hz, 1H); 7.34 (m, 5H); 5.13 (s, 2H); 5.01 (bs, 1H); 3.43 (m, 2H); 2.75 (m, 1H); 1.84 (m, 1H); 1.55 (m, 1H); 1.12 (m, 1H).

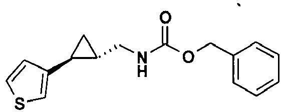

### • ((trans)-2-Thiophen-3-yl-cyclopropylmethyl)-carbamic acid benzyl ester

¹H-RMN (CDCl₃, 300 MHz) δ 7.34 (m, 5H); 7.21 (dd, J=3.0Hz, J=4.9Hz, 1H); 6.85 (s, 1H); 6.78 (d, J=4.9Hz, 1H); 5.11 (s, 2H); 4.90 (bs, 1H); 3.22 (m, 2H); 1.85 (m, 1H); 1.28 (m, 1H); 0.87 (m, 2H).

### Exemple 28 : Couplages de Suzuki-Miyaura sur des cyclopropyltrifluoroborates de potassium trans portant -OBn

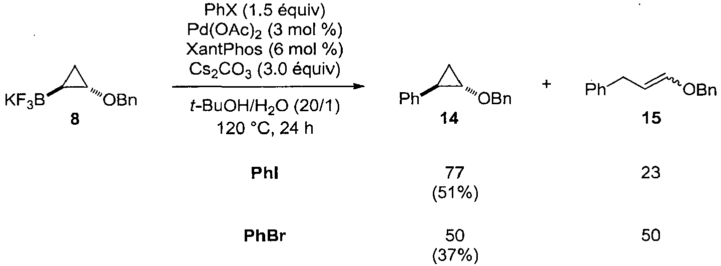

### • Couplage de Suzuki-Miyaura entre le composé 8 et le iodobenzène :

Dans un tube scellé, sous atmosphère inerte, sont successivement additionnés le diacétate de palladium (1.3 mg, 5.9 µmol, 3 mol %), le XantPhos (6.8 mg, 12 µmol, 6 mol %), le carbonate de césium (192 mg, 0.590 mmol, 3.0 équivalents), le cyclopropyltrifluoroborate de potassium **8** (50.0 mg, 0.197 mmol), le *tert*-butanol (1.0 mL), l'eau (50 µL) et enfin le iodobenzène (33 µL, 0.30 mmol, 1.5 équivalents). Le tube est ensuite fermé (bouchon Teflon) puis immergé dans un bain d'huile préchauffé à 120°C. Après 24 heures d'agitation à cette température, le milieu réactionnel est refroidi à température ambiante puis filtré sur pad de célite (rinçage AcOEt) et évaporé à sec sous pression réduite. Une purification par chromatographie sur gel de silice (Eluant : éther de pétrole / AcOEt: 95/5) conduit à un mélange de composé **14** et d'éther d'énol **15** dans un ratio de 77/23. La séparation de ce mélange peut être obtenu par CCM préparative sur plaque de silice (Eluant : éther de pétrole / AcOEt : 95/5) pour conduire à 22.5 mg (51%) de composé **14** sous la forme d'une huile incolore.

### • Couplage de Suzuki-Miyaura entre le composé 8 et le bromobenzène :

Dans un tube scellé, sous atmosphère inerte, sont successivement additionnés le diacétate de palladium (1.3 mg, 5.9 µmol, 3 mol %), le XantPhos (6.8 mg, 12 µmol, 6 mol %), le carbonate de césium (192 mg, 0.590 mmol, 3.0 équivalents), le cyclopropyltrifluoroborate de potassium **8** (50.0 mg, 0.197 mmol), le *tert*-butanol (1.0 mL), l'eau (50 µL) et enfin le bromobenzène (31.1 µL, 0.295 mmol, 1.5 équivalents). Le tube est ensuite fermé (bouchon Teflon) puis immergé dans un bain d'huile préchauffé à 120°C. Après 24 heures d'agitation à cette température, le milieu réactionnel est refroidi à température ambiante puis filtré sur pad de célite (rinçage AcOEt) et évaporé à sec sous pression réduite. Une purification par chromatographie sur gel de silice (Eluant : éther de pétrole / AcOEt: 95/5) conduit à un mélange de cyclopropène **14** et d'éther d'énol **15** dans un ratio 50/50. La séparation de ce mélange peut être obtenu par CCM préparative sur plaque de silice (Eluant : éther de pétrole / AcOEt : 95/5) pour conduire à 16.4 mg (37%) de composé **14** sous la forme d'une huile incolore.

### [(1R*,2S*)-2-(Benzyloxy)cyclopropyl]benzene (14).

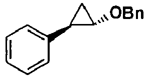

**IR** 1604, 1496, 1454, 1366, 1260, 1208, 1146, 1092, 1071, 1024, 910, 870, 735, 695, 613 cm⁻¹; **¹H RMN** (400 MHz, CDCl₃) *δ* 7.35-7.22 (m, 7H), 7.18-7.13 (m, 1H), 7.01-6.98 (m, 2H), 4.63 (d, AB syst, *J* = 11.7 Hz, 1H), 4.60 (d, AB syst, *J* = 11.7 Hz, 1H), 3.44 (ddd, *J* = 6.5 Hz, *J* = 3.6 Hz et *J* = 2.6 Hz, 1H), 2.15 (ddd, *J* = 10.2 Hz, *J* = 6.4 Hz et *J* = 2.6 Hz, 1H), 1.34 (ddd, *J* = 10.2 Hz, *J* = 6.1 Hz et *J* = 3.6 Hz, 1H), 1.05 (ddd, apparent q, *J* = 6.4 Hz, 1 H); **¹³C RMN** (100 MHz, CDCl₃) *δ* 141.1 (s), 137.8 (s), 128.4 (d, 2C), 128.3 (d, 2C), 128.0 (d, 2C), 127.8 (d), 125.9 (d, 2C), 125.7 (d), 73.0 (t), 61.7 (d), 23.9 (d), 16.0 (t); **EI-MS** *m*/*z* (intensité relative) 224 (M^{+•}, 0.1), 181 (3), 180 (21), 133 (M-Bn⁺, 17), 115 (3), 106 (9), 105 (100), 104 (7), 103 (10), 92 (10), 91 (87), 89 (3), 79 (15), 78 (6), 77 (15), 65 (17), 63 (4), 51 (7). **HRMS** calculé pour C₁₆H₁₆ONa (M+Na⁺): 247.10934. Trouvé: 247.10901.

### [3-(Benzyloxy)prop-2-en-1-yl]benzene (15).

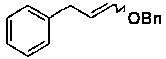

**¹H RMN** (400 MHz, CDCl₃) (Z)-isomer *δ* 7.39-7.14 (m, 10H), 6.14 (dt, *J* = 6.2 Hz et *J* = 1.2 Hz, 1H), 4.84 (s, 2H), 4.62 (td, *J* = 7.5 Hz et *J* = 6.2 Hz, 1H, H₂), 3.48 (br d, *J* = 7.5 Hz, 2H, H₇); (*E*)-isomer *δ* 7.39-7.14 (m, 10H, H_{Ar}), 6.44 (dt, *J* = 12.6 Hz et *J* = 1.2 Hz, 1H, H₃), 5.04 (dt, *J* = 12.6 Hz et *J* = 7.4 Hz, 1 H, H₂), 4.74 (s, 2H, H₄), 3.27 (br d, *J* = 7.1 Hz, 2H, H₇); **¹³C RMN** (100 MHz, CDCl₃) (*Z*)-isomer *δ* 145.1 (d, C₃), 141.6 (s, C₅ or C₉), 137.6 (s, C₉ or C₅), 128.5 (d), 128.4 (d) et 128.3 (d) (6 C_{Ar}), 127.6 (d, 2C, C_{Ar}), 125.7 (d, 2C, C_{Ar}), 106.4 (d, C₂), 73.7 (t, C₄), 30.3 (t, C₇); (*E*)-isomer *δ* 146.9 (d, C₃), 141.5 (s, C₅ or C₉), 137.1 (s, C₉ or C₅), 128.5 (d), 128.4 (d), 128.3 (d) (6 C_{Ar}), 127.9 (d, 2C, C_{Ar}), 125.9 (d, 2C, C_{Ar}), 103.8 (d, C₂), 71.1 (t, C₄), 34.1 (t, C₇).

### Exemple 29 : Couplages de Suzuki-Miyaura sur des cyclopropyltrifluoroborates de potassium cis portant COOEt

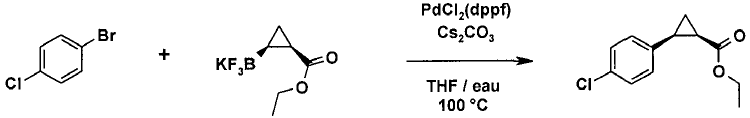

### • (cis)-2-(4-chlorophenyl)cyclopropyl acetic acid ethyl ester

A une solution de trifluoroborate de potassium *cis* (5,0 g, 22,7 mmol) dans un mélange THF (20 ml) et eau (20 ml) sont additionnés successivement le 4-bromochlorobenzène (8,7 g, 45,5 mmol), le palladium-diphénylphosphinoferrocène (832 mg, 1,14 mmol) et le carbonate de césium (22,2 g, 68,2 mmol). Le milieu réactionnel purgé à l'azote est chauffé à 100 °C pendant 48 heures. L'insoluble est filtré sur Clarcel®, puis lavé par de l'eau et de l'acétate d'éthyle. Les phases sont séparées et la phase aqueuse est extraite 3 fois par de l'acétate d'éthyle. Les phases organiques sont séchées sur sulfate de magnésium, filtrées, concentrées à sec pour conduire à 5,55 g d'une huile marron. Une purification par chromatographie sur gel de silice (Eluant : gradient Heptane / AcOEt) conduit au composé attendu sous la forme d'une huile marron.
**¹H-RMN (CDCl₃, 300 MHz)** δ 7.23 (m, 4H), 3.92 (q, J=7.5Hz, 2H), 2.54 (q, J=9.0Hz, 1H), 2.10 (m, 1H), 1.69 (m, 1H), 1.33 (m, 1H), 1.04 (t, J=7.5Hz, 3H)

Tous les autres produits sont obtenus suivant le même mode opératoire.

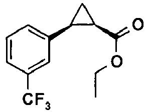

### • (cis)-2-(3-trifluoromethylphenyl)cyclopropanecarboxylic acid ethyl ester

**¹H-RMN (CDCl₃, 300 MHz)** δ 7.38 (m, 4H), 3.81 (qd, J₁=7.3Hz, J₂=1.1Hz, 2H), 2.53 (q, J=8.4Hz, 1H), 2.06 (m, 1H), 1.66 (m, 1H), 1.32 (m, 1H), 0.91 (t, J=7.3Hz, 3H) ; LC/MS >90%, m/z (M+H)⁺ = 259.3

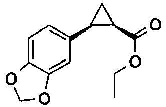

### • (cis)-2-(1,3-benzodioxol-5-yl)cyclopropanecarboxylic acid ethyl ester

**¹H-RMN (acetone-*d6*, 300 MHz)** δ 6.65 (m, 3H), 5.84 (s, 2H), 3.78 (q, J=6.8Hz, 2H), 2.45 (q, J=8.2Hz, 1H), 1.45 (m, 1H), 1.21 (m, 1H), 0.92 (t, J=6.8Hz, 3H) ; LC/MS >98%, m/z (M+H)⁺ = 235.3

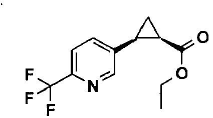

### • (cis)-2-(6-Trifluoromethyl-pyridin-3-yl)cyclopropanecarboxylic acid ethyl ester

**¹H-RMN (acetone-*d6*, 300 MHz)** δ 8.54 (s, 1H), 7.8 (dm, J=9.4Hz, 1H), 7.62 (d, J=9.4Hz, 1H), 3.75 (m, 2H), 2.64 (q, J=8.6Hz, 1H), 2.14 (m, 1H), 1.62 (m, 1H), 1.41 (m, 1H), 0.86 (t, J=7.7Hz, 3H) ; LC/MS >96%, m/z (M+H)⁺ = 260.3

### Exemple 30: Couplages de Suzuki-Miyaura sur des cyclopropyltrifluoroborates de potassium cis portant -OBn

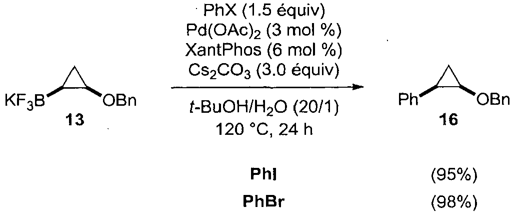

### • Couplage de Suzuki-Miyaura entre le compose 13 et le iodobenzène :

Dans un tube scellé, sous atmosphère inerte, sont successivement additionnés le diacétate de palladium (1.3 mg, 5.9 µmol, 3 mol %), le XantPhos (6.8 mg, 12 µmol, 6 mol %), le carbonate de césium (192 mg, 0.590 mmol, 3.0 équivalents), le cyclopropyltrifluoroborate de potassium **13** (50.0 mg, 0.197 mmol), le *tert*-Butanol (1.0 mL), l'eau (50 µL) et enfin le iodobenzène (33 µL, 0.30 mmol, 1.5 équivalents). Le tube est ensuite fermé (bouchon Teflon) puis immergé dans un bain d'huile préchauffé à 120°C. Après 24 heures d'agitation à cette température, le milieu réactionnel est refroidi à température ambiante puis filtré sur pad de célite (rinçage AcOEt) et évaporé à sec sous pression réduite. Une purification par chromatographie sur gel de silice (Eluant : éther de pétrole / AcOEt: 97/3 puis 96/4) conduit à 42.0 mg (95%) de composé **16** sous la forme d'une huile incolore.

### • Couplage de Suzuki-Miyaura entre le compose 13 et le bromobenzène:

Dans un tube scellé, sous atmosphère inerte, sont successivement additionnés le diacétate de palladium (1.3 mg, 5.9 µmol, 3 mol %), le XantPhos (6.8 mg, 12 µmol, 6 mol %), le carbonate de césium (192 mg, 0.590 mmol, 3.0 équivalents), le cyclopropyltrifluoroborate de potassium **13** (50.0 mg, 0.197 mmol), le *tert*-Butanol (1.0 mL), l'eau (50 µL) et enfin le bromobenzène (31.1 µL, 0.295 mmol, 1.5 équivalents). Le tube est ensuite fermé (bouchon Teflon) puis immergé dans un bain d'huile préchauffé à 120°C. Après 24 heures d'agitation à cette température, le milieu réactionnel est refroidi à température ambiante puis filtré sur pad de célite (rinçage AcOEt) et évaporé à sec sous pression réduite. Une purification par chromatographie sur gel de silice (Eluant : éther de pétrole / AcOEt: 97/3 puis 96/4) conduit à 43.3 mg (98%) de composé **16** sous la forme d'une huile incolore.

### [(1R*,2R*)-2-(Benzyloxy)cyclopropyl]benzene

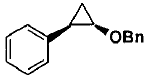

**IR** 1603, 1497, 1454, 1345, 1222, 1182, 1083, 1047, 1027, 938, 766, 733, 694, 642 cm⁻¹; **¹H RMN** (400 MHz, CDCl₃) *δ* 7.32-7.18 (m, 8H, H_{Ar}), 7.10-7.07 (m, 2H, H₁₀), 4.27 (d, AB syst, *J* = 11.2 Hz, 1H, H₄), 4.16 (d, AB syst, *J* = 11.2 Hz, 1H, H_{4'}), 3.59 (ddd, apparent td, *J* = 6.4 Hz et *J* = 3.9 Hz, 1H, H₃), 2.02 (ddd, *J* = 9.5 Hz, *J* = 7.2 Hz et *J* = 6.3 Hz, 1H, H₂), 1.23-1.14 (m, 2H, H₁); **¹³C RMN** (100 MHz, CDCl₃) *δ* 137.7 (s, C₅ or C₉), 137.5 (s, C₉ or C₅), 128.21 (d, 2C), 128.16 (d, 2C), 127.93 (d, 2C), 127.88 (d, 2C) (8 C_{Ar}), 127.6 (d, C₈), 125.6 (d, C₁₀), 72.7 (t, C₄), 58.4 (d, C₃), 22.8 (d, C₂), 12.9 (t, C₁); **EI-MS** *m*/*z* (intensité relative) 224 (M^{+•}, 0.1), 181 (4), 180 (23), 133 (M-Bn⁺, 16), 115 (3), 106 (9), 105 (100), 104 (7), 103 (11), 92 (10), 91 (85), 89 (3), 79 (15), 78 (6), 77 (16), 65 (17), 63 (4), 51 (7). **HRMS** calculé pour C₁₆H₁₆ONa (M+Na⁺): 247.10934. Trouvé: 247.10930.

### Exemple 31 : synthèse du (+/-)-tasimeltéon

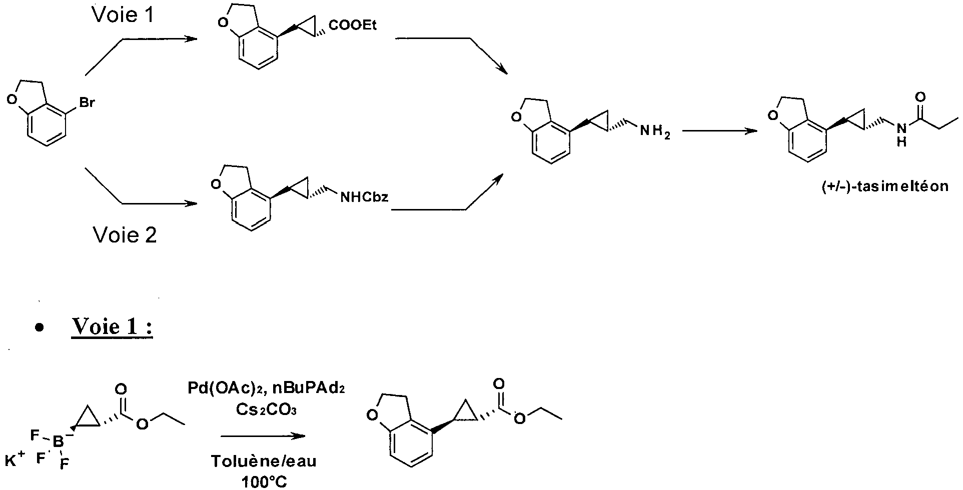

A une solution de trifluoroborate de potassium (**exemple 1**, 2,21g, 10,05 mmol) dans un mélange toluène (55 ml) et eau (5,5 ml) sont additionnés successivement le 4-bromo-2,3-dihydrobenzofurane (1 g, 5,03 mmol), le diacétate de palladium (23 mg, 0,10 mmol) et le *n-*butyldi-adamantylphosphine (54 mg, 0,15 mmol). Le milieu est purgé à l'azote pendant 10 minutes. Du carbonate de césium (4,4 g, 13,56 mmol) est ajouté et le mélange réactionnel est chauffé à 100 °C pendant une nuit. Après hydrolyse à l'eau, la phase aqueuse est extraite 3 fois par de l'acétate d'éthyle. Les phases organiques rassemblées sont séchées sur sulfate de magnésium, filtrées et concentrées à sec pour conduire à 1,4g d'une huile marron. Une purification par chromatographie sur gel de silice (gradient heptane/acétate d'éthyle) conduit à 910 mg (78 %) de composé attendu sous la forme d'une huile jaune.
**¹H-RMN (CDCl₃, 300 MHz)** δ 7.04 (dd, J=7.8Hz, 1H), 6.65 (d, J=8.1Hz, 1H), 6.40 (d, J=7.8Hz, 1 H), 4.60 (t, J=8.7Hz, 2H), 4.18 (q, 7.1Hz, 2H), 3.24 (t, J=8.7Hz, 2H), 2.42 (m, 1 H), 1.90 (m, 1H), 1.58 (m, 1H), 1.30 (m, 4H).
**¹³C-RMN (CDCl₃, 300 MHz) :** 174.0, 160.2, 137.1, 128.7, 127.0, 116.5, 107.9, 71.5, 61.2, 29.0, 24.4, 23.3, 16.6, 14.7
LCUV > 99 %.

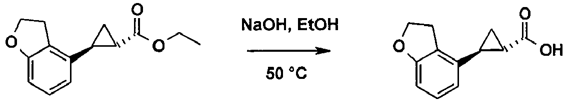

Une solution d'ester éthylique (800 mg, 3,4 mmol) dans un mélange soude aqueuse 2 N (8,6 ml, 17,3 mmol) et éthanol (4 ml est chauffé à 50 °C pendant 2 h. De l'acide chlorhydrique 1 N est ensuite ajouté puis la phase aqueuse est extraite 3 fois par de l'acétate d'éthyle. Les phases organiques rassemblées sont lavées par de l'eau puis de la saumure, séchées sur sulfate de magnésium, filtrées et concentrées à sec pour conduire à 680 mg d'un solide beige (98 %). **mp** = 146 °C.
**¹H-RMN (CDCl₃, 300 MHz)** δ 7.04 (dd, J=7.8Hz, 1H), 6.65 (d, J=7.8Hz, 1H), 6.41 (d, J=7.8Hz, 1H), 4.60 (t, J=8.7Hz, 2H), 3.25 (t, J=8.7Hz, 2H), 2.49 (m, 1H), 1.89 (m, 1H), 1.62 (m, 1H), 1.40 (m, 1H).
**¹³C-RMN (CDCl₃, 300 MHz)** 180.1, 160.3, 136.5, 128.8, 127.1, 116.8, 108.2, 77.5, 29.0, 25.3,23.0, 17.0
**LC/MS** > 99 %, m/z [M+H]⁺ = 205.2.

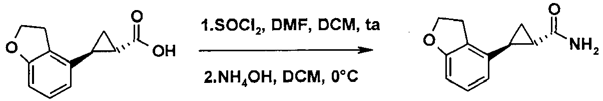

A une solution d'acide carboxylique (680 mg, 3,3 mmol) dans 6,8 ml de dichlorométhane sont additionnés le diméthylformamide (100 µl) et le chlorure de thionyle (500 µl, 6,8 mmol) à température ambiante. Après 2 heures, le mélange réactionnel est concentré à sec, puis repris par 3,4 ml de dichlorométhane. Le milieu est refroidi à 0 °C dans un bain glace / eau, puis de l'ammoniaque (13,6 ml) est additionné lentement. La suspension observée est agitée à température ambiante pendant une nuit. De l'éther isopropylique est additionné, le solide est filtré puis lavé deux fois par de l'éther isopropylique pour conduire à 565 mg (83%) de composé attendu sous la forme d'un solide blanc.
**mp** = 194 °C.
**¹H-RMN (CDCl₃, 300 MHz)** δ 7.05 (dd, J=7.8Hz, 1H), 6.68 (d, J=7.8Hz, 1H), 6.38 (d, J=7.8Hz, 1H), 5.72 (s, 1H), 5.58 (s,1H), 4.60 (t, J=8.7Hz, 2H), 3.26 (m, 2H), 2.44 (m, 1H), 1.83 (m, 1H), 1.63 (m, 1H), 1.30 (m, 1H).
**¹³C-RMN (CDCl₃, 300 MHz)** 174.9, 160.2, 137.5, 128.7, 127.0, 115.9, 107.9, 77.6, 29.0, 25.0, 23.8, 16.2
**LC/MS** > 99 %, m/z [M+H]⁺ = 204.2.

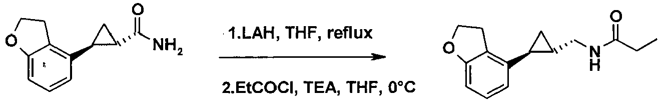

A une suspension de LAH (196 mg, 5,17 mmol) dans le tetrahydrofurane (3 ml) à température ambiante et sous atmosphère inerte, est additionné l'amide (300 mg, 1,48 mmol) par portions puis le milieu est porté à reflux pendant 3 heures et à température ambiante pendant une nuit. A 0°C, de l'eau (200 µl), de la soude aqueuse 6 N (200 µl), puis à nouveau de l'eau (600µl) sont additionnées au milieu. La suspension obtenue est filtrée sur célite et le filtrat est évaporé à sec. L'huile jaune obtenue est reprisse dans 6 ml de tetrahydrofurane, puis de la triéthylamine (1 ml, 7,38 mmol) est additionnée au mélange. Le chlorure de propionyle (410 mg, 4,43 mmol) est additionné goutte à goutte au mélange refroidi à 0°C. Le milieu est agité 1 h à 0°C puis 2 h à température ambiante. De l'eau et du dichlorométhane sont additionnés, puis les phases sont séparées. La phase aqueuse est extraite 3 fois au dichlorométhane. Les phases organiques rassemblées sont lavées à l'eau, à la saumure, puis séchées sur sulfate de magnésium, filtrées et concentrées à sec pour conduire à une huile jaune purifiée par chromatographie sur gel de silice (gradient heptane/acétate d'éthyle). Le produit attendu est obtenu sous forme d'une huile jaune.
**¹H-RMN (CDCl₃, 300 MHz)** δ 7.04 (dd, J=7.8Hz, 1H), 6.63 (d, J=7.8Hz, 1H), 6.35 (d, J=7.8Hz, 1H), 5.64 (s,1H), 4.61 (t, J=8.7Hz, 2H), 3.29 (m, 4H), 2.24 (q, J=7.5Hz, 2H), 1.75 (m, 1H), 1.25 (m, 1H), 1.17 (t, J=7.5Hz, 3H), 0.96 (m, 2H).
**¹³C-RMN (CDCl₃, 300 MHz)** 174.2, 160.0, 139.3, 128.6, 126.4, 116.0, 107.2, 71.5, 44.0, 30.2, 29.0, 22.1, 20.1, 13.9, 10.3.
LC/MS > 97 %, m/z [M+H]⁺ = 246.2.

### • Voie 2 :

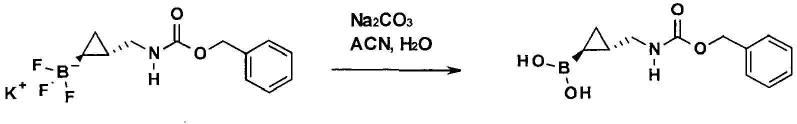

A une solution de trifluoroborate de potassium (**exemple 9**, 730 mg, 2,35 mmol) dans un mélange acétonitrile (23,4 ml) / eau (11,7 ml) est additionné le carbonate de sodium (373 mg, 3,52 mmol). Le milieu est agité 1h à température ambiante. Une solution aqueuse saturée de chlorure d'ammonium est additionnée jusqu'à l'obtention d'un pH voisin de 6 puis le milieu est extrait 4 fois à l'éther isopropylique. Les phases organiques sont lavées à l'eau puis séchées sur sulfate de magnésium, filtrées et concentrées à sec pour conduire à un solide blanc (530 mg, rendement 91 %).
**¹H-RMN (MeOD, 300 MHz)** δ 7.33 (m, 5H), 5.06 (s, 2H), 3.02 (m, 2H), 1.12 (m,1H), 0.63 (m, 1H), 0,48 (m, 1H), -0.17 (m, 1H).

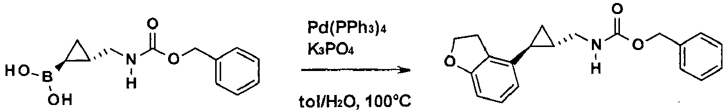

L'acide boronique (530 mg, 2,13 mmol) est mis en solution dans un mélange toluène (10,6 ml) et eau (2,6 ml). Le dihydrobenzofurane bromé (848 mg, 4,26 mmol) et le palladium tetrakis (123 mg, 0,11 mmol) sont additionnés. Le milieu en agitation est mis sous vide, puis sous azote 3 fois. Le phosphate de potassium (1,4 g, 6,39 mmol) est additionné puis le milieu est chauffé à 100°C une nuit sous azote. De l'eau est ajoutée et le produit attendu est extrait 3 fois avec de l'acétate d'éthyle. Les phases organiques rassemblées sont lavées à l'eau puis à la saumure, et elles sont séchées sur sulfate de magnésium, filtrées et concentrées pour conduire à 1,2g d'une huile marron. Le produit attendu est purifié par chromatographie sur gel de silice (gradient heptane/acétate d'éthyle) et il est obtenu sous forme d'une huile jaune (413 mg, rendement 60 %).
**¹H-RMN (CDCl3, 300 MHz)** δ 7.29 (m, 5H), 6.94 (dd, J=8.1Hz, 1H), 6.54 (d, J=7.8Hz, 1H), 6.26 (d, J=7.8Hz, 1H), 5.08 (s, 2H), 4.85 (s, 1H), 4.48 (t, J=8.7Hz, 2H), 3.16 (m, 4H), 1.66 (m, 1H), 1.23 (m,1H), 0.90 (m, 1H), 0,81 (m, 1H).
**¹³C-RMN (CDCl₃, 300 MHz)** 160.0, 156.8, 139.2, 136.9, 129.0, 128.6, 126.5, 116.2, 107.2, 71.4, 67.1, 45.6, 29.0, 22.5, 19.9, 13.5. □

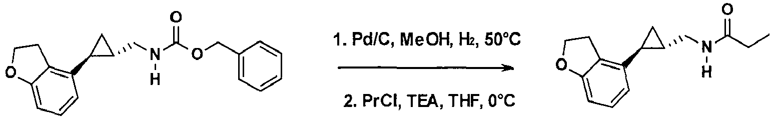

A une solution de carbamate (380mg, 1,17 mmol) dans le méthanol (7,6 ml), dégazé 3 fois par des purges vide / azote, est additionné le palladium sur charbon (38 mg). Le milieu est purgé à nouveau 3 fois à l'azote puis placé sous atmosphère d'hydrogène. Le mélange est chauffé à 50°C sous Hydrogène pendant une nuit. La suspension obtenue est filtrée sur célite puis le filtrat est évaporé à sec. L'huile jaune obtenue est reprise dans 5 ml de tétrahydrofurane, puis de la triéthylamine (819 µl, 5,85 mmol) est additionnée au mélange. Le chlorure de propionyle (308 µl, 3,51 mmol) est additionné goutte à goutte au mélange refroidi à 0°C. Le milieu est agité 1h à 0°C puis 1h30 à température ambiante. De l'eau et du dichlorométhane sont additionnés, puis les phases sont séparées. La phase aqueuse est extraite 3 fois au dichlorométhane. Les phases organiques rassemblées sont lavées à l'eau, à la saumure, puis séchées sur sulfate de magnésium, filtrées et concentrées à sec pour conduire à une huile jaune purifiée par chromatographie sur gel de silice (gradient heptane/acétate d'éthyle). Le produit attendu est obtenu sous forme d'une huile jaune.
**¹H-RMN (CDCl₃, 300 MHz)** δ 7.04 (dd, J=7.8Hz, 1H), 6.63 (d, J=7.8Hz, 1H), 6.35 (d, J=7.8Hz, 1H), 5.64 (s,1H), 4.61 (t, J=8.7Hz, 2H), 3.29 (m, 4H), 2.24 (q, J=7.5Hz, 2H), 1.75 (m, 1H), 1.25 (m, 1H), 1.17 (t, J=7.5Hz, 3H), 0.96 (m, 2H).
**¹³C-RMN (CDCl₃, 300 MHz)** 174.2, 160.0, 139.3, 128.6, 126.4, 116.0, 107.2, 71.5, 44.0, 30.2, 29.0, 22.1, 20.1, 13.9, 10.3.

## Revendications

1. Procédé de préparation d'un composé répondant à la formule (I-A) suivante 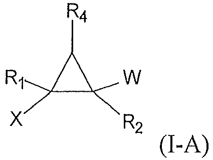 dans laquelle :
▪ X représente un atome de bore substitué choisi dans le groupe comprenant B(OH)₂, B(OR)₂, BF₃M, B(OR')₃M dans lesquels :
∘ R est un groupe alkyle comportant de 1 à 14 atomes de carbone ou un groupe aryle, éventuellement substitués, ou est tel que (OR)₂ forme un cycle entre les deux atomes d'oxygène, (OR)₂ étant notamment choisi dans le groupe comprenant les radicaux bivalents dérivant de diols, tels que O-CH₂-CH₂-O, O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O et ses esters, et les radicaux bivalents dérivant de diacides, tels que OCO-CH₂-N(CH₃)-CH₂-COO,
∘ R' est un groupe alkyle comportant de 1 à 14 atomes de carbone ou est tel que :
• (OR')₃ forme un cycle entre deux des atomes d'oxygène, (OR')₃ étant alors sous la forme OR'(OR)₂, où R' est un groupe alkyle comportant de 1 à 14 atomes de carbone et (OR)₂ est tel que défini ci-dessus, ou
• (OR')₃ forme un bicycle entre les trois atomes d'oxygène, (OR')₃ étant notamment choisi dans le groupe comprenant les radicaux trivalents dérivant de triols, tels que H₃C-C-(CH₂-O)₃,
∘ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} R^{e}, R^{f} sont choisis parmi H ou une chaîne carbonée saturée comportant notamment 1 à 6 atomes de carbone choisis indépendamment les uns des autres,
et notamment X représente B(OH)₂, B(OR)₂ ou BF₃K,
▪ R₁ et R₄ identiques ou différents sont choisis dans le groupe constitué:
1. de H
2. des aryles comportant des cycles de 6 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONH^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles de 1 à 15 atomes de carbone éventuellement substitués,
∘ par des radicaux alcényles de 1 à 15 atomes de carbone éventuellement substitués,
∘ par des radicaux alcynyles de 1 à 15 atomes de carbone éventuellement substitués,
∘ par des aryles de 6 à 12 atomes de carbone éventuellement substitués,
∘ par des hétérocycles aromatiques ou non-aromatiques de 2 à 12 atomes de carbone éventuellement substitués,
dans lesquels R^{a} et R^{b}, identiques ou différents, représentent des groupements alkyles, alcényles, alcynyles linéaires, cycliques ou ramifiés, aromatiques ou hétérocycliques aromatiques ou non-aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone, linéaires ou ramifiés éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des aryles de 6 à 12 atomes de carbone éventuellement substitués,
∘ par des hétérocycles aromatiques ou non-aromatiques comportant de 2 à 12 atomes de carbone, éventuellement substitués,
3. des hétérocycles ou des hétéroaryles comportant des cycles de 2 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF³, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués par :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non aromatiques comportant de 2 à 12 atomes de carbone éventuellement substitués,
4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non aromatiques comportant de 2 à 12 atomes de carbone éventuellement substitués,
5. des alcynyles linéaires ou ramifiés comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a} -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
6. des groupes alkyles linéaires, cycliques ou ramifiés comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR³,-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR', -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
l'un au moins des groupes R₁ et R₄ représentant H,
▪ R₂ est choisi dans le groupe constitué par les groupes pouvant être représentés par R₁ ou R₄, ainsi que -COR^{a}, -COOR^{a}, -CONH₂, -CONHR^{a}, -CONR^{a}R^{b}, -CN et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a},-N(C=NH)NH₂, -N-(C=NCOR^{a})NHCOR^{b}, -N(C=NCOOR^{a})NHCOOR^{b},
dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
R^{a} et R^{b} pouvant être reliés pour former un cycle, éventuellement substitué,
▪ W représente un groupe fonctionnel choisi parmi -COR^{a}, -COOH, -COOR^{a},-CONH₂, -CONHR^{a}, -CONR^{a}R^{b}, -CONH-SO₂-R^{a}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a}, dans lesquels Z représente un groupe protecteur d'une fonction amine, et
dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a} -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
ledit procédé comprenant :
• une étape de réaction entre :
▪ un dérivé diazoïque de formule suivante : 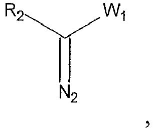 dans laquelle R₂ est tel que défini ci-dessus, W₁ étant choisi dans le groupe constitué de -COR^{a}, -COOR^{a}, -CONH₂, -CONHR^{a}, et -CONR^{a}R^{b},
et
▪ un composé vinyltrifluoroborate de formule suivante : 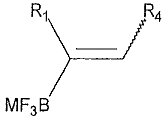 dans laquelle R₁, R₄ et M sont tels que définis ci-dessus,
en présence d'un catalyseur contenant un métal de transition, pour obtenir un composé de formule suivante : 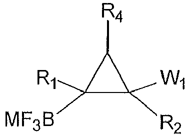 dans laquelle R₁, R₂, R₄, W₁ et M sont tels que définis ci-dessus,
si W est différent de W₁ et/ou X est différent de MF₃B, ledit procédé comprenant en outre les étapes suivantes :
• une étape de conversion de W₁ en W permettant d'obtenir 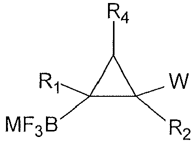 à partir de 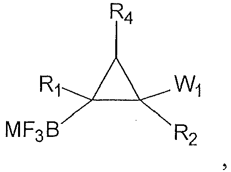 en particulier, lorsque W₁ = COOR^{a} et W = CHO, par réduction pour former l'alcool correspondant, puis oxydation dudit alcool, lorsque W₁ = -COOR^{a} et W= CH₂OH ou - CH₂OR^{b}, par formation d'un aldéhyde tel que décrit précédemment, puis par réduction dudit aldéhyde et alkylation éventuelle, lorsque W₁ = -COR^{a} et W = -CHR^{a}OH,-CHR^{a}OR^{b}, par réduction puis alkylation éventuelle de l'alcool obtenu, lorsque W₁=-COR^{a} et W= CR^{a}R^{b}OH ou -CR^{a}R^{b}OR^{b'} par addition d'un Grignard puis alkylation éventuelle de l'alcool obtenu, lorsque W₁ = -CONH₂, -CONHR^{a}ou -CONR^{a}R^{b} et W =-CH₂NH₂, -CH₂NHR^{a}, -CH₂NR^{a}R^{b}, -CH₂NHZ ou -CH₂-NH-COR^{a}, par réduction puis protection éventuelle par Z de l'amine obtenue ou réaction éventuelle avec le chlorure d'acide R^{a}COCl, lorsque W₁ = -CONH₂ et W = -CONHSO₂R^{a}, par action du chlorure de sulfonyle ClSO₂R^{a} sur l'amide,
et
• une étape de conversion de -BF₃M en -X permettant d'obtenir 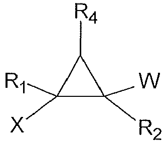 à partir de 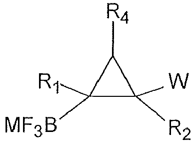 en particulier, lorsque X = B(OH)₂, par hydrolyse basique ou acide, ou par passage par un dihalogénoborane, plus particulièrement un dichloroborane, lorsque X = B(OR)₂, par passage par X = B(OH)₂ comme décrit précédemment puis par action d'un alcool, en particulier un alcool de formule ROH, un diol ou un triol, ou par passage par un dihalogénoborane, plus particulièrement un dichloroborane, puis par action d'un alcool, en particulier un alcool de formule ROH, un diol ou un triol,
ou
• une étape de conversion de -BF₃M en -X permettant d'obtenir 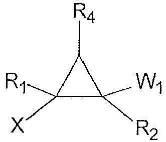 à partir de 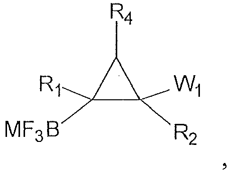 et
• une étape de conversion de W₁ en W permettant d'obtenir 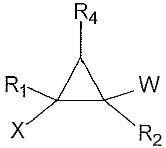 à partir de

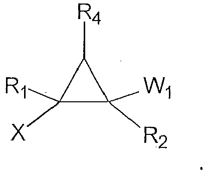

2. Procédé selon la revendication 1, dans lequel ledit catalyseur contenant un métal de transition, ledit catalyseur étant en particulier un complexe de palladium (II), plus particulièrement Pd(OAc)₂ ou Pd(acac)₂, un complexe du cuivre (II), plus particulièrement CuSO₄, Cu(acac)₂, Cu(tBuSalen)₂, Cu(OTf)₂, un complexe de cuivre (I), plus particulièrement Cul ou Cu(OTf), ou un complexe du rhodium (II), plus particulièrement Rh₂(OAc)₄, Rh₂(Octanoate)₄ ou Rh₂(5S-MEPY)₄ (catalyseur de Doyle).

3. Procédé selon l'une quelconque des revendications 1 à 2, dans lequel W₁ représente-COOR^{a}, R^{a} étant tel que défini dans la revendication 1, en particulier dans laquelle W représente -COOR^{a}, -CONH₂, -CONHR^{a}, -CONR^{a}R^{b},-CH₂OH, -CH₂OR^{a}, -CHR^{b}OH, -CHR^{b}OR^{a}, -CR^{b}R^{b'}OH, -CR^{b}R^{b'}OR^{a}, -CH₂NH₂, -CH₂NHZ, -CHR^{a}NHZ, ledit procédé comprenant :
• une étape de réaction entre :
un dérivé diazoïque de formule suivante : 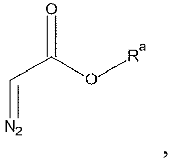 dans laquelle R^{a} est tel que défini dans la revendication 1,
et
un composé vinyltrifluoroborate de formule suivante : 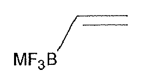 dans laquelle M est tel que défini dans la revendication 1, M représentant en particulier K,
en présence d'un catalyseur contenant un métal de transition, pour obtenir un composé de formule suivante : 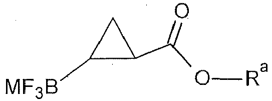 dans laquelle R^{a} et M sont tels que définis ci-dessus,
si W est différent de -COOR^{a} et/ou X est différent de MF₃B, ledit procédé comprenant en outre les étapes suivantes :
• une étape de conversion de -COOR^{a} en W permettant d'obtenir 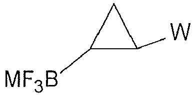 à partir de 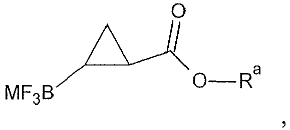 et
• une étape de conversion de -BF₃M en -X permettant d'obtenir 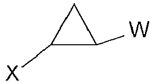 à partir de 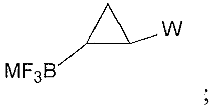 ou
• une étape de conversion de -BF₃M en -X permettant d'obtenir 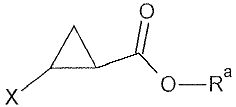 à partir de 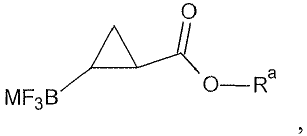 et
• une étape de conversion de -COOR^{a} en W permettant d'obtenir 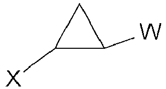 à partir de

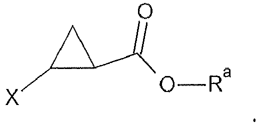

4. Procédé selon l'une quelconque des revendications 1 à 2, dans lequel :
❖ R₂ est choisi dans le groupe constitué par les groupes -COR^{a}, -COOR^{a}, -CONH₂,-CONHR^{a}, -CONR^{a}R^{b}, -CN et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b}, -CHO_{,} -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, , -N=C-NHCOR^{a}, -N=C-NH-COOR^{a}, - N(C=NH)NH₂, -N-(C=NCOR^{a})NHCOR^{b}, -N(C=NCOOR^{a})NHCOOR^{b},
dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
R^{a} et R^{b} pouvant être reliés pour former un cycle, éventuellement substitué,
❖ ou dans lequel R₂ représente H,
❖ ou dans lequel R₁, R₂ et R₄ représentent H,
❖ ou dans lequel R₁ représente H,
❖ ou dans lequel R₁ représente H et R₄ représente un aryle, un hétérocycle, un hétéroaryle ou un alkyle tels que définis dans la revendication 1,
❖ ou dans lequel R₄ représente H,
❖ ou dans lequel R₄ représente H et R₁ représente un aryle, un hétérocycle, un hétéroaryle ou un alkyle tels que définis dans la revendication 1,
❖ ou dans lequel R₁ et R₂ représentent H et R₄ représente un aryle, un hétérocycle, un hétéroaryle ou un alkyle tels que définis dans la revendication 1,
❖ ou dans lequel R₂ et R₄ représentent H et R₁ représente un aryle, un hétérocycle, un hétéroaryle ou un alkyle tels que définis dans la revendication 1.

5. Procédé selon l'une quelconque des revendications 1 à 4, dans lequel W représente un groupe fonctionnel choisi parmi -CONH-SO₂-cyclopropyle, -CH₂-NH-CO-CH₂-CH₃, et le groupe de formule suivante :

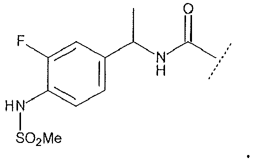

6. Composé répondant à la formule (I-A) suivante 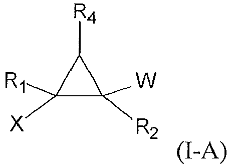 dans laquelle :
▪ X représente un atome de bore substitué choisi dans le groupe comprenant B(OH)₂, B(OR)₂, BF₃M, B(OR')₃M dans lesquels :
∘ R est un groupe alkyle comportant de 1 à 14 atomes de carbone, un groupe aryle éventuellement substitués, ou est tel que (OR)₂ forme un cycle entre les deux atomes d'oxygène, (OR)₂ étant notamment choisi dans le groupe comprenant les radicaux bivalents dérivant de diols, tels que O-CH₂-CH₂-O, O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O et ses esters, et les radicaux bivalents dérivant de diacides, tels que OCO-CH₂-N(CH₃)-CH₂-COO,
∘ R' est un groupe alkyle comportant de 1 à 14 atomes de carbone ou est tel que :
• (OR')₃ forme un cycle entre deux des atomes d'oxygène, (OR')₃ étant alors sous la forme OR'(OR)₂, où R' est un groupe alkyle comportant de 1 à 14 atomes de carbone et (OR)₂ est tel que défini ci-dessus, ou
• (OR')₃ forme un bicycle entre les trois atomes d'oxygène, (OR')₃ étant notamment choisi dans le groupe comprenant les radicaux trivalents dérivant de triols, tels que H₃C-C-(CH₂-O)₃,
∘ M représente l'ion lithium Li⁺, l'ion sodium Na⁺, l'ion potassium K⁺, l'ion césium Cs⁺, l'ion ammonium R^{c}R^{d}R^{e}R^{f}N⁺ où R^{c}, R^{d} R^{e}, R^{f} sont choisis parmi H ou une chaîne carbonée saturée comportant notamment 1 à 6 atomes de carbone choisis indépendamment les uns des autres,
et notamment X représente B(OH)₂, B(OR)₂ ou BF₃K,
R₁ et R₄ identiques ou différents sont choisis dans le groupe constitué:
1. de H
2. des aryles comportant des cycles de 6 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCORa)NHCOR^{b}, -N(C=NCOOR^{a})NHCOOR^{b},
∘ par des radicaux alkyles de 1 à 15 atomes de carbone éventuellement substitués,
∘ par des radicaux alcényles de 1 à 15 atomes de carbone éventuellement substitués,
∘ par des radicaux alcynyles de 1 à 15 atomes de carbone éventuellement substitués,
∘ par des aryles de 6 à 12 atomes de carbone éventuellement substitués,
∘ par des hétérocycles aromatiques ou non-aromatiques de 2 à 12 atomes de carbone éventuellement substitués,
dans lesquels R^{a} et R^{b}, identiques ou différents, représentent des groupements alkyles, alcényles, alcynyles linéaires, cycliques ou ramifiés, aromatiques ou hétérocycliques aromatiques ou non-aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCORb, -N(C₌NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone, linéaires ou ramifiés éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des aryles de 6 à 12 atomes de carbone éventuellement substitués,
∘ par des hétérocycles aromatiques ou non-aromatiques comportant de 2 à 12 atomes de carbone, éventuellement substitués,
3. des hétérocycles ou des hétéroaryles comportant des cycles de 2 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF3, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués par :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non aromatiques comportant de 2 à 12 atomes de carbone éventuellement substitués,
4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONH^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non aromatiques comportant de 2 à 12 atomes de carbone éventuellement substitués,
5. des alcynyles linéaires ou ramifiés comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NH^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a} -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR³, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
6. des groupes alkyles linéaires, cycliques ou ramifiés comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a} -NHR^{a}, -NR^{a}R^{b}, -SR^{a}, -OCOR^{a}, -OCONHR^{a} -OCONR^{a}R^{b},-CHO, -COR^{a}, -COOH, -CN, -COOR^{a}, -CONHR^{a}, -CONR^{a}R^{b}, -CF₃, -NO₂ -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
l'un au moins des groupes R₁ et R₄ représentant H,
▪ R₂ est choisi dans le groupe constitué par les groupes pouvant être représentés par R₁ ou R₄, ainsi que -COR^{a}, -COOR^{a}, -CONH₂, -CONHR^{a}, -CONR^{a}R^{b}, -CN et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a},-N(C=NH)NH₂, -N-(C=NCOR^{a})NHCOR^{b}, -N(C=NCOOR^{a})NHCOOR^{b},
dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CONH-SO₂-R^{a}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a}, dans lesquels Z représente un groupe protecteur d'une fonction amine, et
dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
o par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués, sous réserve que :
▪ lorsque R₁, R₂ et R₄ représentent H et que B représente B(OH)₂, B(OR)₂, ou B(OR')₃M, alors W est choisi parmi -COR^{a}, -CONH₂, -CONHR^{a}, -CONR^{a}R^{b},
▪ lorsque R₁, R₂ et R₄ représentent H et que B représente BF₃M, alors W est choisi parmi-COOH, -COOR^{a}, -CHO, -COR^{a} -CONH₂, -CONHR^{a}, -CONR^{a}R^{b},
▪ lorsque W représente CH₂OH, -CH₂OR^{a}, -CHR^{b}OH, -CHR^{b}OR^{a}, -CR^{b}R^{b'}OH, ou - CR^{b}R^{b'}OR^{a}, et que B représente B(OH)₂, B(OR)₂, ou B(OR')₃M, alors :
∘ R₁ ne représente pas H, ou
∘ R₂ et R₄ ne représentent pas H,
▪ lorsque W représente CH₂OH, -CH₂OR^{a}, -CHR^{b}OH, -CHR^{b}OR^{a}, -CR^{b}R^{b'}OH, ou-CR^{b}R^{b'}OR^{a}, et que B représente BF₃M, alors R₁ ou R₂ ne représente pas H.
▪ lorsque W représente -COOH ou -COOR^{a}, et que B représente B(OH)₂, B(OR)₂, ou B(OR')₃M, alors :
∘ R₁ ne représente pas H, ou
∘ R₂ et R₄ ne représentent pas H,.

7. Composé selon la revendication 6, dans lequel W représente un groupe fonctionnel choisi parmi -CHO, -COR^{a}, -COOH, -COOR^{a}, -CONH₂, -CONHR^{a}, -CONR^{a}R^{b}, -CH₂OH,-CH₂OR^{a}, -CHR^{b}OH, -CHR^{b}OR^{a}, -CR^{b}R^{b'}OH, -CR^{b}R^{b'}OR^{a}, -CH₂NH₂, -CH₂NHZ,-CHR^{a}NHZ,
❖ en particulier dans lequel W représente un groupe fonctionnel choisi parmi -CHO, -COR^{a}, -COOH, -COOR^{a}, -CONH₂, -CONHR^{a}, -CONR^{a}R^{b}, -CH₂OH, -CH₂OR^{a}, -CHR^{b}OH,-CHR^{b}OR^{a}, -CR^{b}R^{b'}OH, -CR^{b}R^{b'}OR^{a},
∘ plus particulièrement dans lequel W représente un groupe fonctionnel choisi parmi -CHO, -COR^{a}, -COOH, -COOR^{a}, -CONH₂, -CONHR^{a}, -CONR^{a}R^{b}.
❖ en particulier dans lequel W représente un groupe fonctionnel choisi parmi -CONH-SO₂- cyclopropyle, -CH₂-NH-CO-CH₂-CH₃, et le groupe de formule suivante : 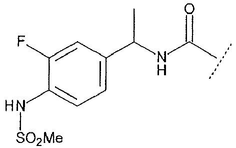
❖ en particulier dans lequel R₂ représente H,
❖ en particulier dans lequel R₂ est choisi dans le groupe constitué par les groupes -COR^{a},-COOR^{a}, -CONH₂, -CONHR^{a}, -CONR^{a}R^{b}, -CN et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a},-N(C=NH)NH₂, -N-(C=NCOR^{a})NHCOR^{b}, -N(C=NCOOR^{a})NHCOOR^{b},
dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
o par des radicaux aryles comportant de 6 à 12 atomes de carbone, linéaires ou ramifiés, éventuellement substitués,
o par des hétérocycles aromatiques ou non-aromatiques comportant de 2 à 12 atomes de carbone éventuellement substitués,
R^{a} et R^{b} pouvant être reliés pour former un cycle, éventuellement substitué,
❖ en particulier dans lequel R₁ et R₄ représentent H, un aryle, un hétérocycle, un hétéroaryle ou un alkyle tels que définis dans la revendication 6,
❖ en particulier dans lequel R₁, R₂ et R₄ représentent H,
❖ en particulier dans lequel R₁ représente H,
❖ en particulier dans lequel R₁ représente H et R₄ représente un aryle, un hétérocycle, un hétéroaryle ou un alkyle tels que définis dans la revendication 6,
❖ en particulier dans lequel R₄ représente H,
❖ en particulier dans lequel R₄ représente H et R1 représente un aryle, un hétérocycle, un hétéroaryle ou un alkyle tels que définis dans la revendication 6,
❖ en particulier dans lequel R₁ et R₂ représentent H et R₄ représente un aryle, un hétérocycle, un hétéroaryle ou un alkyle tels que définis dans la revendication 6,
❖ en particulier dans lequel R₂ et R₄ représentent H et R₁ représente un aryle, un hétérocycle, un hétéroaryle ou un alkyle tels que définis dans la revendication 6.

8. Composé selon l'une quelconque des revendications 6 à 7, ledit composé étant choisi dans le groupe constitué des composés de formule suivante et leur énantiomère:

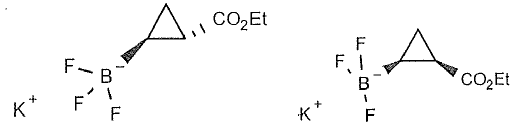

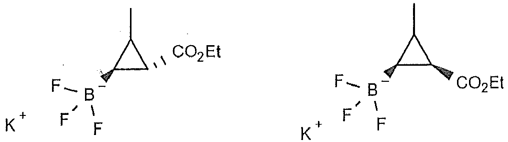

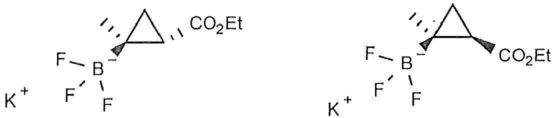

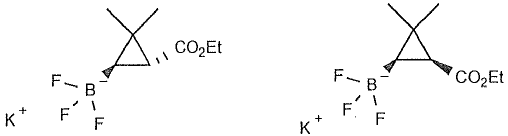

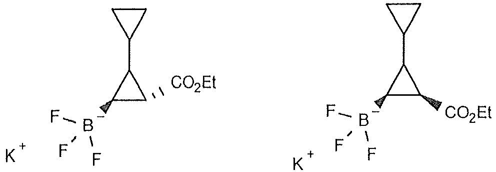

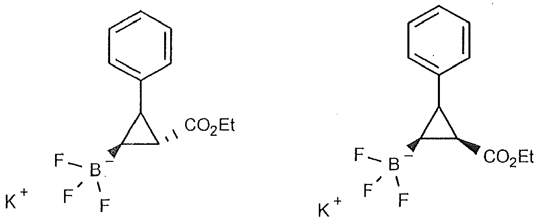

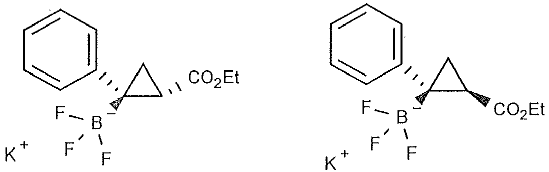

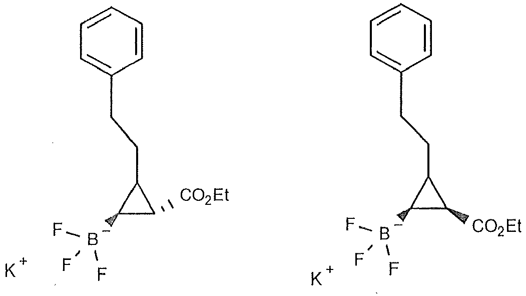

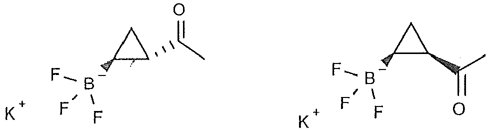

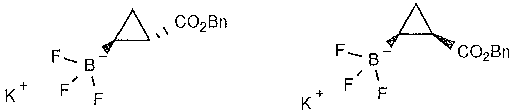

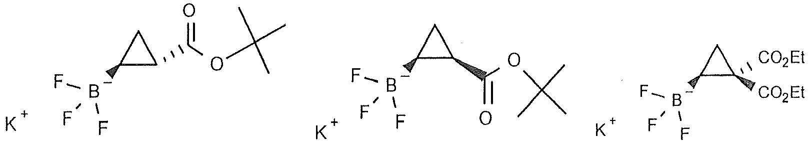

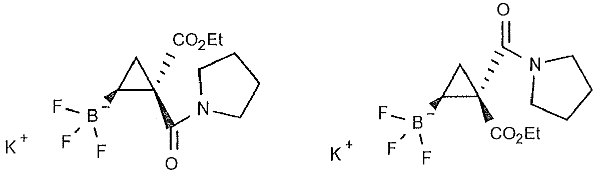

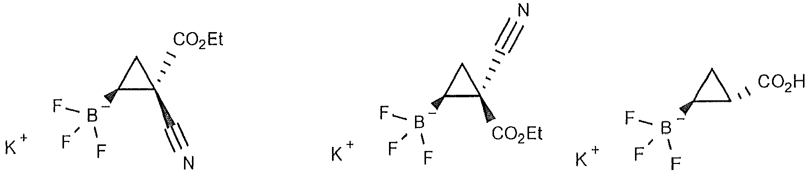

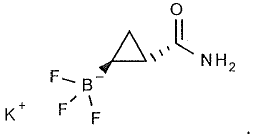

9. Utilisation d'un composé de formule (I-A) selon la revendication 6, pour la préparation de composés de formule (II-A) suivante : 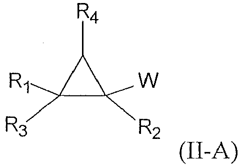 dans laquelle :
▪ R₁, R₃ et R₄ identiques ou différents sont choisis dans le groupe constitué:
1. de H, sous réserve que R₃ ne représente pas H,
2. des aryles comportant des cycles de 6 à 15 atomes de carbone éventuellement substitués :
o par 1 ou plusieurs atomes d'halogène comprenant le fluor, le chlore, le brome ou l'iode,
o par des radicaux hydroxy, amino ou thio éventuellement protégés par des groupes protecteurs « ad hoc »,
o par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles de 1 à 15 atomes de carbone éventuellement substitués,
∘ par des radicaux alcényles de 1 à 15 atomes de carbone éventuellement substitués,
∘ par des radicaux alcynyles de 1 à 15 atomes de carbone éventuellement substitués,
∘ par des aryles de 6 à 12 atomes de carbone éventuellement substitués,
∘ par des hétérocycles aromatiques ou non-aromatiques de 2 à 12 atomes de carbone éventuellement substitués,
dans lesquels R^{a} et R^{b}, identiques ou différents, représentent des groupements alkyles, alcényles, alcynyles linéaires, cycliques ou ramifiés, aromatiques ou hétérocycliques aromatiques ou non-aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
o par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone, linéaires ou ramifiés éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des aryles de 6 à 12 atomes de carbone éventuellement substitués,
∘ par des hétérocycles aromatiques ou non-aromatiques comportant de 2 à 12 atomes de carbone, éventuellement substitués,
3. des hétérocycles ou des hétéroaryles comportant des cycles de 2 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués par :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non aromatiques comportant de 2 à 12 atomes de carbone éventuellement substitués,
4. des alcényles linéaires ou ramifiés comportant de 1 à 12 atomes de carbone, éventuellement substitués, ou des cycles carbonés comportant de 3 à 12 atomes de carbone et une ou plusieurs liaisons doubles C=C, éventuellement substituées :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non aromatiques comportant de 2 à 12 atomes de carbone éventuellement substitués,
5. des alcynyles linéaires ou ramifiés comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a}, -CONHR^{a}, -CONR^{a}R^{b},-CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
6. des groupes alkyles linéaires, cycliques ou ramifiés comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b} -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
∘ par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a},-OCONR^{a}R^{b}, -CHO, -COR^{a}, -COOH, -CN, -COOR^{a} -CONHR^{a}, -CONR^{a}R^{b},-CF₃, -NO₂ -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b}, dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
l'un au moins des groupes R₁ et R₄ représentant H,
▪ R₂ est choisi dans le groupe constitué par les groupes pouvant être représentés par R₁ ou R₄, ainsi que -COR^{a}, -COOR^{a}, -CONH₂, -CONHR^{a}, -CONR^{a}R^{b}, -CN et -NO₂,
dans lesquels R^{a} et R^{b} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
o par 1 ou plusieurs atomes d'halogène comprenant le fluor, le chlore, le brome ou l'iode,
∘ par des radicaux hydroxy, amino ou thio éventuellement protégés par des groupes protecteurs « ad hoc »,
∘ par des radicaux -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a},-N(C=NH)NH₂, -N-(C=NCOR^{a})NHCOR^{b}, -N(C=NCOOR^{a})NHCOOR^{b},
dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
R^{a} et R^{b} pouvant être reliés pour former un cycle, éventuellement substitué,
▪ W représente un groupe fonctionnel choisi parmi -CHO, -COR^{a}, -COOH, -COOR^{a},-CONH₂, -CONHR^{a}, -CONR^{a}R^{b}, -CONH-SO₂-R^{a}, -CH₂OH, -CH₂OR^{a}, -CHR^{b}OH,-CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a}, dans lesquels Z représente un groupe protecteur d'une fonction amine, et
dans lesquels R^{a}, R^{b} et R^{b'} identiques ou différents représentent des groupements alkyles, alcényles, alcynyles linéaires, cycliques ou ramifiés, aromatiques ou hétérocycliques aromatiques ou non aromatiques comportant de 1 à 15 atomes de carbone éventuellement substitués :
o par 1 ou plusieurs atomes d'halogène comprenant le fluor, le chlore, le brome ou l'iode,
o par des radicaux hydroxy, amino ou thio éventuellement protégés par des groupes protecteurs « ad hoc »,
o par des radicaux -OR^{a}, -NHR^{a}, -NR^{a}R^{b}, -SR^{a}, -OCOR^{a}, -OCONHR^{a}, -OCONR^{a}R^{b},-CHO, -COR^{a}, -COOH, -CN, -COOR^{a}, -CONHR^{a}, -CONR^{a}R^{b}, -CF₃, -NO₂ -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
dans lesquels R^{a} et R^{b}, identiques ou différents, représentent des groupements alkyles, alcényles, alcynyles linéaires ou ramifiés, aromatiques ou hétérocycliques aromatiques ou non-aromatiques comportant de 1 à 15 atomes de carbone,
∘ par des radicaux alkyles comportant de 1 à 15 atomes de carbone éventuellement substitués,
∘ par des radicaux alcényles comportant de 1 à 15 atomes de carbone, linéaires ou ramifiés, éventuellement substitués,
∘ par des radicaux alcynyles comportant de 1 à 15 atomes de carbone, linéaires ou ramifiés éventuellement substitués,
∘ par des radicaux aryles comportant de 6 à 12 atomes de carbone, linéaires ou ramifiés, éventuellement substitués,
∘ par des hétérocycles aromatiques ou non-aromatiques comportant de 2 à 12 atomes de carbone éventuellement substitués,
sous réserve que :
▪ lorsque R₁, R₂ et R₄ représentent H et que B représente B(OH)₂, B(OR)₂, ou B(OR')₃M, alors W est choisi parmi -COR^{a}, -CONH₂, -CONHR^{a}, et -CONR^{a}R^{b},
▪ lorsque R₁, R₂ et R₄ représentent H et que B représente BF₃M, alors W est choisi parmi -COOH, -COOR^{a}, -CHO, -COR^{a} -CONH₂, -CONHR^{a}, -CONR^{a}R^{b},
▪ lorsque W représente CH₂OH, -CH₂OR^{a}, -CHR^{b}OH, -CHR^{b}OR^{a}, -CR^{b}R^{b'}OH, ou - CR^{b}R^{b'}OR^{a}, et que B représente B(OH)₂, B(OR)₂, ou B(OR')₃M, alors :
∘ R₁ ne représente pas H, ou
∘ R₂ et R₄ ne représentent pas H,
▪ lorsque W représente CH₂OH, -CH₂OR^{a}, -CHR^{b}OH, -CHR^{b}OR^{a}, -CR^{b}R^{b'}OH, ou - CR^{b}R^{b'}OR^{a}, et que B représente BF₃M, alors R₁ ou R₂ ne représente pas H.
▪ lorsque W représente -COOH ou -COOR^{a}, et que B représente B(OH)₂, B(OR)₂, ou B(OR')₃M, alors :
∘ R₁ ne représente pas H, ou
∘ R₂ et R₄ ne représentent pas H,
par réaction dudit composé de formule (I) avec un composé de formule (III) suivante :
R₃-X₂,
dans laquelle R₃ est tel que défini ci-dessus, et X₂ est choisi dans le groupe constitué des halogénures, en particulier l'iode, le brome et le chlore, et du triflate (OTf), en présence d'un catalyseur contenant un métal de transition, et, éventuellement, d'un ligand.

10. Utilisation selon la revendication 9, dans laquelle R₃ représente un groupe choisi parmi :

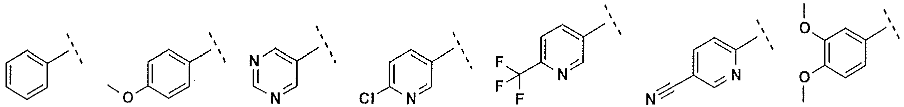

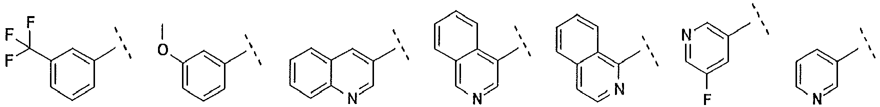

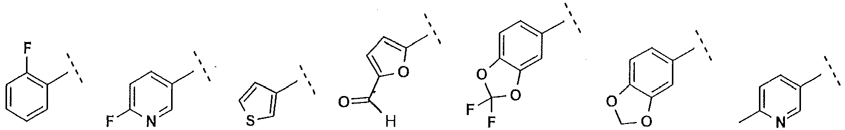

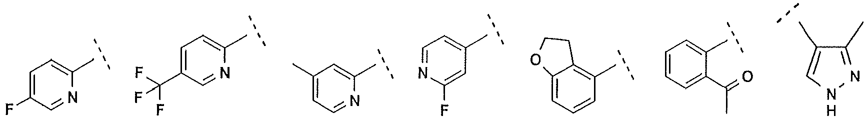

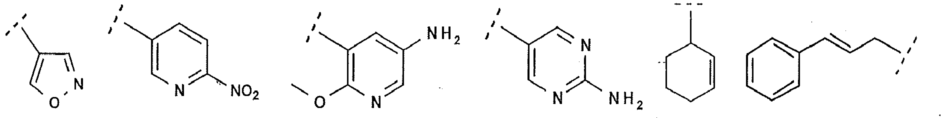

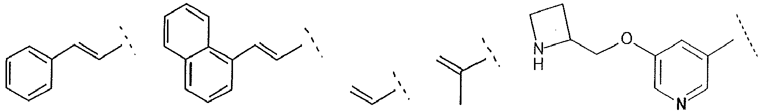

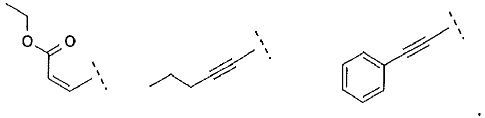

## Patentansprüche

1. Verfahren zur Herstellung einer Verbindung, die folgender Formel (I-A) entspricht: 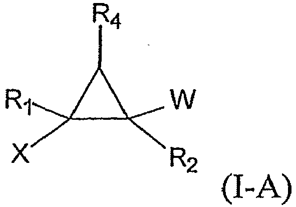 wobei:
* X ein substituiertes Boratom darstellt, das in der Gruppe ausgewählt ist, umfassend B(OH)₂, B(OR)₂, BF₃M, B(OR')₃M, wobei:
- R eine Alkylgruppe, umfassend 1 bis 14 Kohlenstoffatome, oder eine Arylgruppe, die eventuell substituiert sind, oder derart ist, das (OR)₂ einen Zyklus zwischen den beiden Sauerstoffatomen bildet, wobei (OR)₂ insbesondere in der Gruppe ausgewählt ist, umfassend die von Diolen abgeleiteten bivalenten Radikale, wie O-CH₂-CH₂-O, O-CH₂-CH₂-CH₂-O, O-CH₂-C(CH3)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O und seine Ester, und die von den Disäuren abgeleiteten bivalenten Radikale, wie OCO-CH₂-N(CH₃)-CH₂-COO,
- R' eine Alkylgruppe, umfassend 1 bis 14 Kohlenstoffatome oder derart ist, dass:
* (OR')₃ einen Zyklus zwischen zwei Sauerstoffatomen bildet, wobei (OR')₃ nun in Form von OR'(OR)₂ ist, wobei R' eine Alkylgruppe, umfassend 1 bis 14 Kohlenstoffatome, und (OR)₂ wie oben definiert ist, oder
* (OR')₃ einen Bizyklus zwischen den drei Sauerstoffatomen bildet, wobei (OR')₃ insbesondere in der Gruppe ausgewählt ist, umfassend die von Triolen abgeleiteten trivalenten Radikale, wie H₃C-C-(CH₂-O)₃,
- M das Lithiumion Li⁺, das Natriumion Na⁺, das Kaliumion K⁺, das Cäsiumion Cs⁺, das Ammoniumion R^{c}R^{d}R^{e}R^{f}N⁺ darstellt, wobei R^{c}R^{d}R^{e}R^{f} unter H oder einer kohlenstoffhaltigen gesättigten Kette ausgewählt sind, umfassend insbesondere 1 bis 6 Kohlenstoffatome, die unabhängig voneinander ausgewählt sind,
und insbesondere X B(OH)₂, B(OR)₂ oder BF₃K darstellt,
* R₁ und R₄ identisch oder unterschiedlich sind und in der Gruppe ausgewählt sind, die gebildet ist von:
1. H
2. Arylen, umfassend Zyklen von 6 bis 15 Kohlenstoffatomen, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale mit 1 bis 15 eventuell substituierten Kohlenstoffatomen,
- durch Alcenylradikale mit 1 bis 15 eventuell substituierten Kohlenstoffatomen,
- durch Alcynylradikale mit 1 bis 15 eventuell substituierten Kohlenstoffatomen,
- durch Aryle mit 6 bis 12 eventuell substituierten Kohlenstoffatomen,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte, lineare oder verzweigte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Aryle mit 6 bis 12 eventuell substituierten Kohlenstoffatomen,
- durch aromatische oder nicht-aromatische Heterozyklen mit 2 bis 12 eventuell substituierten Kohlenstoffatomen,
3. Heterozyklen oder Heteroarylen, umfassend Zyklen von 2 bis 15 Kohlenstoffatomen, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
4. linearen oder verzweigten Alcenylen, umfassend 1 bis 12 eventuell substituierte Kohlenstoffatome oder kohlenstoffhaltige Zyklen, umfassend 3 bis 12 Kohlenstoffatome oder eine oder mehrere Doppelverbindungen C=C, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nichtaromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
5. linearen oder verzweigten Alcynylen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N (C=NCOOR^{a}) NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
6. linearen, zyklischen oder verzweigten Alkylgruppen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N (C=NCOOR^{a}) NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR ^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen, wobei mindestens eine der Gruppen R₁ und R₄ H darstellt,
* R₂ in der Gruppe ausgewählt ist, die von den Gruppen gebildet ist, die durch R₁ oder R₄ dargestellt werden können, sowie -COR^{a}, -COOR^{a}, -CONH₂, -CONHR^{a}, -CONR^{a}R^{b}, -CN und NO₂,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} verbunden sein können, um einen eventuell substituierten Zyklus zu bilden,
* W eine funktionelle Gruppe darstellt, die ausgewählt ist unter -CHO, -COR^{a}, -COOH, -COOR^{a}, CONH₂, -CONHR^{a},-CONR^{a}R^{b}, -COHNH-SO₂R^{a}, -CH₂OH, -CH₂OR^{a}, -CHR^{b}OH,-CHR^{b}OR^{a}, -CR^{b}R^{b'}OR^{a}, -CH₂NHZ, -CHR^{a}NHZ, -CH₂-NH-COR^{a}, wobei Z eine Schutzgruppe einer Aminfunktion darstellt, und
wobei R^{a}, R^{b} und R^{b'} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N (C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei das Verfahren umfasst:
* einen Reaktionsschritt zwischen:
* einem Diazoderivat folgender Formel: 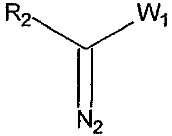 wobei R₂ wie oben definiert ist, wobei W₁ in der Gruppe ausgewählt ist, die von -COR^{a}, -COOR^{a}, CONH₂,-CONHR^{a} und -CONR^{a}R^{b} gebildet ist, und
* einer Vinyltrifluorboratverbindung folgender Formel: 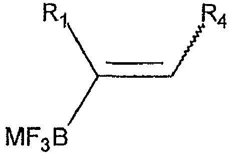 wobei R₁, R₄ und M wie oben definiert sind,
im Beisein eines Katalysators, der ein Übergangsmetall enthält, um eine Verbindung folgender Formel zu erhalten: 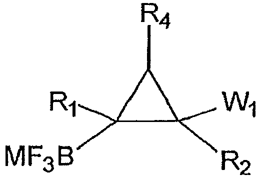 wobei R₁, R₂, R₄, W₁ und M wie oben definiert sind, wobei das Verfahren, wenn W zu W₁ unterschiedlich ist und/oder X zu MF₃B unterschiedlich ist, ferner die folgenden Schritte umfasst:
* einen Schritt der Umwandlung von W₁ in W, der es ermöglicht, folgendes zu erhalten 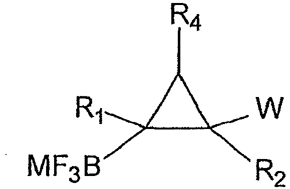 aus 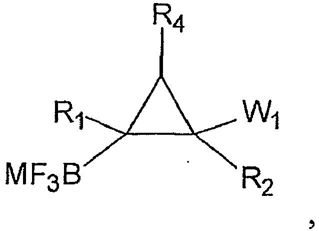 insbesondere wenn W₁ = -COOR^{a} und W = CHO, durch Reduktion, um den entsprechenden Alkohol zu bilden, dann Oxydation des Alkohols, wenn W₁ = -COOR^{a} und W = - CH₂OH oder -CH₂OR^{b}, durch Bildung eines Aldehyds, wie vorher beschrieben, dann durch Reduktion des Aldehyds und eventuelle Alkylation, wenn W₁ = -COR^{a} und W =-CHR^{a}OH, -CH^{a}OR^{b}, durch Reduktion dann eventuelle Alkylation des erhaltenen Alkohols, wenn W₁ = -COR^{a} und W = -CR^{a}R^{b}OH oder -CR^{a}R^{b}OR^{b'} durch Beigabe eines Grignard, dann eventuelle Alkylation des erhaltenen Alkohols, wenn W₁ = -CONH₂, -CONHR^{a} oder - CONR^{a}R^{b} und W = -CH₂NH₂, -CH₂NHR^{a}, -CH₂NR^{a}R^{b}, -CH₂NHZ oder -CH₂-NH-COR^{a} durch Reduktion, dann eventuellen Schutz durch Z des erhaltenen Amins oder eventuelle Reaktion mit dem Säurechlorid R^{a}COCI, wenn W₁ = -CONH₂ und W =-CONHSO₂R^{a}, durch Wirkung des Sulfonylchlorids CISO₂R^{a} auf das Amid,
und
* einen Schritt der Umwandlung von -BF₃M in -X, der es ermöglicht, folgendes zu erhalten: 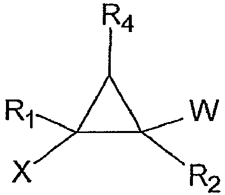 aus 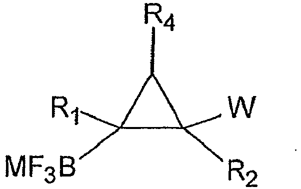 insbesondere wenn X = B(OH)₂ durch basische oder saure Hydrolyse oder durch Durchgang durch ein Dihalogenboran, insbesondere ein Dichlorboran, wenn X = B(OR)₂, durch Durchgang durch X = B(OH)₂, wie vorher beschrieben, dann Wirkung eines Alkohols, insbesondere eines Alkohols der Formel ROH, eines Diols oder eines Triols, oder durch Durchgang durch ein Dihalogenboran, insbesondere ein Dichlorboran, dann durch Wirkung eines Alkohols, insbesondere eines Alkohols der Formel ROH, eines Diols oder eines Triols,
oder
* einen Schritt der Umwandlung von -BF₃M in -X, der es ermöglicht, folgendes zu erhalten: 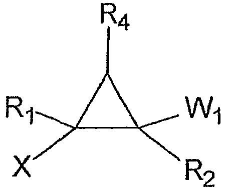 aus 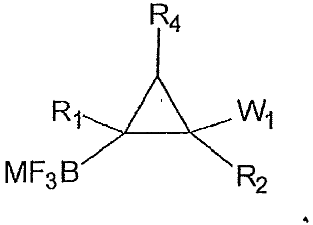 und
* einen Schritt der Umwandlung von W₁ in W, der es ermöglicht, folgendes zu erhalten: 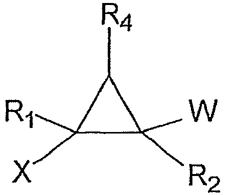 aus

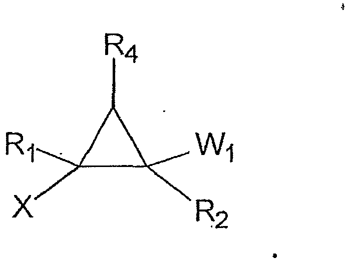

2. Verfahren nach Anspruch 1, bei dem der Katalysator ein Übergangsmetall enthält, wobei der Katalysator insbesondere ein Komplex aus Palladium (II), besonders bevorzugt Pd(OAc)₂ oder Pd(acac)₂, ein Komplex aus Kupfer (II), besonders bevorzugt CuSO₄, Cu(acac)₂, Cu(tBuSalen)₂, Cu(OTf)₂, Ein Komplex aus Kupfer (I), besonders bevorzugt CuI oder Cu(OTf), oder ein Komplex aus Rhodium (II), besonders bevorzugt Rh₂(OAc)₄, Rh₂(Octanoat)₄ oder Th₂(5S-MEPY)₄ (Doyle-Katalysator) ist.

3. Verfahren nach einem der Ansprüche 1 bis 2, bei dem W₁ -COOR^{a} darstellt, wobei R^{a} wie in Anspruch 1 definiert ist,
bei dem W insbesondere -COOR^{a}, CONH₂, -CONHR^{a},-CONR^{a}R^{b}, -CH₂OH, -CH₂OR^{a}, -CHR^{b}OH, -CHR^{b}OR^{a}, -CR^{b}R^{b'}OR^{a},-CH₂NH₂, -CH₂NHZ, -CHR^{a}NHZ darstellt, wobei das Verfahren umfasst:
* einen Reaktionsschritt zwischen:
einem Diazoderivat folgender Formel: 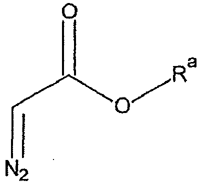 wobei R^{a} wie in Anspruch 1 definiert ist,
und
einer Vinyltrifluorboratverbindung folgender Formel: 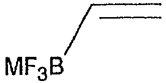 wobei M wie in Anspruch 1 definiert ist, wobei M insbesondere K darstellt,
im Beisein eines Katalysators, der ein Übergangsmetall enthält, um eine Verbindung folgender Formel zu erhalten: 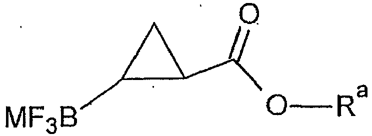
wobei R^{a} und M wie oben definiert sind,
wenn W anders als -COOR^{a} und/oder X anders als MF₃B ist, wobei das Verfahren ferner die folgenden Schritte umfasst:
* einen Schritt der Umwandlung von -COOR^{a} in W, die es ermöglicht, folgendes zu erhalten 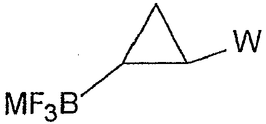 aus 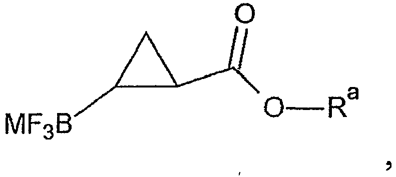 und
* einen Schritt der Umwandlung von -BF₃M in -X, der es ermöglicht, folgendes zu erhalten 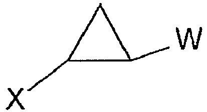 aus 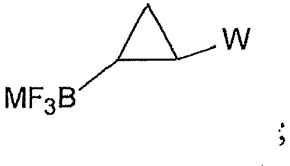 oder
* einen Schritt der Umwandlung von -BF₃M in -X, der es ermöglicht, folgendes zu erhalten 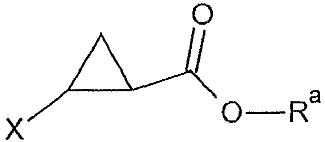 aus 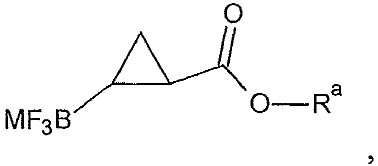 und
* einen Schritt der Umwandlung von -COOR^{a} in W, der es ermöglicht, folgendes zu erhalten 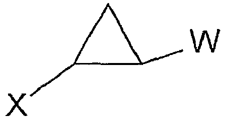 aus

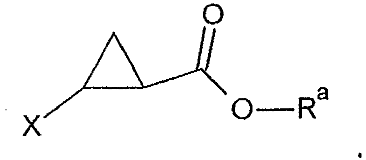

4. Verfahren nach einem der Ansprüche 1 bis 2, bei dem:
* R₂ in der Gruppe ausgewählt ist, die von den Gruppen -COR^{a}, -COOR^{a}, -CONH₂, -CONHR^{a}, -CONR^{a}R^{b}, -CN und -NO₂ gebildet ist,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} verbunden sein können, um einen eventuell substituierten Zyklus zu bilden,
* oder wobei R₂ H darstellt,
* oder wobei R₁, R₂ und R₄ H darstellen.
* oder wobei R₁ H darstellt,
* oder wobei R₁ H darstellt und R₄ ein Aryl, einen Heterozyklus, ein Heteroaryl oder ein Alkyl, wie in Anspruch 1 definiert, darstellt,
* oder wobei R₄ H darstellt,
* oder wobei R₄ H darstellt und R₁ ein Aryl, einen Heterozyklus, ein Heteroaryl oder ein Alkyl, wie in Anspruch 1 definiert, darstellt,
* oder wobei R₁ und R₂ H darstellen und R₄ ein Aryl, einen Heterozyklus, ein Heteroaryl oder ein Alkyl, wie in Anspruch 1 definiert, darstellt,
* oder wobei R₂ und R₄ H darstellen und R₄ ein Aryl, einen Heterozyklus, ein Heteroaryl oder ein Alkyl, wie in Anspruch 1 definiert, darstellt.

5. Verfahren nach einem der Ansprüche 1 bis 4, bei dem W eine funktionelle Gruppe darstellt, die ausgewählt ist unter -CONH-SO₂-Zyklopropyl, -CH₂-NH-CO-CH₂-CH₃ und der folgenden funktionellen Gruppe:

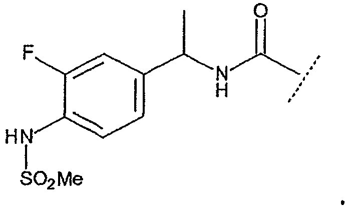

6. Verbindung, die folgender Formel (I-A) entspricht 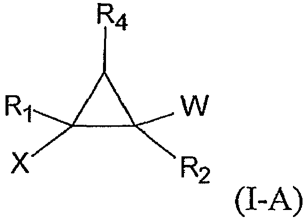 wobei:
* X ein substituiertes Boratom darstellt, das in der Gruppe ausgewählt ist, umfassend B(OH)₂, B(OR)₂, BF₃M, B(OR')₃M, wobei:
- R eine Alkylgruppe, umfassend 1 bis 14 Kohlenstoffatome, oder eine Arylgruppe, die eventuell substituiert sind, oder derart ist, das (OR)₂ einen Zyklus zwischen den beiden Sauerstoffatomen bildet, wobei (OR)₂ insbesondere in der Gruppe ausgewählt ist, umfassend die von Diolen abgeleiteten bivalenten Radikale, wie O-CH₂-CH₂-O, O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O und seine Ester, und die von den Disäuren abgeleiteten bivalenten Radikale, wie OCO-CH₂-N(CH₃)-CH₂-COO,
- R' eine Alkylgruppe, umfassend 1 bis 14 Kohlenstoffatome oder derart ist, dass:
* (OR')₃ einen Zyklus zwischen zwei Sauerstoffatomen bildet, wobei (OR')₃ nun in Form von OR'(OR)₂ ist, wobei R' eine Alkylgruppe, umfassend 1 bis 14 Kohlenstoffatome, und (OR)₂ wie oben definiert ist, oder
* (OR')₃ einen Bizyklus zwischen den drei Sauerstoffatomen bildet, wobei (OR')₃ insbesondere in der Gruppe ausgewählt ist, umfassend die von Triolen abgeleiteten trivalenten Radikale, wie H₃C-C-(CH₂-O)₃,
- M das Lithiumion Li⁺, das Natriumion Na⁺, das Kaliumion K⁺, das Cäsiumion Cs⁺, das Ammoniumion R^{c}R^{d}R^{e}R^{f}N⁺ darstellt, wobei R^{c}R^{d} R^{e} R^{f} unter H oder einer kohlenstoffhaltigen gesättigten Kette ausgewählt sind, umfassend insbesondere 1 bis 6 Kohlenstoffatome, die unabhängig voneinander ausgewählt sind,
und insbesondere X B(OH)₂, B(OR)₂ oder BF₃K darstellt,
* R₁ und R₄ identisch oder unterschiedlich sind und in der Gruppe ausgewählt'sind, die gebildet ist von:
1. H
2. Arylen, umfassend Zyklen von 6 bis 15 Kohlenstoffatomen, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale mit 1 bis 15 eventuell substituierten Kohlenstoffatomen,
- durch Alcenylradikale mit 1 bis 15 eventuell substituierten Kohlenstoffatomen,
- durch Alcynylradikale mit 1 bis 15 eventuell substituierten Kohlenstoffatomen,
- durch Aryle mit 6 bis 12 eventuell substituierten Kohlenstoffatomen,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N (C=NCOOR^{a}) NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte, lineare oder verzweigte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Aryle mit 6 bis 12 eventuell substituierten Kohlenstoffatomen,
- durch aromatische oder nicht-aromatische Heterozyklen mit 2 bis 12 eventuell substituierten Kohlenstoffatomen,
3. Heterozyklen oder Heteroarylen, umfassend Zyklen von 2 bis 15 Kohlenstoffatomen, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N (C=NCOOR^{a}) NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N (C=NCOOR^{a}) NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
4. linearen oder verzweigten Alcenylen, umfassend 1 bis 12 eventuell substituierte Kohlenstoffatome oder kohlenstoffhaltige Zyklen, umfassend 3 bis 12 Kohlenstoffatome oder eine oder mehrere Doppelverbindungen C=C, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N (C=NCOOR^{a}) NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
5. linearen oder verzweigten Alcynylen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N (C=NCOOR^{a}) NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
6. linearen, zyklischen oder verzweigten Alkylgruppen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N (C=NCOOR^{a}) NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N (C=NCOOR^{a}) NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei mindestens eine der Gruppen R₁ und R₄ H darstellt,
* R₂ in der Gruppe ausgewählt ist, die von den Gruppen gebildet ist, die durch R₁ oder R₄ dargestellt werden können, sowie -COR^{a}, -COOR^{a}, -CONH₂, -CONHR^{a}, -CONR^{a}R^{b}, -CN und NO₂,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} verbunden sein können, um einen eventuell substituierten Zyklus zu bilden,
* W eine funktionelle Gruppe darstellt, die ausgewählt ist unter -CHO, -COR^{a}, -COOH, -COOR^{a}, CONH₂, -CONHR^{a},-CONR^{a}R^{b}, -COHNH-SO₂R^{a}, -CH₂OH, -CH₂OR^{a}, -CHR^{b}OH,-CHR^{b}OR^{a}, -CR^{b}R^{b'}OR^{a}, -CH₂NHZ, -CHR^{a}NHZ, -CH₂-NH-COR^{a}, wobei Z eine Schutzgruppe einer Aminfunktion darstellt, und
wobei R^{a}, R^{b} und R^{b'} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a}) NHCOOR^{b}, wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
vorbehaltlich dessen, dass:
* wenn R₁, R₂ und R₄ H darstellen und B B(OH)₂ oder B(OR')₂ oder B(OR')₃M darstellt, W unter -COR^{a}, CONH₂, -CONHR^{a}, -CONR^{a}R^{b} ausgewählt ist,
* wenn R₁, R₂ und R₄ H darstellen und BF₃M darstellt, W unter -COOH, COOR^{a}, -COR^{a}, CONH₂, -CONHR^{a}, -CONR^{a}R^{b} ausgewählt ist,
* wenn W -CH₂OH, -CH₂OR^{a}, -CHR^{b}OH, -CHR^{b}OR^{a}, -CR^{b}R^{b'}OH oder -CR^{b}R^{b'}OR^{a} darstellt und B B(OH)₂ oder B(OR')₂ oder B(OR')₃M darstellt:
- stellt R₁ kein H dar, oder
- stellt R₂ und R₄ kein H dar,
* wenn W -CH₂OH, -CH₂OR^{a}, -CHR^{b}OH, -CHR^{b}OR^{a}, -CR^{b}R^{b'}OH oder -CR^{b}R^{b'}OR^{a} darstellt und B BF₃M darstellt, stellen R₁ oder R₂ kein H dar,
* wenn W -COOH oder -COOR^{a} darstellt und B B(OH)₂ oder B(OR')₂ oder B(OR')₃M darstellt:
- stellt R₁ kein H dar, oder
- stellen R₂ und R₄ kein H dar.

7. Verbindung nach Anspruch 6, bei der W eine funktionelle Gruppe darstellt, die ausgewählt ist unter -CHO, -COR^{a}, -COOH, -COOR^{a}, CONH₂, -CONHR^{a},-CONR^{a}R^{b}, -CH₂OH, -CH₂OR^{a}, -CHR^{b}OH, -CHR^{b}OR^{a}, -CR^{b}R^{b'}OH-CR^{b}R^{b'}OR^{a}, -CH₂NH₂, -CHR^{a}NHZ, -CHR^{a}NHZ,
* wobei W insbesondere eine funktionelle Gruppe darstellt, die ausgewählt ist unter -CHO, -COR^{a},-COOH, -COOR^{a}, CONH₂, -CONHR^{a}, -CONR^{a}R^{b}, -CH₂OH, -CH₂OR^{a}, -CHR^{b}OH, -CHR^{b}OR^{a}, -CR^{b}R^{b'}OR^{a}, -CR^{b}R^{b'}OR^{a},
* wobei W insbesondere eine funktionelle Gruppe darstellt, die ausgewählt ist unter -CHO, -COR^{a},-COOH, -COOR^{a}, CONH₂, -CONHR^{a}, -C*ONR^{a}R^{b},
* wobei W insbesondere eine funktionelle Gruppe darstellt, die ausgewählt ist unter -CONH-SO₂-Zyklopropyl, -CH₂-NH-CO-CH₂-CH₃ und der Gruppe der folgenden Formel: 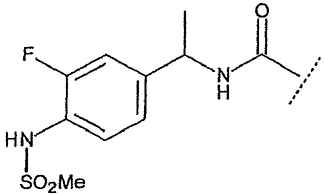
* wobei R₂ insbesondere H darstellt,
* wobei R₂ insbesondere in der Gruppe ausgewählt ist, die von den Gruppen -COR^{a}, -COOR^{a}, CONH₂, -CONHR^{a}, -CONR^{a}R^{b}, -CN und -NO₂ gebildet ist,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} verbunden sein können, um einen eventuell substituierten Zyklus zu bilden,
* wobei insbesondere R₁ und R₄ H, ein Aryl, einen Heterozyklus, ein Heteroaryl oder ein Alkyl, wie in Anspruch 6 definiert, darstellen,
* wobei insbesondere R₁, R₂ und R₄ H darstellen,
* wobei R₁ H darstellt,
* wobei R₁ H darstellt und R₄ ein Aryl, einen Heterozyklus, ein Heteroaryl oder ein Alkyl, wie in Anspruch 6 definiert, darstellt,
* wobei insbesondere R₄ H darstellt,
* wobei R₄ H darstellt und R₁ ein Aryl, einen Heterozyklus, ein Heteroaryl oder ein Alkyl, wie in Anspruch 6 definiert, darstellt,
* wobei R₁ und R₂ H darstellen und R₄ ein Aryl, einen Heterozyklus, ein Heteroaryl oder ein Alkyl, wie in Anspruch 6 definiert, darstellt,
* wobei R₂ und R₄ H darstellen und R₁ ein Aryl, einen Heterozyklus, ein Heteroaryl oder ein Alkyl, wie in Anspruch 6 definiert, darstellt.

8. Verbindung nach einem der Ansprüche 6 bis 7, wobei die Verbindung in der Gruppe ausgewählt ist, die von den Verbindungen folgender Formel und ihrem Enantiomer gebildet ist:

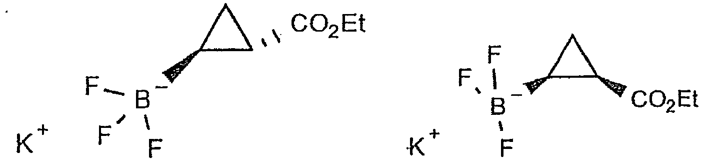

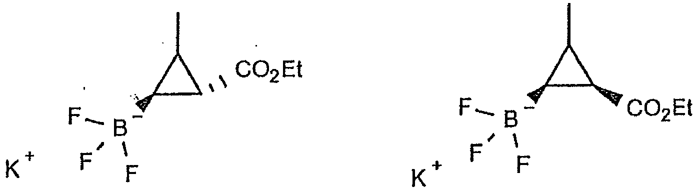

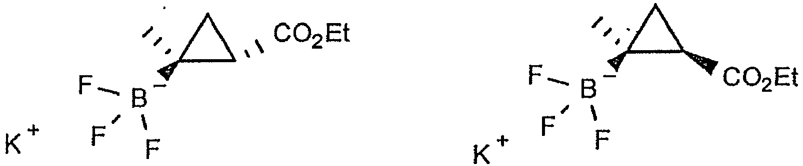

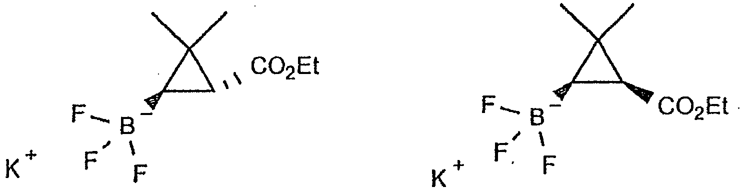

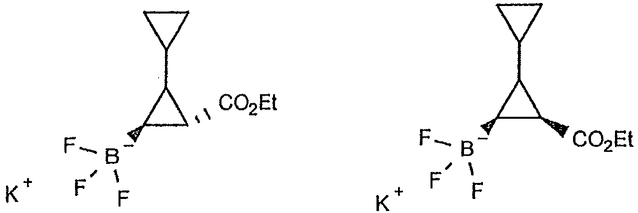

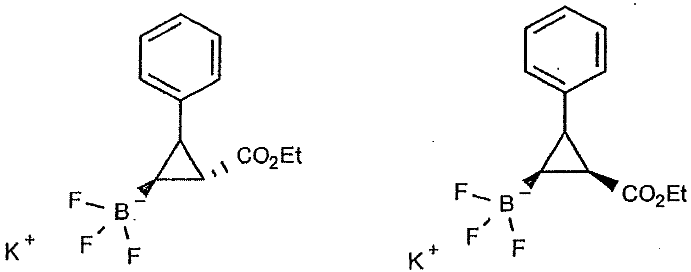

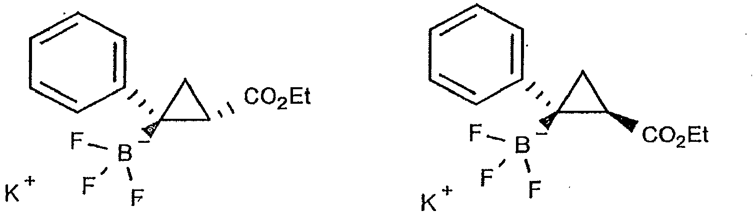

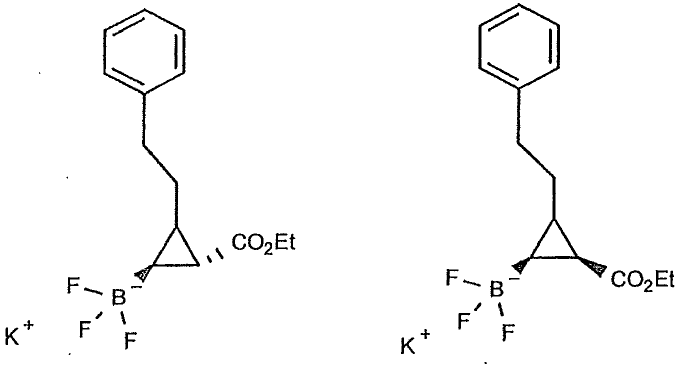

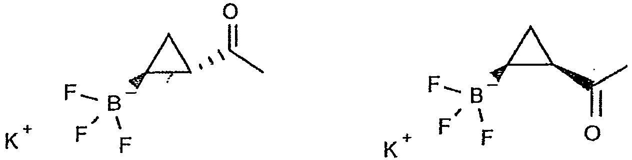

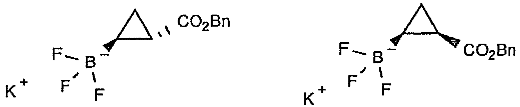

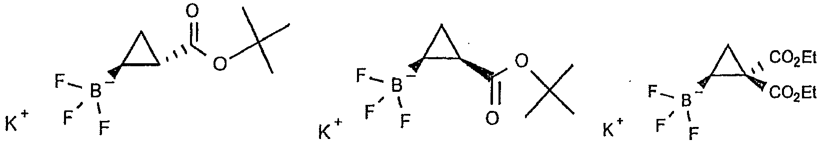

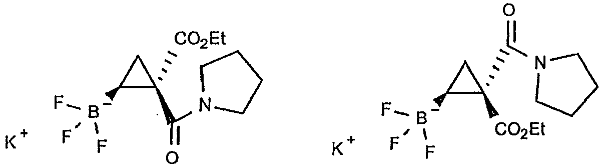

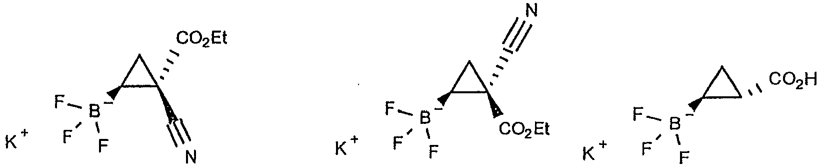

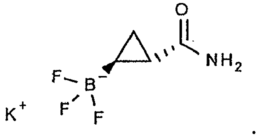

9. Verwendung einer Verbindung der Formel (I-A) nach Anspruch 6 zur Herstellung von Verbindungen mit folgender Formel (II-A): 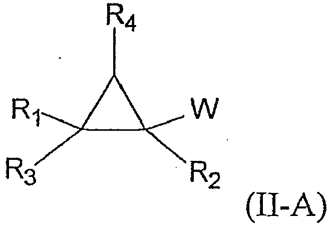 wobei:
* R₁, R₃ und R₄ identisch oder unterschiedlich sind und in der Gruppe ausgewählt sind, die gebildet ist:
1. von H, vorbehaltlich dessen, dass R₃ nicht H darstellt,
2. von den Arylen, umfassend Zyklen von 6 bis 15 Kohlenstoffatomen, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b}, - N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale, umfassend 1 bis 15 eventuell substituierte Kohlenstoffatome,
- durch Alcenylradikale, umfassend 1 bis 15 eventuell substituierte Kohlenstoffatome,
- durch Alcynylradikale, umfassend 1 bis 15 eventuell substituierte Kohlenstoffatome,
- durch Arylradikale, umfassend mit 6 bis 12 eventuell substituierte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte, lineare oder verzweigte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Aryle, umfassend 6 bis 12 eventuell substituierte Kohlenstoffatome,
- durch aromatische oder nicht-aromatische Heterozyklen mit 2 bis 12 eventuell substituierten Kohlenstoffatomen,
3. von den Heterozyklen oder den Heteroarylen, umfassend Zyklen mit 2 bis 15 Kohlenstoffatomen, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind;
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
4. linearen oder verzweigten Alcenylen, umfassend 1 bis 12 eventuell substituierte Kohlenstoffatome oder kohlenstoffhaltige Zyklen, umfassend 3 bis 12 Kohlenstoffatome oder eine oder mehrere Doppelverbindungen C=C, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a}) NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
5. linearen oder verzweigten Alcynylen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
6. linearen, zyklischen oder verzweigten Alkylgruppen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend mit 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a}, - OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N (C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei mindestens eine der Gruppen R₁ und R₄ H darstellt,
* R₂ in der Gruppe ausgewählt ist, die von den Gruppen gebildet ist, die durch R₁ oder R₄ dargestellt werden können, sowie -COR^{a}, -COOR^{a}, -CONH₂, -CONHR^{a}, -CONR^{a}R^{b}, -CN und NO₂,
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
wobei R^{a} und R^{b} verbunden sein können, um einen eventuell substituierten Zyklus zu bilden,
* W eine funktionelle Gruppe darstellt, die ausgewählt ist unter -CHO, -COR^{a}, -COOH, -COOR^{a}, CONH₂, -CONHR^{a},-CONR^{a}R^{b}, -COHNH-SO₂R^{a}, -CH₂OH, -CH₂OR^{a}, -CHR^{b}OH,-CHR^{b}OR^{a}, -CR^{b}R^{b'}OR^{a}, -CH₂NHZ, -CHR^{a}NHZ, -CH₂-NH-COR^{a}, wobei Z eine Schutzgruppe einer Aminfunktion darstellt, und
wobei R^{a}, R^{b} und R^{b'} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome, eventuell substituiert:
- durch 1 oder mehrere Halogenatome, umfassend Fluor, Chlor, Brom oder Jod,
- durch Hydroxy-, Amino- oder Thio-Radikale, die eventuell durch "ad hoc"-Schutzgruppen geschützt sind,
- durch Radikale -OR^{a}, -NHR^{a}, -NR^{a}R^{b}, -SR^{a}, -OCOR^{a},-OCONHR^{a}, -OCONR^{a}R^{b}, -CHO, -COR^{a}, -COOH, -CN, -COOR^{a},-CONHR^{a}, -CONR^{a}R^{b}, -CF₃, -NO₂, -N=C-NHR^{a}, -N=C-NR^{a}R^{b},-N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b},
wobei R^{a} und R^{b} identisch oder unterschiedlich sind und lineare, zyklische oder verzweigte, aromatische oder heterozyklische aromatische oder nicht-aromatische Alkyl-, Alcenyl-, Alcynylgruppierungen darstellen, umfassend 1 bis 15 Kohlenstoffatome,
- durch Alkylradikale, umfassend 1 bis 15 eventuell substituierte Kohlenstoffatome,
- durch Alcenylradikale, umfassend 1 bis 15 eventuell substituierte, lineare oder verzweigte Kohlenstoffatome,
- durch Alcynylradikale, umfassend 1 bis 15 eventuell substituierte, lineare oder verzweigte Kohlenstoffatome,
- durch Arylradikale, umfassend 6 bis 12 eventuell substituierte, lineare oder verzweigte Kohlenstoffatome,
- durch aromatische oder nicht-aromatische Heterozyklen mit 2 bis 12 eventuell substituierten Kohlenstoffatomen,
vorbehaltlich dessen, dass:
* wenn R₁, R₂ und R₄ H darstellen und B B(OH)₂ oder B(OR')₂ oder B(OR')₃M darstellt, W unter -COR^{a}, CONH₂, -CONHR^{a}, -CONR^{a}R^{b} ausgewählt ist,
* wenn R₁, R₂ und R₄ H darstellen und BF₃M darstellt, W unter -COOH, COOR^{a}, -COR^{a}, CONH₂, -CONHR^{a}, -CONR^{a}R^{b} ausgewählt ist,
* wenn W -CH₂OH, -CH₂OR^{a}, -CHR^{b}OH, -CHR^{b}OR^{a}, -CR^{b}R^{b'}OH oder -CR^{b}R^{b'}OR^{a} darstellt und B B(OH)₂ oder B(OR')₂ oder B(OR')₃M darstellt:
- stellt R₁ kein H dar, oder
- stellt R₂ und R₄ kein H dar,
* wenn W -CH₂OH, -CH₂OR^{a}, -CHR^{b}OH, -CHR^{b}OR^{a}, -CR^{b}R^{b'}OH oder -CR^{b}R^{b'}OR^{a} darstellt und B BF₃M darstellt, stellen R₁ oder R₂ kein H dar,
* wenn W -COOH oder -COOR^{a} darstellt und B B(OH)₂ oder B(OR')₂ oder B(OR')₃M darstellt:
- stellt R₁ kein H dar, oder
- stellen R₂ und R₄ kein H dar,
durch Reaktion der Verbindung der Formel (I) mit einer Verbindung mit der folgenden Formel (III)
R₃ - X₂,
wobei R₃ wie oben definiert ist, und X₂ in der Gruppe ausgewählt ist, die von den Halogeniden, insbesondere Jod, Brom und Chlor, und Triflat (OTf) gebildet ist, im Beisein eines Katalysators, der ein Übergangsmetall und eventuell einen Liganden enthält.

10. Verwendung nach Anspruch 9, wobei R₃ eine Gruppe darstellt, die ausgewählt ist unter:

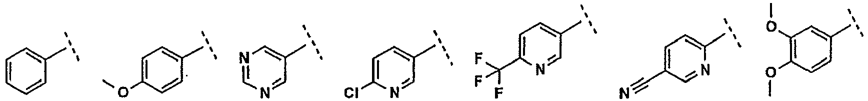

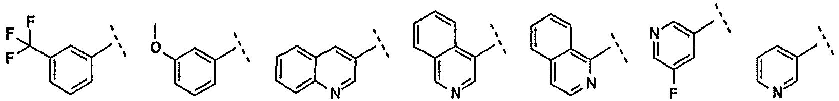

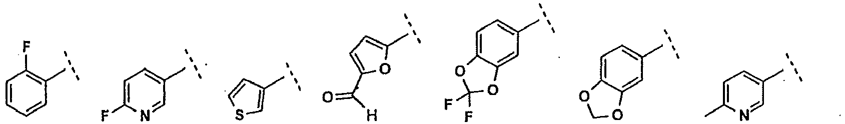

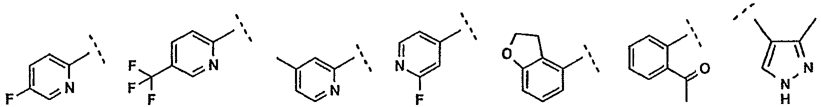

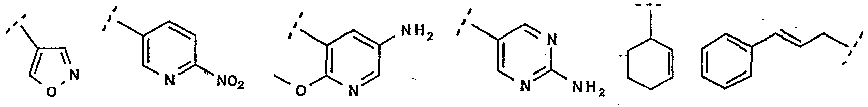

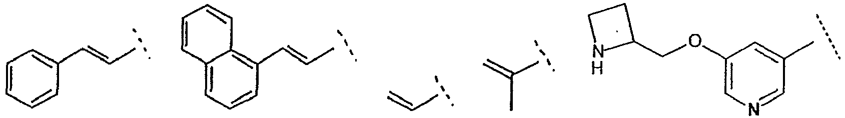

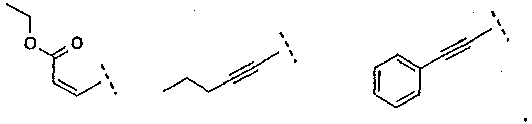

## Claims

1. Process for the preparation of a compound corresponding to the following formula (I-A) 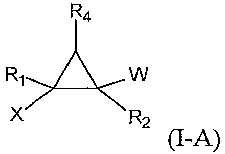 in which:
▪ X represents a substituted boron atom chosen from the group comprising B(OH)₂, B(OR)₂, BF₃M, B(OR')₃M in which:
∘ R is an alkyl group comprising 1 to 14 carbon atoms or an aryl group, optionally substituted, or is such that (OR)₂ forms a ring between the two oxygen atoms, (OR)₂ being in particular chosen from the group comprising the bivalent radicals deriving from diols, such as O-CH₂-CH₂-O, O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O and its esters, and the bivalent radicals deriving from diacids, such as OCO-CH₂-N(CH₃)-CH₂-COO,
∘ R' is an alkyl group comprising 1 to 14 carbon atoms or is such that:
• (OR')₃ forms a ring between two of the oxygen atoms, (OR')₃ then being in the form OR'(OR)₂, where R' is an alkyl group comprising 1 to 14 carbon atoms and (OR)₂ is as defined above, or
• (OR')₃ forms a bicycle between the three oxygen atoms, (OR')₃ being in particular chosen from the group comprising the trivalent radicals deriving from triols, such as H₃C-C-(CH₂-O)₃,
∘ M represents the lithium Li⁺ ion, the sodium Na⁺ ion, the potassium K⁺ ion, the caesium Cs⁺ ion, the ammonium R^{c}R^{d}R^{e}R^{f}N⁺ ion where R^{c}, R^{d} R^{e}, R^{f} are chosen from H or a saturated carbon-containing chain comprising in particular 1 to 6 carbon atoms chosen independently of one another,
and in particular X represents B(OH)₂, B(OR)₂ or BF₃K,
▪ R₁ and R₄, identical or different, are chosen from the group constituted by:
1. H
2. aryls comprising rings with 6 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals with 1 to 15 carbon atoms, optionally substituted,
∘ alkenyl radicals with 1 to 15 carbon atoms, optionally substituted,
∘ alkynyl radicals with 1 to 15 carbon atoms, optionally substituted,
∘ aryls with 6 to 12 carbon atoms, optionally substituted,
∘ aromatic or non-aromatic heterocycles with 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a,} -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, linear or branched optionally substituted,
∘ linear or branched alkenyl radicals comprising 1 to 15 carbon atoms, optionally substituted,
∘ alkynyl radicals comprising 1 to 15 carbon atoms, linear or branched optionally substituted,
∘ aryls with 6 to 12 carbon atoms, optionally substituted,
∘ by the aromatic or non-aromatic heterocycles comprising 2 to 12 carbon atoms, optionally substituted,
3. heterocycles or heteroaryls comprising rings with 2 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyls, alkenyl, alkynyls groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
4. linear or branched alkenyls comprising 1 to 12 carbon atoms, optionally substituted, or carbon rings comprising 3 to 12 carbon atoms and one or more C=C double bonds, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic,
or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
5. linear or branched alkynyls comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic,
or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
6. linear, cyclic or branched alkyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
at least one of the R₁ and R₄ groups representing H,
▪ R₂ is chosen from the group constituted by the groups being able to be represented by R₁ or R₄, as well as -COR^{a}, -COOR^{a}, -CONH₂, -CONHR^{a}, -CONR^{a}R^{b}, -CN and -NO₂,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
R^{a} and R^{b} being able to be linked in order to form a ring, optionally substituted,
▪ W represents a functional group chosen from -CHO, -COR^{a}, -COOH, -COOR^{a}, -CONH₂, -CONHR^{a}, -CONR^{a}R^{b}, -CONH-SO₂-R^{a}, -CH₂OH, -CH₂OR^{a}, -CHR^{b}OH, -CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a,}
in which Z represents a protective group of an amine function, and
in which R^{a}, R^{b} and R^{b'}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
said process comprising:
• a step of reaction between:
▪ a diazoic derivative of the following formula: 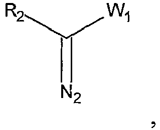 in which R₂ is as defined above, W₁ being chosen from the group constituted by -COR^{a}, -COOR^{a}, -CONH₂, -CONHR^{a}, and -CONR^{a}R^{b}, and
▪ a vinyltrifluoroborate compound of the following formula: 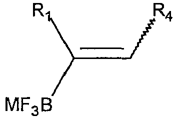 in which R₁, R₄ and M are as defined above,
in the presence of a catalyst containing a transition metal, in order to obtain a compound of the following formula: 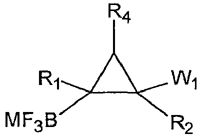 in which R₁, R₂, R₄, W₁ and M are as defined above,
if W is different from W₁ and/or X is different from MF₃B, said process also comprising the following steps:
• a step of conversion of W₁ to W making it possible to obtain 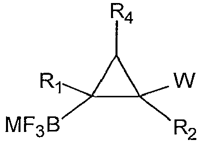 from 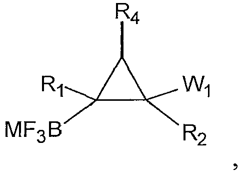 in particular, when W₁ = COOR^{a} and W = CHO, by reduction in order to form the corresponding alcohol, then oxidation of said alcohol, when W₁ = -COOR^{a} and W = CH₂OH or -CH₂OR^{b}, by the formation of an aldehyde as described previously, then by reduction of said aldehyde and optional alkylation, when W₁ = -COR^{a} and W = -CHR^{a}OH, -CHR^{a}OR^{b}, by reduction then optional alkylation of the obtained alcohol, when W₁ = -COR^{a} and W = CR^{a}R^{b}OH or -CR^{a}R^{b}OR^{b'} by addition of a Grignard reagent then optional alkylation of the obtained alcohol, when W₁ = -CONH₂, -CONHR^{a} or -CONR^{a}R^{b} and W = -CH₂NH₂, -CH₂NHR^{a}, -CH₂NR^{a}R^{b}, -CH₂NHZ or -CH₂-NH-COR^{a}, by reduction then optional protection by Z of the obtained amine or optional reaction with the acid chloride R^{a}COCl, when W₁ = -CONH₂ and W = -CONHSO₂R^{a}, by the action of sulphonyl chloride ClSO₂R^{a} on the amide,
and
• a step of conversion of -BF₃M to -X making it possible to obtain 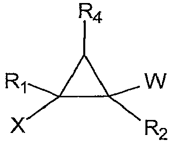 from 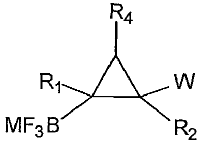 in particular, when X = B(OH)₂, by basic or acid hydrolysis, or by passing via a dihalogenoborane, more particularly a dichloroborane, when X = B(OR)₂, by passing via X = B(OH)₂ as described previously then by the action of an alcohol, in particular an alcohol of formula ROH, a diol or a triol, or by passing via a dihalogenoborane, more particularly a dichloroborane, then by the action of an alcohol, in particular an alcohol of formula ROH, a diol or a triol,
or
• a step of conversion of -BF₃M to -X making it possible to obtain 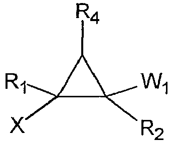 from 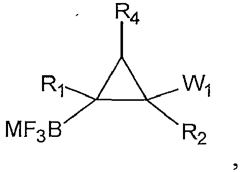 and
• a step of conversion of W₁ to W making it possible to obtain 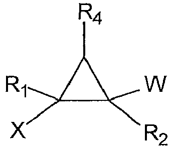 from

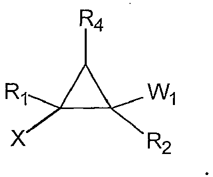

2. Process according to claim 1, in which said catalyst containing a transition metal, said catalyst being in particular a palladium (II) complex, more particularly Pd(OAc)₂ or Pd(acac)₂, a copper (II) complex, more particularly CuSO₄, Cu(acac)₂, Cu(tBuSalen)₂, Cu(OTf)₂, a copper (I) complex, more particularly CuI or Cu(OTf), or a rhodium (II) complex, more particularly Rh₂(OAc)₄, Rh₂(Octanoate)₄ or Rh₂(5S-MEPY)₄ (Doyle catalyst).

3. Process according to any one of claims 1 to 2, in which W₁ represents -COOR^{a}, R^{a} being as defined in claim 1, in particular in which W represents -COOR^{a}, -CONH₂, -CONHR^{a}, -CONR^{a}R^{b}, -CH₂OH, -CH₂OR^{a}, -CHR^{b}OH, -CHR^{b}OR^{a}, -CR^{b}R^{b'}OH, -CR^{b}R^{b'}OR^{a}, -CH₂NH₂, -CH₂NHZ, -CHR^{a}NHZ, said process comprising:
• a step of reaction between:
a diazoic derivative of the following formula: 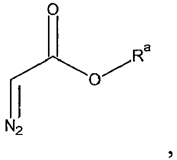 in which R^{a} is as defined in claim 1,
and
a vinyltrifluoroborate compound of the following formula: 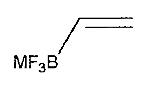 in which M is as defined in claim 1, M representing in particular K,
in the presence of a catalyst containing a transition metal, in order to obtain a compound of the following formula: 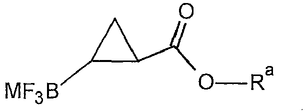 in which R^{a} and M are as defined above,
if W is different from -COOR^{a} and/or X is different from MF₃B, said process also comprising the following steps:
• a step of conversion of -COOR^{a} to W making it possible to obtain 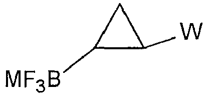 from 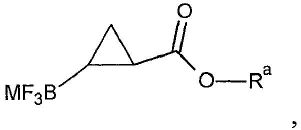 and
• a step of conversion of -BF₃M to -X making it possible to obtain 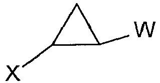 from 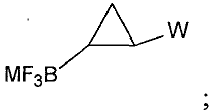 or
• a step of conversion of -BF₃M to -X making it possible to obtain 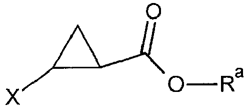 from 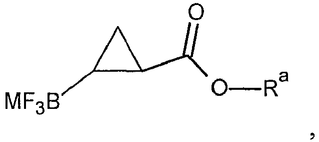 and
• a step of conversion of -COOR^{a} to W making it possible to obtain 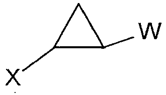 from

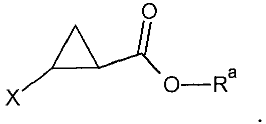

4. Process according to any one of claims 1 to 2, in which:
❖ R₂ is chosen from the group constituted by the -COR^{a}, -COOR^{a}, -CONH₂, -CONHR^{a}, -CONR^{a}R^{b}, -CN and -NO₂ groups, in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
R^{a} and R^{b} being able to be linked in order to form a ring, optionally substituted,
❖ or in which R₂ represents H,
❖ or in which R₁, R₂ and R₄ represent H,
❖ or in which R₁ represents H,
❖ or in which R₁ represents H and R₄ represents an aryl, a heterocycle, a heteroaryl or an alkyl as defined in claim 1,
❖ or in which R₄ represents H,
❖ or in which R₄ represents H and R₁ represents an aryl, a heterocycle, a heteroaryl or an alkyl as defined in claim 1,
❖ or in which R₁ and R₂ represent H and R₄ represents an aryl, a heterocycle, a heteroaryl or an alkyl as defined in claim 1,
❖ or in which R₂ and R₄ represent H and R₁ represents an aryl, a heterocycle, a heteroaryl or an alkyl as defined in claim 1.

5. Process according to any one of claims 1 to 4, in which W represents a functional group chosen from -CONH-SO₂-cyclopropyl, -CH₂-NH-CO-CH₂-CH₃, and the group of the following formula:

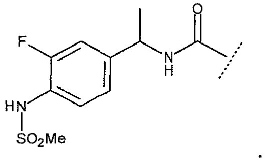

6. Compound corresponding to the following formula (I-A) 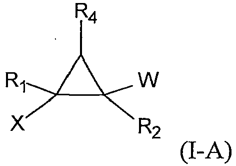 in which:
▪ X represents a substituted boron atom chosen from the group comprising B(OH)₂, B(OR)₂, BF₃M, B(OR')₃M in which:
∘ R is an alkyl group comprising 1 to 14 carbon atoms, an aryl group, optionally substituted, or is such that (OR)₂ forms a ring between the two oxygen atoms, (OR)₂ being in particular chosen from the group comprising the bivalent radicals deriving from diols, such as O-CH₂-CH₂- O-CH₂-CH₂-CH₂-O, O-CH₂-C(CH₃)₂-CH₂-O, O-C(CH₃)₂-CH₂-CH₂-C(CH₃)₂-O, O-CH(CH₃)-CH₂-CH₂-CH(CH₃)-O, O-CH(Ph)-CH(Ph)-O, O-CH(CH₃)-CH₂-C(CH₃)₂-O, O-*o*-Ph-O, O-CH₂-CH₂-NH-CH₂-CH₂-O, O-CH₂-CH₂-N(CH₂-CH₂-CH₂-CH₃)-CH₂-CH₂-O, O-CH(COOH)-CH(COOH)-O and its esters, and the bivalent radicals deriving from diacids, such as OCO-CH₂-N(CH₃)-CH₂-COO,
∘ R' is an alkyl group comprising 1 to 14 carbon atoms or is such that:
• (OR')₃ forms a ring between two of the oxygen atoms, (OR')₃ then being in the form OR'(OR)₂, where R' is an alkyl group comprising 1 to 14 carbon atoms and (OR)₂ is as defined above, or
• (OR')₃ forms a bicycle between the three oxygen atoms, (OR')₃ being in particular chosen from the group comprising the trivalent radicals deriving from triols, such as H₃C-C-(CH₂-O)₃,
∘ M represents the lithium Li⁺ ion, the sodium Na⁺ ion, the potassium K⁺ ion, the caesium Cs⁺ ion, the ammonium R^{c}R^{d}R^{e}R^{f}N⁺ ion where R^{c}, R^{d} R^{e}, R^{f} are chosen from H or a saturated carbon-containing chain comprising in particular 1 to 6 carbon atoms chosen independently of one another,
and in particular X represents B(OH)₂, B(OR)₂ or BF₃K,
R₁ and R₄, identical or different, are chosen from the group constituted by:
1. H
2. aryls comprising rings with 6 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals with 1 to 15 carbon atoms, optionally substituted,
∘ alkenyl radicals with 1 to 15 carbon atoms, optionally substituted,
∘ alkynyl radicals with 1 to 15 carbon atoms, optionally substituted,
∘ aryls with 6 to 12 carbon atoms, optionally substituted,
∘ aromatic or non-aromatic heterocycles with 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ linear or branched alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ aryls with 6 to 12 carbon atoms, optionally substituted,
∘ aromatic or non-aromatic heterocycles comprising 2 to 12 carbon atoms, optionally substituted,
3. heterocycles or heteroaryls comprising rings with 2 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, =NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
4. linear or branched alkenyls comprising 1 to 12 carbon atoms, optionally substituted, or carbon rings comprising 3 to 12 carbon atoms and one or more C=C double bonds, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic,
or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
5. linear or branched alkynyls comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic,
or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
6. linear, cyclic or branched alkyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
at least one of the R₁ and R₄ groups representing H,
▪ R₂ is chosen from the group constituted by the groups being able to be represented by R₁ or R₄, as well as -COR^{a}, -COOR^{a}, -CONH₂, -CONHR^{a}, -CONR^{a}R^{b}, -CN and -NO₂,
in which R^{a} and R^{b}, identical or different, represent linear, cyclic or branched, aromatic, or aromatic or non-aromatic, heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
R^{a} and R^{b} being able to be linked in order to form a ring, optionally substituted,
▪ W represents a functional group chosen from -CHO, -COR^{a}, -COOH, -COOR^{a}, -CONH₂, -CONHR^{a}, -CONR^{a}R^{b}, -CONH-SO₂-R^{a}, -CH₂OH, -CH₂OR^{a}, -CHR^{b}OH, -CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a,} in which Z represents a protective group of an amine function, and
in which R^{a}, R^{b} and R^{b'}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ by the aromatic or non-aromatic heterocycles comprising 2 to 12 carbon atoms, optionally substituted,
provided that:
▪ when R₁, R₂ and R₄ represent H and B represents B(OH)₂, B(OR)₂, or B(OR')₃M, then W is chosen from -COR^{a}, -CONH₂, -CONHR^{a}, -CON_{R}^{a}_{R}^{b},
▪ when R₁, R₂ and R₄ represent H and B represents BF₃M, then W is chosen from -COOH, -COOR^{a}, -CHO, -COR^{a}, -CONH₂, -CONHR^{a}, -CONR^{a}R^{b},
▪ when W represents CH₂OH, -CH₂OR^{a}, -CHR^{b}OH, -CHR^{b}OR^{a}, -CR^{b}R^{b'}OH, or -CR^{b}R^{b'}OR^{a}, and B represents B(OH)₂, B(OR)₂, or B(OR')₃M, then:
∘ R₁ does not represent H, or
∘ R₂ and R₄ do not represent H,
▪ when W represents CH₂OH, -CH₂OR^{a}, -CHR^{b}OH, -CHR^{b}OR^{a}, -CR^{b}R^{b'}OH, or -CR^{b}R^{b'}OR^{a}, and B represents BF₃M, then R₁ or R₂ does not represent H.
▪ when W represents -COOH or -COOR^{a}, and B represents B(OH)₂, B(OR)₂, or B(OR')₃M, then:
∘ R₁ does not represent H, or
∘ R₂ and R₄ do not represent H.

7. Compound according to claim 6, in which W represents a functional group chosen from -CHO, -COR^{a}, -COOH, -COOR^{a}, -CONH₂, -CONHR^{a}, -CONR^{a}R^{b}, -CH₂OH, -CH₂OR^{a}, -CHR^{b}OH, -CHR^{b}OR^{a}, -CR^{b}R^{b'}OH, -CR^{b}R^{b'}OR^{a}, -CH₂NH₂, -CH₂NHZ, -CHR^{a}NHZ,
❖ in particular in which W represents a functional group chosen from -CHO, -COR^{a}, -COOH, -COOR^{a}, -CONH₂, -CONHR^{a}, -CONR^{a}R^{b}, -CH₂OH, -CH₂OR^{a}, -CHR^{b}OH, -CHR^{b}OR^{a}, -CR^{b}R^{b'}OH, -CR^{b}R^{b'}OR^{a},
∘ more particularly in which W represents a functional group chosen from -CHO, -COR^{a}, -COOH, -COOR^{a}, -CONH₂, -CONHR^{a}, -CONR^{a}R^{b}.
❖ in particular in which W represents a functional group chosen from -CONH-SO₂-cyclopropyl, -CH₂-NH-CO-CH₂-CH₃^{,} and the group of the following formula: 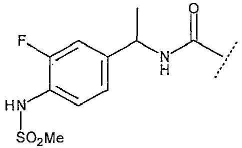
❖ in particular in which R₂ represents H,
❖ in particular in which R₂ is chosen from the group constituted by the -COR^{a}, -COOR^{a}, -CONH₂, -CONHR^{a}, -CONR^{a}R^{b}, -CN and -NO₂ groups,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
R^{a} and R^{b} being able to be linked in order to form a ring, optionally substituted,
❖ in particular in which R₁ and R₄ represent H, an aryl, a heterocycle, a heteroaryl or an alkyl as defined in claim 6,
❖ in particular in which R₁, R₂ and R₄ represent H,
❖ in particular in which R₁ represents H,
❖ in particular in which R₁ represents H and R₄ represents an aryl, a heterocycle, a heteroaryl or an alkyl as defined in claim 6,
❖ in particular in which R₄ represents H,
❖ in particular in which R₄ represents H and R₁ represents an aryl, a heterocycle, a heteroaryl or an alkyl as defined in claim 6,
❖ in particular in which R₁ and R₂ represent H and R₄ represents an aryl, a heterocycle, a heteroaryl or an alkyl as defined in claim 6,
❖ in particular in which R₂ and R₄ represent H and R₁ represents an aryl, a heterocycle, a heteroaryl or an alkyl as defined in claim 6.

8. Compound according to any one of claims 6 to 7, said compound being chosen from the group constituted by the compounds of the following formulae and their enantiomers:

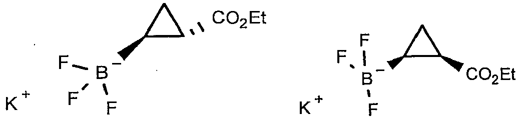

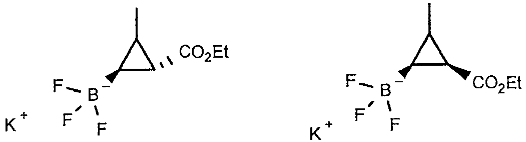

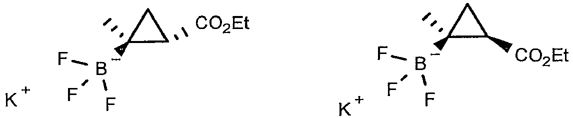

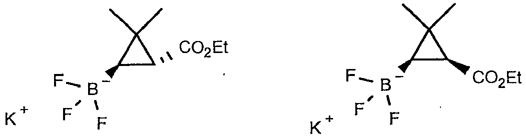

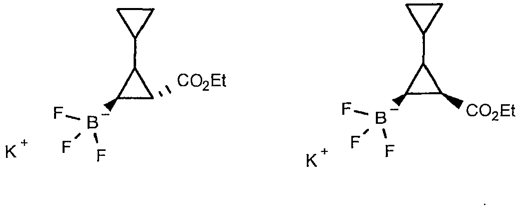

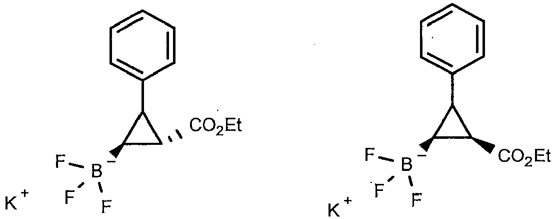

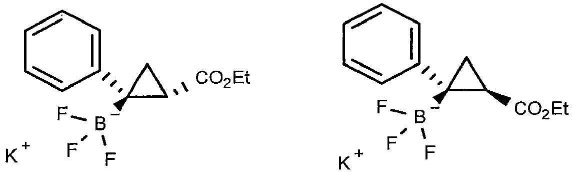

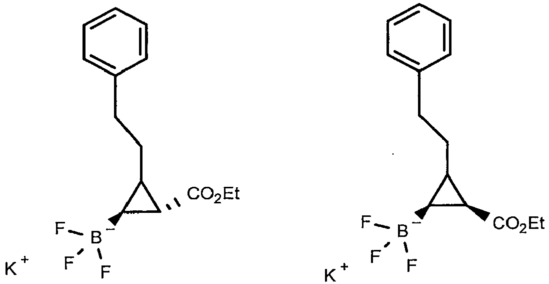

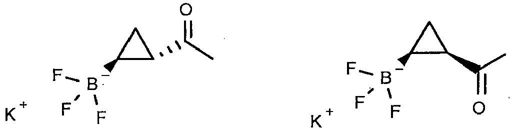

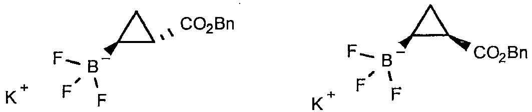

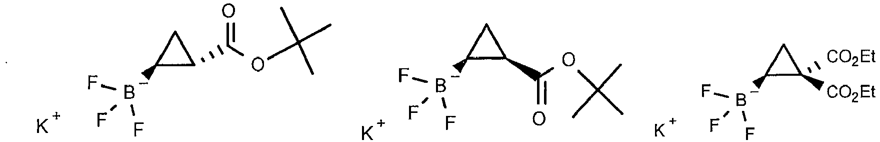

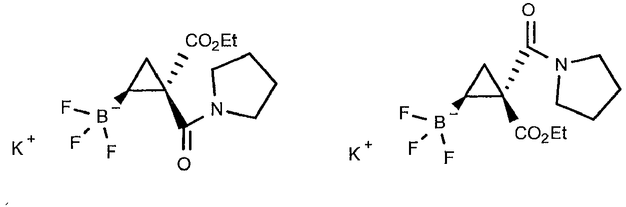

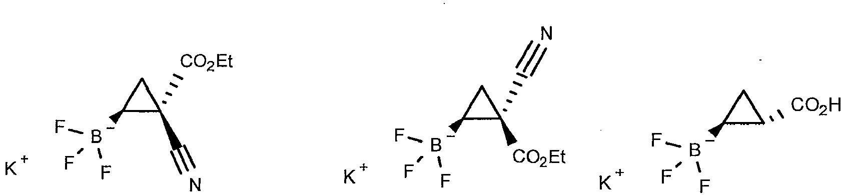

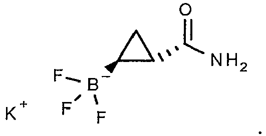

9. Use of a compound of formula (I-A) according to claim 6, for the preparation of compounds of the following formula (II-A): 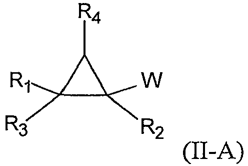 in which:
▪ R₁, R₃ and R₄, identical or different, are chosen from the group constituted by:
1. H, provided that R₃ does not represent H,
2. aryls comprising rings with 6 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals with 1 to 15 carbon atoms, optionally substituted,
∘ alkenyl radicals with 1 to 15 carbon atoms, optionally substituted,
∘ alkynyl radicals with 1 to 15 carbon atoms, optionally substituted,
∘ aryls with 6 to 12 carbon atoms, optionally substituted,
∘ aromatic or non-aromatic heterocycles with 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic,
or aromatic or non-aromatic heterocyclic alkyl, alkenyl, alkynyl groups comprising 1 to 15 carbon atoms,
∘ linear or branched alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ aryls with 6 to 12 carbon atoms, optionally substituted,
∘ aromatic or non-aromatic heterocycles comprising 2 to 12 carbon atoms, optionally substituted,
3. heterocycles or heteroaryls comprising rings with 2 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
4. the linear or branched alkenyls comprising 1 to 12 carbon atoms, optionally substituted, or carbon rings comprising 3 to 12 carbon atoms and one or more C=C double bonds, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
5. the linear or branched alkynyls comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
6. linear, cyclic or branched alkyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
at least one of the R₁ and R₄ groups representing H,
▪ R₂ is chosen from the group constituted by the groups being able to be represented by R₁ or R₄, as well as -COR^{a}, -COOR^{a}, -CONH₂, -CONHR^{a}, -CONR^{a}R^{b}, -CN and -NO₂,
in which R^{a} and R^{b}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
R^{a} and R^{b} being able to be linked in order to form a ring, optionally substituted,
▪ W represents a functional group chosen from -CHO, -COR^{a}, -COOH, -COOR^{a}, -CONH₂, -CONHR^{a}, -CONR^{a}R^{b}, -CONH-SO₂-R^{a}, -CH₂OH, -CH₂OR^{a}, -CHR^{b}OH, -CHR^{b}OR^{a}, -CR^{b}R^{b'}OH, -CR^{b}R^{b'}OR^{a}, -CH₂NH₂, -CH₂NHZ, -CHR^{a}NHZ, -CH₂-NH-COR^{a}, in which Z represents a protective group of an amine function, and
in which R^{a}, R^{b} and R^{b'}, identical or different, represent linear, cyclic or branched, aromatic, or aromatic or non-aromatic heterocyclic alkyl, alkenyl, alkynyl groups comprising 1 to 15 carbon atoms, optionally substituted by:
∘ one or more halogen atoms comprising fluorine, chlorine, bromine or iodine,
∘ hydroxy, amino or thio radicals optionally protected by "ad hoc" protective groups,
∘ -OR^{a}, -NHR^{a}, -NR^{a}R^{b}, -SR^{a}, -OCOR^{a}, -OCONHR^{a}, -OCONR^{a}R^{b}, -CHO, -COR^{a}, -COOH, -CN, -COOR^{a}, -CONHR^{a}, -CONR^{a}R^{b}, -CF₃, -NO₂, -N=C-NHR^{a}, -N=C-NR^{a}R^{b}, -N=C-NH₂, -N=C-NHCOR^{a}, -N=C-NH-COOR^{a}, -N(C=NH)NH₂, -N-(C=NCOR^{a})NHCOR^{b}, -N(C=NCOOR^{a})NHCOOR^{b} radicals,
in which R^{a} and R^{b}, identical or different, represent linear or branched, aromatic, or aromatic or non-aromatic heterocyclic alkyl, alkenyl, alkynyl groups comprising 1 to 15 carbon atoms,
∘ alkyl radicals comprising 1 to 15 carbon atoms, optionally substituted,
∘ linear or branched alkenyl radicals comprising 1 to 15 carbon atoms, optionally substituted,
∘ linear or branched alkynyl radicals comprising 1 to 15 carbon atoms, optionally substituted,
∘ linear or branched aryl radicals comprising 6 to 12 carbon atoms, optionally substituted,
∘ aromatic or non-aromatic heterocycles comprising 2 to 12 carbon atoms, optionally substituted,
provided that:
▪ when R₁, R₂ and R₄ represent H and B represents B(OH)₂, B(OR)₂, or B(OR')₃M, then W is chosen from -COR^{a}, -CONH₂, -CONHR^{a}, and -CONR^{a}R^{b},
▪ when R₁, R₂ and R₄ represent H and B represents BF₃M, then W is chosen from -COOH, -COOR^{a}, -CHO, -COR^{a} -CONH₂, -CONHR^{a}, -CONR^{a}R^{b},
▪ when W represents CH₂OH, -CH₂OR^{a}, -CHR^{b}OH, -CHR^{b}OR^{a}, -CR^{b}R^{b'}OH, or -CR^{b}R^{b'}OR^{a}, and B represents B(OH)₂, B(OR)₂, or B(OR')₃M, then:
∘ R₁ does not represent H, or
∘ R₂ and R₄ do not represent H,
▪ when W represents CH₂OH, -CH₂OR^{a}, -CHR^{b}OH, -CHR^{b}OR^{a}, -CR^{b}R^{b'}OH, or -CR^{b}R^{b'}OR^{a}, and B represents BF₃M, then R₁ or R₂ does not represent H.
▪ when W represents -COOH or -COOR^{a}, and B represents B(OH)₂, B(OR)₂, or B(OR')₃M, then:
∘ R₁ does not represent H, or
∘ R₂ and R₄ do not represent H,
by reaction of said compound of formula (I) with a compound of the following formula (III):
R₃-X₂,
in which R₃ is as defined above, and X₂ is chosen from the group constituted by the halides, in particular iodine, bromine and chlorine, and triflate (OTf), in the presence of a catalyst containing a transition metal, and, optionally, of a ligand.

10. Use according to claim 9, in which R₃ represents a group chosen from:
